(19) Europäisches Patentamt
European Patent Office
Office européen des brevets

(11) EP 1 881 000 A1

(12) **EUROPEAN PATENT APPLICATION**

(43) Date of publication:
**23.01.2008 Bulletin 2008/04**

(21) Application number: **06291157.3**

(22) Date of filing: **17.07.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(51) Int Cl.:
***C07H 13/04*** *(2006.01)* ***C07H 13/12*** *(2006.01)*
***A61K 31/7028*** *(2006.01)* ***A61P 35/00*** *(2006.01)*

(71) Applicant: **INSTITUT NATIONAL DE LA SANTE ET DE LA RECHERCHE MEDICALE (INSERM)**
**75013 Paris (FR)**

(72) Inventor: **The designation of the inventor has not yet been filed**

(74) Representative: **Le Coupanec, Pascale A.M.P.**
**Nony & Associés**
**3, rue de Penthièvre**
**75008 Paris (FR)**

(54) **Conjugates of 2-fluoro-2-deoxy-glucose and their uses as anti cancer agents**

(57) The present invention relates to compounds of general formula (I):

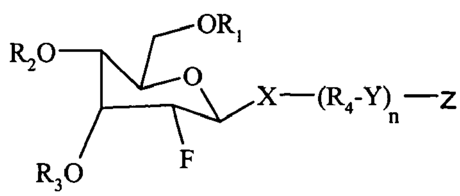

(I)

wherein:
- $R_1$, $R_2$ and $R_3$ mean, independently from the others, an hydrogen atom or an optionally substituted lower alkyl, a ($C_1$-$C_7$)acyl group or a benzyl,
- $R_4$ is an optionally substituted and/or interrupted hydrocarbon linker,
- X and Y are selected independently from each other from the group consisting of -NH-, -NR'-, -CO-, -NH(CO)-, -NH(CO)NH-, -NR'(CO)-, -O(CO)NH-, -O(CO)NR'-, -(CO)NH-, -(CO)NR'-, -NH(CO)(R")NH(CO)-, -(CO)NH(R"(CO)NH-, -O-, -(CO)O-, -NH(CO)O-, -SC(O)NH-, -$NHSO_2$-, -$NR'SO_2$- and -O(CO)- and preferably are different one from the other, with R' being an optionally substituted lower alkyl group, and R" being a ($C_1$-$C_3$) alkylene,
- Z moiety represents an anti-neoplasic agent,
- n means 0 or 1,
- and the pharmaceutically acceptable salts thereof.

It relates also to the use of such compounds as anti cancer agents.

EP 1 881 000 A1

## Description

The present invention relates to methods for treating cancer and pre-cancerous cells and tissues, as well as compounds that are conjugates of glucose or analogs or derivatives thereof, and anti-cancer compositions for use in the methods of the invention.

The term "cancer" generally refers to any of a group of more than 100 diseases caused by the uncontrolled growth of abnormal cells. Unlike normal cells, which reproduce until maturation and then only as necessary to replace wounded cells, cancer cells can grow and divide endlessly, crowding out nearby cells and eventually spreading to other parts of the body.

Cancer can take the form of solid tumors and lymphomas, and non-solid cancers such as leukemia.

The principal problem of cancer therapy is achieving efficient and selective killing of tumor cells.

In effect, a great number of drugs used to kill cancer cells are also toxic to cells of normal tissue, and so side effects are often severe.

Furthermore, the majority of drug-mediated cancer therapies rely on drugs that selectively poison dividing cells. These drugs can be effective, because cancer cells generally divide more frequently than normal cells. Unfortunately, there are exceptions to this rule in most cancers, which means that such drugs almost inevitably are unable to kill all cells in a tumor.

Accordingly, there is still a need for cytotoxic compounds that preferentially target cancer cells and efficiently killed them.

To achieve in part this goal, scientists considered that inhibition of anaerobic glycolysis by metabolic poisons could be useful to preferentially target cancer cells. In effect, due to the markedly enhanced utilization of glucose in cancer cells mediated by increased activity of the proteins mediating these steps, the 2-deoxy-D-glucose and related conjugates are accumulated preferentially in cancer cells compared to normal cells.

Thus, the compound 2-deoxy-D-glucose (also known and referred to herein as 2-deoxyglucose and 2-DG) is such a metabolic poison. 2-DG inhibits glycolysis in cancer cells, as reported in the reference Woodward, 1954, Cancer Res. 14:599-605. However, 2-DG has not be approved for the treatment of cancer in the United States or in Europe.

Other known anti-cancer agents having a structure based on a glucose moiety attached to a cytotoxic agent have also been used to treat cancer. For example, Glufosfamide (beta-D-glucosyl-ifosfamide mustard) is an anti-cancer agent that can be described as a cytotoxic agent linked to a glucose moiety. This compound contains the cytotoxic agent ifosfamide coupled to glucose via an ester linkage at the oxygen atom at the 1-position of glucose (see U.S. Pat. No. 5,622,936). Prodrug forms of pharmacologically active substances, including anti-cancer agents, and that contain a glucose or other sugar moiety linked to a pharmacologically active agent either directly or through a spacer at the 1 position (see U.S. Pat. No. 5,621,002) or at the 2 position (see U.S. Pat. N° 7,001,888) have also been described.

However, while compounds have been made that contain a cytotoxic agent linked to glucose, most of those compounds have not been approved for the treatment of cancer, and none of those compounds appears to have significant specificity for cancer cells.

Accordingly, there remains a need for methods and compositions for treating cancer, including tumors, non-solid cancers, and cancer cells, that may be widely applicable in a variety of cancers.

The present invention provides such methods, as well as compounds and compositions useful in those methods.

In particular, the present invention derives in part from the unexpected discovery by the inventors that conjugates of 2-fluoro-2-deoxy-glucose or derivatives, and anti-neoplasic agents linked via non-releasable linkages experience enhance activity in cancer cells.

Thus in a first aspect, the present invention provides compounds of general formula (I):

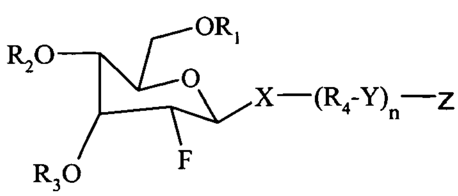

(I)

wherein:

- $R_1$, $R_2$ and $R_3$ mean, independently from the others, an hydrogen atom, or an optionally substituted lower alkyl, a ($C_1$-$C_7$)acyl group or a benzyl, and preferably an hydrogen atom or a lower alkyl having 2 carbon atoms,
- $R_4$ is an optionally substituted and/or interrupted hydrocarbon linker,
- X and Y are selected independently from each other, from the group consisting of -NH-, -NR'-, -CO-, -NH(CO)-,

-NH(CO)NH-, -NR'(CO)-, -O(CO)NH-, - O(CO)NR'-, -(CO)NH-, -(CO)NR'-, -NH(CO)(R")NH(CO)-, -(CO)NH(R")-(CO)NH-, -O-, -(CO)O-, -NH((CO)O-, -SC(O)NH-, $-NHSO_2-$, $-NR'SO_2-$ and -O(CO)- and préférably are different one from the other, with R' being an optionally substituted lower alkyl group, and R" being a $(C_1-C_3)$alkylene,

- Z moiety represents an anti-neoplasic agent,
- n means 0 or 1,
- and the pharmaceutically acceptable salts thereof.

In a further aspect, the present invention provides pharmaceutical composition including, in a physiological acceptable vehicle, at least one compound according to the invention.

In a further aspect, the present invention relates to the use of a compound according to the invention as an anti-cancer agent and in particular for preparing a pharmaceutical composition intended to the treatment of cancer and in particular solid cancer.

In a further aspect, the present invention provides a method for treatment of cancer, in particular solid cancer comprising the administration to a patient in need thereof, of at least a compound according to the instant invention.

The 2-fluoro-2-deoxy-glucose, 2-FDG, is a known reagent more particularly used for detecting cancer tissue in an animal based on the use of positron emission tomography (PET scan). These detecting methods takes advantage of the increased uptake and retention by malignant cells of 18F-labeled glucose derivative (FDG, 18F-2-fluoro-2-deoxyglucose). This glucose derivative, lacking a hydroxy group at the 2-position, cannot be further metabolized by the cells and is simply accumulated in the cells.

Unexpectedly, the inventors noticed that the conjugates based on the 2-fluoro-2-deoxy-glucose can be used to treat cancer tissue and cancer cells preferentially. The selectivity of the conjugates for preferentially killing cancer cells is provided by the increased transport, relative to non-cancer cells, of the conjugate due to the up-regulation of the glucose transporters (GLUTs) in cancer cells, as well as by, at least in some cancer cells, the up-regulated expression of hexokinase.

The term lower alkyl as used herein refers to a $(C_1-C_7)$alkyl and in particular straight or branched-chain hydrocarbon radical of one to seven carbon atoms and cyclized manifestations thereof unless otherwise indicated. Included within the scope of this term are such moieties as methyl, ethyl, isopropyl, n-butyl, t-butyl, t-butylmethyl, cyclopropyl, N-propyl, pentyl, cyclopentyl, n-hexyl, cyclohexyl, cyclohexylmethyl, 2-ethylbutyl etc.

As used herein, the term "optionally substituted lower alkyl" means a lower alkyl as defined above optionally substituted by one or more group chosen from halogen atom, hydroxy group, $(C_1-C_7)$alkoxy group and amino group.

More particularly, in the compounds, according to the invention, the linking group formed by $R_4$ includes linear or acyclic portions, cyclic portions, aromatic rings or combinations thereof. It can be saturated or unsaturated.

Additionally, this linking group is sufficiently robust so that it is stable to reaction conditions used in conjugate assembly, e.g., the protection/deprotection chemistries used to prepare the conjugates of the invention.

More specifically, $R_4$, when present, is of formula

- -(Arylene)m-(P)p-(Alkylene)q-
  or
- -(Alkylene)m-(P)p-(Arylene)q-

wherein

- m, p and q mean, independently one from the others, 0 or 1 with the proviso that at least one of them is different from 0,
- Alkylene means an optionally substituted (C1-C7)alkylene group and preferably a (C1-C3)alkylene and more preferably a methylene group,
- Arylene means an optionally substituted C5 to C10 arylene or heteroarylene, and
- P means a group as proposed for the definition of X and Y and preferably selected from -NH(CO)-,-NR'(CO)-,-O(CO)NH-,-O(CO)NR',-(CO)NH,-(CO)NR'-,-O(CO)-,-(CO)O-,-NH(CO)O-,-NR'(CO)O with R' as previously defined, and more preferably NH(CO)-.

As used herein, the term "alkylene" refers to a saturated or unsaturated straight or branched chain or cyclic divalent hydrocarbon radical. Examples of "alkylene" as used herein include, but are not limited to, methylene, ethylene, trimethylene, tetramethylene and the like. It is to be understood that where cyclic moities are intended, at least three carbone in said alkylene must be present. Such cyclic moieties include cyclopropylene, cyclobutylene, cyclopentylene, cyclohexylene and cycloheptylene.

As used herein, the term "arylene" refers to divalent unsaturated aromatic radicals having a single ring such as phenylene, or multiple condensed rings, such as naphtylene or anthrylene.

Preferably, the arylene group is selected from the group consisting of benzylene, phenylene, biphenylene,

anthrylene, phenylbenzylene, fluorenylene, naphtylene, dihydronaphtylene, tetrahydronaphtylene, indenylene and indanylene, and more preferably is the phenylene group.

As used herein, the term "heteroarylene" refers to a divalent radical which may be an aromatic monocycle, an aromatic bicycle, an aromatic tricycle, or a bicycle or a tricycle of which one or two of the cycles is aromatic and the other cycle(s) is or are partially hydrogenated, from $C_5$ to $C_{14}$ comprising within the cyclic system one, two or three heteroatoms, identical or different, selected among oxygen, nitrogen and sulphur.

Each one of these alkylene, arylene or heteroarylene groups may optionally comprise one or more substitutions, identical or different, for example one to three and more preferably one or two, selected from the group consisting of a halogen, the hydroxy group, a straight or linear $(C_1-C_7)$alkyl group, $(C_5-C_{10})$aryl group optionnaly substituted like a p.tolyl group, a straight or linear $(C_1-C_7)$alkenyl group, a $(C_1-C_7)$alkoxy group, a $(C_7-C_{13})$arylalkoxy group, the cyano group, the nitro group, the amino group, a $(C_1-C_7)$alkylamino group, a HO-$(C_1-C_7)$alkyl- group, a $H_2N$-$(C_1-C_7)$alkyl- group, a HO-(C=O)-group, a $(C_1-C_7)$alkyl-O-(C=O)- group, a $(C_1-C_7)$alkyl-(C=0)- group, a $(C_1-C_7)$alkyl-(C=O)-$(C_1-C_8)$alkyl-group, a $HSO_3(C_1-C_7)$alkyl- group, a $H_2N$-(C=O)- group and a $H_2N$-(C=O)-$(C_1-C_7)$alkyl- group.

The term halogen refers to fluorine, chlorine, bromine or iodine atom.

Fluorine and chlorine are preferred halogen atoms in the framework of the present invention.

The term $(C_1-C_7)$alkoxy refers to alkoxy radical made up of an oxygen radical bearing a saturated straight or branched chain hydrocarbon radical of one to seven carbon atoms. Included within the scope of this term are methoxy, ethoxy, propoxy, n-butoxy, isobutoxy, sec-butoxy, t-butoxy, n-pentoxy, isopentoxy, sec-pentoxy, t-pentoxy... and the like.

A first class of such compounds of formula (I), hereinafter referring to compounds of formula (Ia), is such that n is equal to zero.

Among this first class of compounds of formula (Ia), the compounds for which the therapeutical use is the most preferred are characterized in that X means:

- -O(CO)-,
- -NH(CO)- or
- -NH(CO)NH-.

A second class of such compounds of formula (I), hereinafter referring to compounds of formula (Ib), is such that n is different from zero and $R_4$ comprises or is

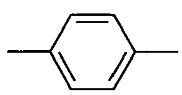

.

Among this second class of compounds of formula (Ib), the compounds for which the therapeutical use is the most preferred are characterized in that X is selected from the group consisting of -NH-, -NH(CO)-,-O-, -O(CO)-,-O(CO)NH- and -NH(CO)O-.

More particularly, Y is -NHCO-, -O(CO)-,-OCONH- or -NH(CO)O-.

A third class of such compounds of formula (I), hereinafter referring to compounds of formula (Ic), is such that n is different from zero and $R_4$ includes or is a $(C_1-C_7)$alkylene group in particular a $(C_1-C_3)$alkylene group and more particularly a methylene group.

Among this third class of compounds of formula (Ic), the compounds for which the therapeutical use is the most preferred are characterized in that X and Y represent a group selected from the group consisting of -NH(CO)-, -(CO)NH-, -NH(CO)NH-, -O(CO)-, -O(CO)NH and -NH(CO)O-.

A fourth class of such compounds of formula (I), hereinafter referring to compounds of formula (Id), is such that n is different from zero and $R_4$ means a radical of formula (Arylene)-P-(Alkylene) or (Alkylene)-P-(Arylene) wherein:

- Arylene is

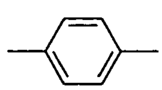

,

- Alkylene is a $(C_1-C_3)$alkylene group and in particular a methylene group, and
- P is -NHCO-, CONH-, - NR' or -NH- with R' being as previously defined.

In one particular embodiment of the present invention, $R_1$, $R_2$ and $R_3$ are in formula I, Ia, Ib, Ic and Id different from hydrogen. More particularly, they mean an acyl group, and in particular an acetyl group.

Regarding the anti-neoplasic agents, it is noticed that it can be chosen among a large number of anti-cancer agents including chemotherapeutic agents and radioisotopic agents.

To facilitate uptake of the conjugate through a glucose transporter, however, the anti-neoplasic agent is, in a preferred embodiment, a relatively small molecule, for example, a molecule having a molecular weight less than 1,000 daltons, preferably less than 800 daltons, and most preferably less than 500 daltons. In further preferred embodiments, the anti-neoplasic agent and the linking group together have a molecular weight of from about 100 to about 2000 daltons, or 250 to about 1500 daltons, or 300 to about 1250 daltons, or 400 to about 1000 daltons.

In particular, the attached anti-neoplasic agent is preferably an agent that kills dividing cells, arrests cell division, or prevents metastasis.

More particularly, the terms "anti-neoplasic agent" and "anti-cancer agent" refer to a compound that prevents, kills, or blocks the growth and spread of cancer cells. The term is inclusive of those agents that are considered alkylating agents, intercalating agents, antimetabolites, radionuclides, metal poisons, enzyme inhibitors, microtubule "inhibitors" and the like.

For obvious reasons, the choice of the anti-neoplasic agent may be operated with respect to the kind of cancer to be treated.

Thus, the methods and compounds of the present invention can be used to treat the most common cancers, including but not limited to bladder cancer, breast cancer, colorectal cancer, endometrial cancer, head and neck cancer, leukemia, lung cancer, lymphoma, melanoma, non-small cell lung cancer, ovarian cancer, and prostate cancer.

In particular, the methods and compounds of the present invention can also be used to treat cancers that have originated in or metastasized to the bone, brain, breast, digestive and gastrointestinal systems, endocrine system, eye, genitourinary tract, germ cells, gynecological system, head and neck, hematologic system, blood, lung, respiratory system, thorax, musculoskeletal system, and skin.

The compounds of the present invention are generally applicable to all cancers but have particularly significant therapeutic benefit in the treatment of solid tumors, which are characterized by extensive regions of hypoxic tissue.

Thus, an agent like cyclophosphamide, doxorubicin and vincristine (CAV); etoposide and cisplatin (VP-16); and cyclophosphamide, doxorubicin and VP-16 (CAVP-16) has been reported for small cell lung cancer.

The most active drugs in the treatment of ovarian cancer have been alkylating agents or cross-linking agents, including cyclophosphamide, ifosfamide, melphalan, chlorambucil, thiotepa, cisplatin, and carboplatin.

The agents studied most extensively for treating prostate cancer are estramustine phosphate, prednimustine, and cisplatin.

Additionally, conjugates with 5-fluorouracil, camptothecin and camptothecin analogs are particularly useful for the treatment of colon cancer.

Microtubule "inhibitors," which may inhibit either microtubule assembly or disassembly, useful in the practice of the present invention include but are not limited to vincristine (Oncovin), vinblastine (Velban), paclitaxel (Taxol, Paxene), vinorelbine (Navelbine), docetaxel (Taxotere), epothilone B or D or a derivative of either, and discodermolide or its derivatives.

Nitrosoureas useful in the practice of the present invention include but are not limited to procarbazine (Matulane), lomustine, CCNU (CeeBU), carmustine (BCNU, BiCNU, Gliadel Wafer), and estramustine (Emcyt).

Nucleoside analogs useful in the practice of the present invention include but are not limited to mercaptopurine, 6-MP (Purinethol), fluorouracil, 5-FU, (fluorouracil) (Adrucil), thioguanine, 6-TG (Thioguanine), hydroxyurea (Hydrea), cytarabine (Cytosar-U, DepoCyt), floxuridine (FUDR), fludarabine (Fludara), pentostatin (Nipent), cladribine (Leustatin, 2-CdA), gemcitabine (Gemzar), and capecitabine (Xeloda).

Therefore, the previous agents or derivatives thereof may be efficiently conjugated with 2-FDG according to the instant invention, for enhancing their uptake by cancer cells.

More particularly, the compounds according to the invention includes an anti-neoplasic agent selected among chlorambucil, nitrosoureas, cisplatin, carboplatin, and fluorouracil.

The compounds of formula (I) can comprise one or more asymmetrical carbon atoms. They can thus exist in the form of enantiomers or of diastereoisomers. These enantiomers, diastereoisomers, as their mixtures, including the racemic mixtures form part of the invention.

The compounds of the invention may exist in the form of free bases or of addition salts with pharmaceutically acceptable acids.

Suitable pharmaceutically acceptable salts of compounds of formula (I) include base addition salts and where appropriate acid addition salts. Suitable physiologically acceptable base addition salts of compounds of formula (I) include alkali metal or alkaline metal salts such as sodium, potassium, calcium, and magnesium salts, and ammonium salts, formed with amino acids (e.g. lysine and arginine) and organic bases (e.g. procaine, phenylbenzylamine, ethanolamine diethanolamine and N-methyl glucosamine).

Suitable acid addition salts may be formed with organic acid and inorganic acids e.g. hydrochloric acid.
The compounds of formula (I) and or salts thereof may form solvates (e.g. hydrates) and the invention includes all such solvates.
More specifically, the instant invention is also directed to the following compounds :

3,4,6-tri-O-acetyl-1-[4-{4-[bis(2-chloroethyl)amino]phenyl}butyrate]-2-deoxy-2-fluoro-α,β-D-glucopyranose (14a).
3,4,6-tri-O-benzyl-1-[4-{4-[bis(2-chloroethyl)amino]phenyl}butyrate]-2-deoxy-2-fluoro-α,β-D-glucopyranose (14b).
1-[4-{4-[bis(2-chloroethyl)amino]phenyl}butyrate]-2-deoxy-2-fluoro-α,β-D-glucopyranose (14c).
N-(3,4,6-tri-O-acetyl-2-deoxy-2-fluoro-β-D-glucopyranosyl)-4-{4-[bis(2-chloroethyl)amino]phenyl}butanamide (15a).
4-{4-[bis(2-chloroethyl)amino]phenyl}-N-(2-deoxy-2-fluoro-β-D-glucopyranosyl)butanamide (15b).
N-(3,4,6-tri-O-acetyl-2-deoxy-2-fluoro-β-D-glucopyranosyl)-N'-{3-(4-[bis(2-chloroethyl)amino]phenyl)propyl}urea (17a).
N-{3-(4-[bis(2-chloroethyl)amino]phenyl)propyl}-N'-(2-deoxy-2-fluoro-β-D-glucopyranosyl)urea (17b).
N-(3,4,6-tri-O-acetyl-2-deoxy-2-fluoro-β-D-glucopyranosyl)-4-(4-{4-[bis(2-chloroethyl)amino]phenyl}butanamido) benzamide (20a).
4-(4-{4-[bis(2-chloroethyl)amino]phenyl}butanamido-N-(2-deoxy-2-fluoro-β-D-glucopyranosyl)benzamide (20b)
N-[2-(3,4,6-tri-O-acetyl-2-deoxy-2-fluoro-β-D-glucopyranosylamino)-2-oxoethyl-4-{4-[bis(2-chloroethyl)amino]phenyl}butanamide (21a).
4-{4-[bis(2-chloroethyl)amino]phenyl}-N-[2-(2-deoxy-2-fluoro-β-D-glucopyranosylamino)-2-oxoethyl]butanamide (21b).
N-[4-(3,4,6-tri-O-acetyl-2-deoxy-2-fluoro-β-D-glucopyranosylamino)-4-oxobutyl]-4-{4-[bis(2-chloroethyl)amino] phenyl}butanamide (22a)
4-{4-[bis(2-chloroethyl)amino]phenyl}-N-[4-(2-deoxy-2-fluoro-β-D-glucopyranosylamino)-4-oxobutyl]butanamide (22b)
N-[3-(3,4,6-tri-O-acetyl-2-deoxy-2-fluoro-β-D-glucopyranosylamino)-3-oxopropyl]-4-{4-[bis(2-chloroethyl)amino] phenyl}butanamide (31a)
4-{4-[bis(2-chloroethyl)amino]phenyl}-N-[3-(2-deoxy-2-fluoro-β-D-glucopyranosylamino)-3-oxopropyl]butanamide (3 1 b)
N-[4-(4-{4-[bis(2-chloroethyl)amino]phenyl}butanoyloxy)phenyl-3,4,6-tri-O-benzyl-2-deoxy-2-fluoro-β-D-glucopyranosylamine (34a).
N-[4-(4-{4-[bis(2-chloroethyl)amino]phenyl}butanoyloxy)phenyl-2-deoxy-2-fluoro-β-D-glucopyranosylamine (34b).
N-[4-(3,4,6-tri-O-acetyl-2-deoxy-2-fluoro-β-D-glucopyranosyloxy)phenyl]-4-{4-bis(2-chloroethyl)amino]phenyl}butanamide (37a)
4-{4-[bis(2-chloroethyl)amino]phenyl}-N-[4-(2-deoxy-2-fluoro-β-D-glucopyranosyloxy)phenyl]butanamide (37b)
N-{2-[4-(3,4,6-tri-O-acetyl-2-deoxy-2-fluoro-β-D-glucopyranosyloxy)phenyl]amino-2-oxoethyl}-4-{4-[bis(2-chloroethyl)amino]phenyl}butanamide (40a)
4-{4-[bis(2-chloroethyl)amino]phenyl}-N-{2-[4-(2-deoxy-2-fluoro-β-D-glucopyranosyloxy)phenyl]amino-2-oxoethyl} butanamide (40b) and their salts

The following compounds are particularly advantageous:

N-(3,4,6-tri-O-acetyl-2-deoxy-2-fluoro-β-D-glucopyranosyl)-4-(4- {4-[bis(2-chloroethyl)amino]phenyl}butanamido) benzamide (20a).
4-(4-{4-[bis(2-chloroethyl)amino]phenyl}butanamido-N-(2-deoxy-2-fluoro-β-D-glucopyranosyl)benzamide (20b)
N-[2-(3,4,6-tri-O-acetyl-2-deoxy-2-fluoro-β-D-glucopyranosylamino)-2-oxoethyl-4-{4-[bis(2-chloroethyl)amino]phenyl}butanamide (21 a).
4-{4-[bis(2-chloroethyl)amino]phenyl}-N-[2-(2-deoxy-2-fluoro-β-D-glucopyranosylamino)-2-oxoethyl]butanamide (21b).
N-{2-[4-(3,4,6-tri-O-acetyl-2-deoxy-2-fluoro-β-D-glucopyranosyloxy)phenyl]amino-2-oxoethyl}-4-{4-[bis(2-chloroethyl)amino]phenyl}butanamide (40a) and
4-{4-[bis(2-chloroethyl)amino]phenyl}-N-{2-[4-(2-desoxy-2-fluoro-β-D-glucopyranosyloxy)phenyl]amino-2-oxoethyl}butanamide (40b).

The compounds according to the invention may be obtained by a direct or indirect attachment of the anti-neoplasic agent to the 2-fluoro-2-deoxyglucose or derivatives. The linking chemistry has to be compatible with both the anti-neoplasic agent and the 2-FDG or derivatives and to provide a non releasable attachment between those two components. Indeed, the linkage must be effective for keeping the anti-neoplasic agent into the cancer cells a sufficient

time.

Typically X and Y derive from functional groups that are used to interact with and form covalent bonds with functional groups in the components of the conjugates i.e. the 2-FDG and the anti-neoplasic agent.

Examples of functional groups prior to interaction include $-NH_2$, $-NHNH_2$, $-ONH_2$, $-NHC(O)NHNH_2$, -OH, $-CO_2H$ or -SH. Others suitable bond forming groups are well-known in the literature and can be used to prepare conjugates of the present invention.

One of skill in the art will understand that each of these functional groups can form a covalent linkage to a suitable functional group on the glucose portion or on the anti-neoplasic portion of the conjugate during synthesis of the conjugates.

For example, amino, hydroxy and hydrazino groups can each form a covalent bond with a reactive carboxyl group (e.g., a carboxylic acid chloride or activated ester such as an N-hydroxysuccinimide ester (NHS)).

Thus a possible deoxyglucose moiety for forming the conjugates of the present invention is an amino 2-FDG derivative as the hydrochloride salt. This compound has an amino group that provides a chemical handle for conjugations. Anti-neoplasic agent can be conjugated directly to this amino group via alkyl, amide, carbamate or sulfonamide linkages, for example.

In a first embodiment, the anti-neoplasic agent can be attached directly to 2-FDG or derivatives using, for example, an available functional group or the anti-neoplasic agent. Such a function may be also if necessary transformed in a more convenient function for obtaining the desired conjugate.

This embodiment is in particular considered for preparing compounds of formula (I) wherein n is zero and thus of formula (II):

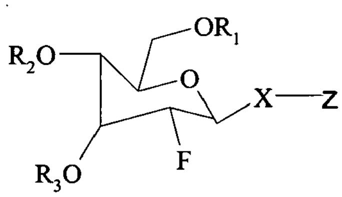

(II)

with $R_1$, $R_2$, $R_3$, X and Z being as defined in formula (I).

For example, X may be issued from the reaction of an hydroxyl function or an amino function located in position 1 of the 2-FDG with an acid function on the anti-neoplasic agent or from the reaction of an amino function located in position 1 of the 2-FDG with an isocyanate issued from the conversion of a carboxy group on the anti-neoplasic agent.

Such a synthesis is illustrated by the following Schemes 1 a, b and c.

The following schemes involve the following compounds as starting materials.

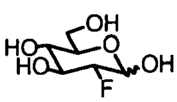

1, FDG

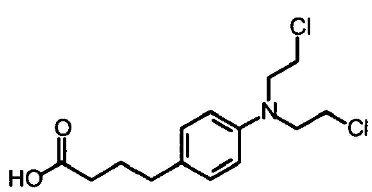

2, Chlorambucil

It is noticed that a number of protected glucose compounds or derivatives are commercially available, or can be prepared according to well-established methods. In many instances, the glucose moiety having "n" hydroxy or amino groups, will have "n-1" protecting groups, thereby leaving one available reactive functional group located in position 1 for either the anti-neoplasic agent or a linking group. For the preparation of compounds possessing an ester linkage between the active drug and the anchor point of the carrier, the use of hydroxyl protecting groups others than acetates appeared preferable. Benzyl ethers were privileged.

## Scheme 1a

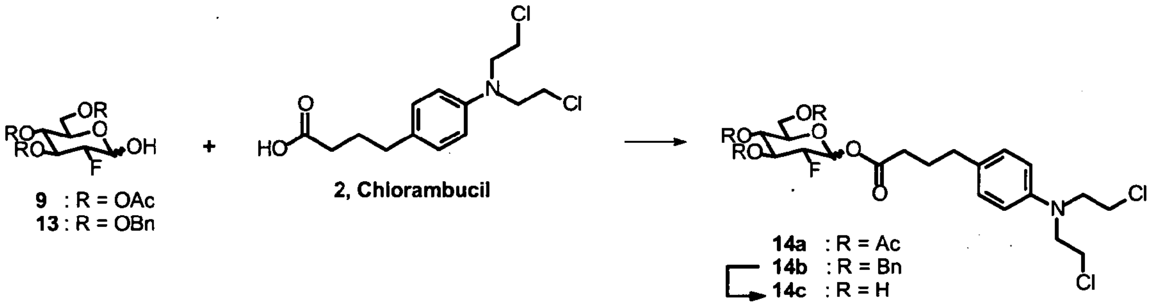

9 : R = OAc
13 : R = OBn

2, Chlorambucil

14a : R = Ac
14b : R = Bn
14c : R = H

## Scheme 1b

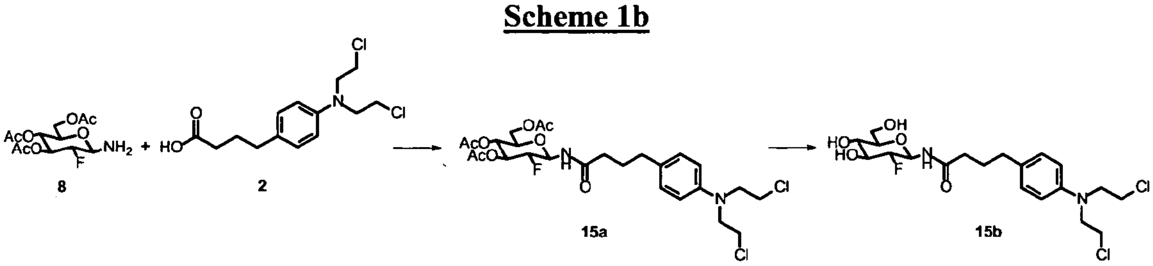

8

2

15a

15b

## Scheme 1c

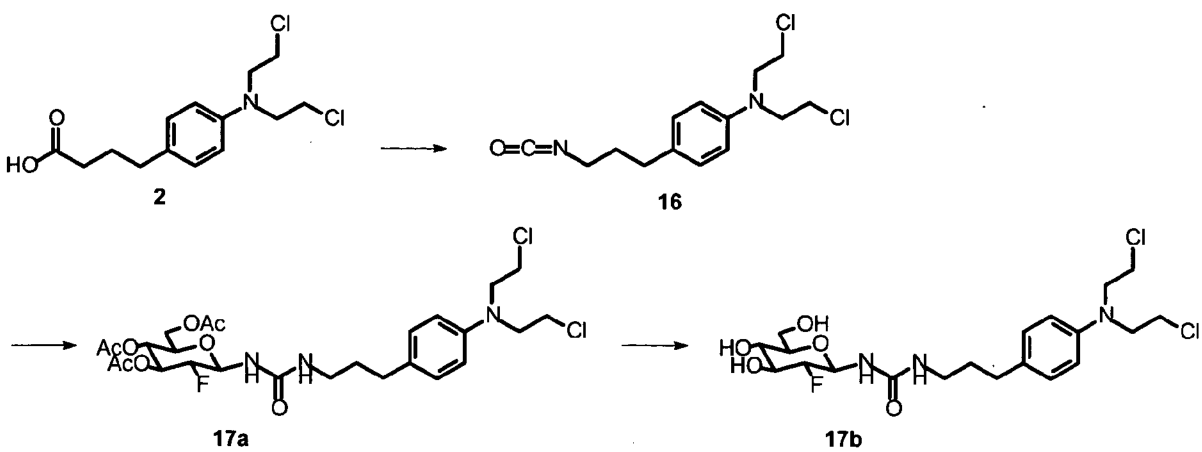

2

16

17a

17b

In a second embodiment, a linking group may be used to attach the anti-neoplasic agent covalently to the 2-FDG derivative.

In such embodiment, any of the commercially available bifunctional linking group can be used.

Alternatively, one of skill in the art can construct a linking group having at least two functional group to attach from one hand the 2-FDG and from the other hand the anti-neoplasic agent.

This embodiment, is more particularly concerned with the preparation of compounds of formula (III):

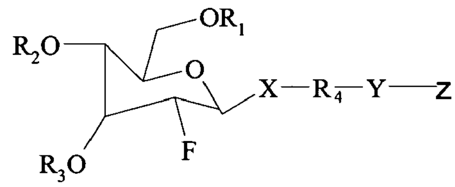

(III)

with $R_1$, $R_2$, $R_3$, X, Y and Z being as defined in formula (I).

Typically a linker or linking group has functional groups that are used to interact with and form covalent bonds with functional groups in the components (e.g., anti-neoplasic agents and glucose or glucose derivatives) of the conjugates described and used herein. Examples of functional groups on the linking groups (prior to interaction with other components) include $-NH_2$, $-NHNH_2$, $-ONH_2$, $-NHC(O)NHNH_2$, -OH, $-CO_2H$, and -SH.

Such synthesis is illustrated by the following Schemes 2a, b, c and d, in the case where Z is chloroambucil.

## Scheme 2a

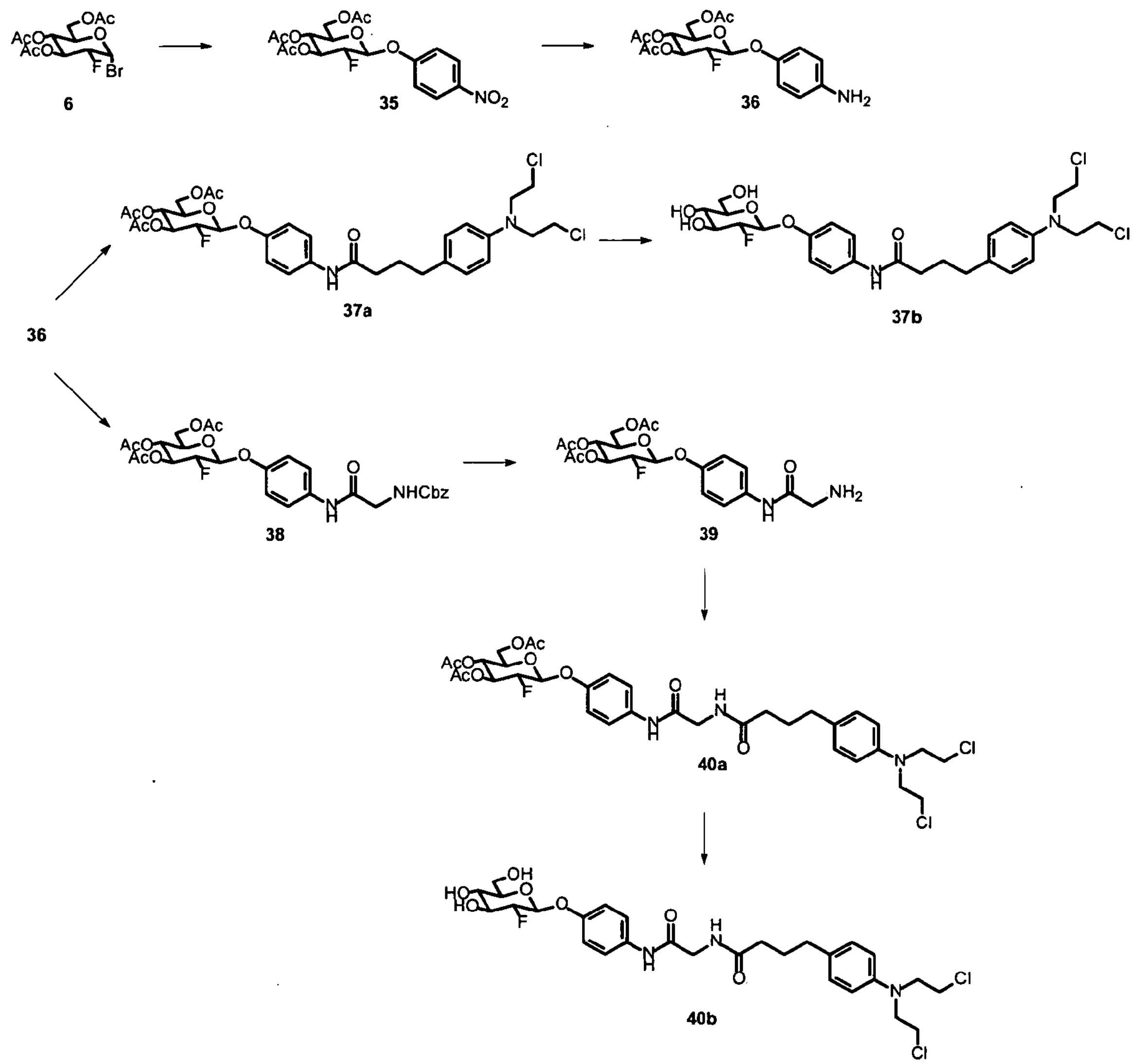

6 35 36 37a 37b 36 38 39 40a 40b

## Scheme 2b

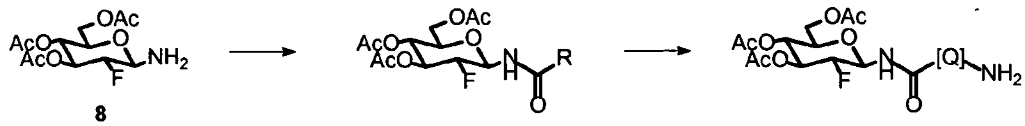

8

**18a** : R = 4-$NO_2C_6H_4$
**18b** : R = $CH_2NHCbz$
**18c** : R = $(CH_2)_3N_3$

**19a** : Q = $C_6H_4$
**19b** : Q = $CH_2$
**19c** : Q = $(CH_2)_3$

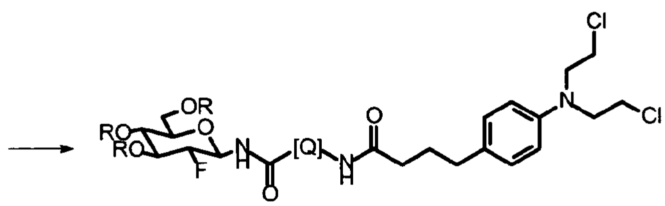

R = OAc

**20a** : Q = $C_6H_4$
**21a** : Q = $CH_2$
**22a** : Q = $(CH_2)_3$

R = OH

**20b** : Q = $C_6H_4$
**21b** : Q = $CH_2$
**22b** : Q = $(CH_2)_3$

## Scheme 2c

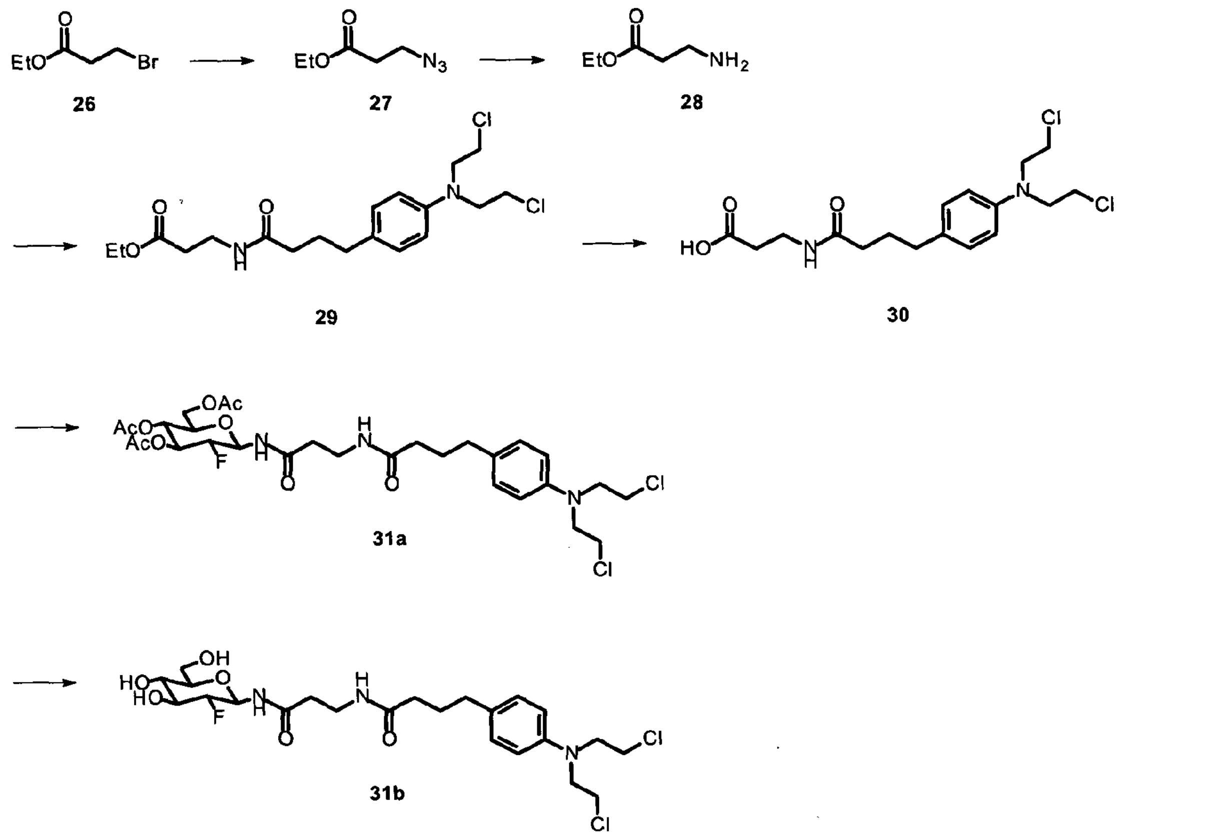

26 27 28 29 30 31a 31b

## Scheme 2d

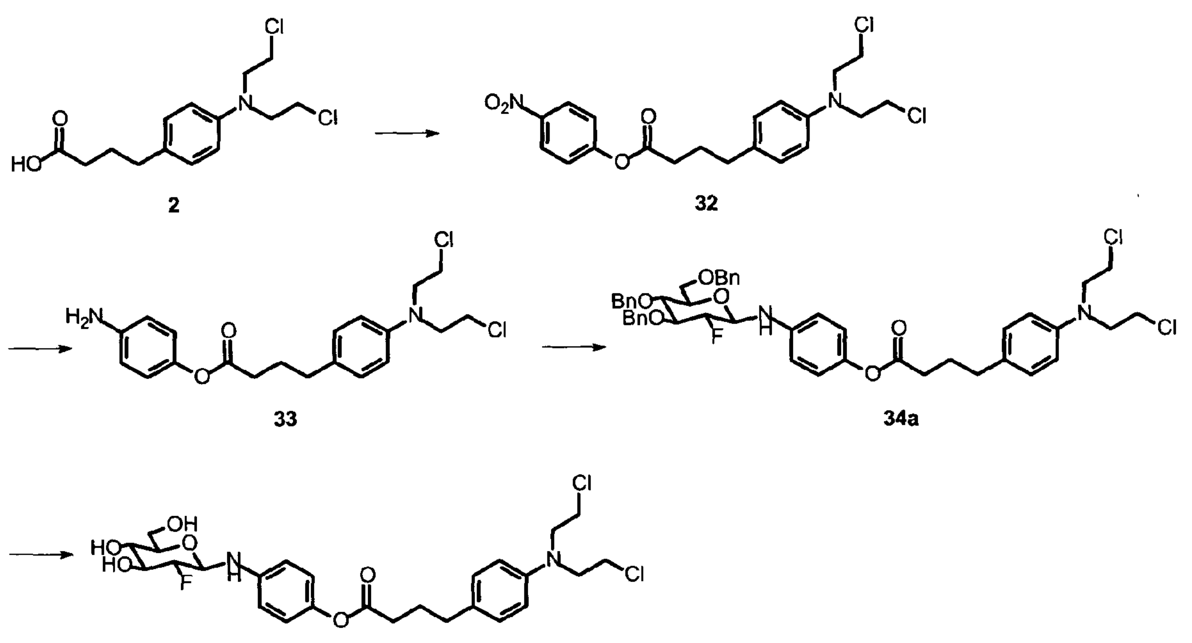

2 32 33 34a 34b

In Schemes 2a and 2b, the linking group firstly interacts with a free amino function of a 2-FDG derivative. The so-obtained coupling product, if necessary, is then activated to obtain a function able to interact with the carboxylic function of chlorambucil to lead to the expected conjugate of formula (III).

In Schemes 2c and 2d, the linking group firstly interacts with the anti-neoplasic agent, i.e. the chlorambucil and then its 2nd function is activated for forming a covalently link with a free amino function or bromide of a 2-FDG derivative.
The following Examples illustrate in detail the preparation of some compounds according to the invention, and more particularly compounds chosen among compounds (14) to (40) and of formula (I).
The figures 1 present the CTW time course of B16 melanoma obtained for compounds 21 a, 40a and chlorambucil administered at the MTD dosage (figure 1a) and 0,75 MTD dosage (figure 1b).
The figure 2 presents the CTW time course of CT 26 colon carcinoma cells obtained for compounds 21 a, 40a and chlorambucil.

## EXPERIMENTAL PART

All solvents and reagents obtained from commercial sources were used without further purification. Nuclear magnetic resonance spectra (1H NMR and 13C NMR) were performed on a Bruker AM 200 (4.5 T) spectrometer. Chemical shifts are reported in parts per million relative to the internal tetramethylsilane standard for 1H NMR and the solvent for 13C NMR (acetone-d, δ = 29.8 ppm; DMSO-d6, δ = 39.5 ppm; CDCl3, δ = 77.2 ppm) The abbreviations used for signal patterns are : br, broad; s, singlet; d, doublet; dd, doublet of doublets; ddd, doublet of doublet of doublets; t, triplet; dt, doublet of triplets; q, quadruplet; qt, quintuplet; m, multiplet. Coupling constants (J values) are given in Hertz. Infrared (IR) spectra were recorded on a FTIR-Nicolet Impact 410 spectrophotometer Melting points were determined on an Electrothermal digital apparatus (Reichert) and were uncorrected. Medium-pressure column chromatography was carried out on silica gel 60 (Chromagel, 35-70 μm, SDS) using the indicated solvent mixture expressed as volume/volume ratios. Analytical thin-layer chromatography (TLC) was conducted on precoated silica gel plates (SDS, 60 F254, 0.2 mm thick) with both detection by ultraviolet light and/or visualization with vanillin in sulfuric acid. Mass spectra were recorded on a Bruker Esquire-LC spectrometer. Electrospray ionization mass spectrometry (ESI-MS) was used in positive mode.
1,3,4,5-tetra-*O*-acetyl-2-deoxy-2-fluoro-β-D-glucopyranose 5 and its derivatives 6-13 (following Scheme 3) are the key intermediates for all the syntheses in which chlorambucil bonds directly or via a spacer to C-1 of the sugar moiety.

**Scheme 3**

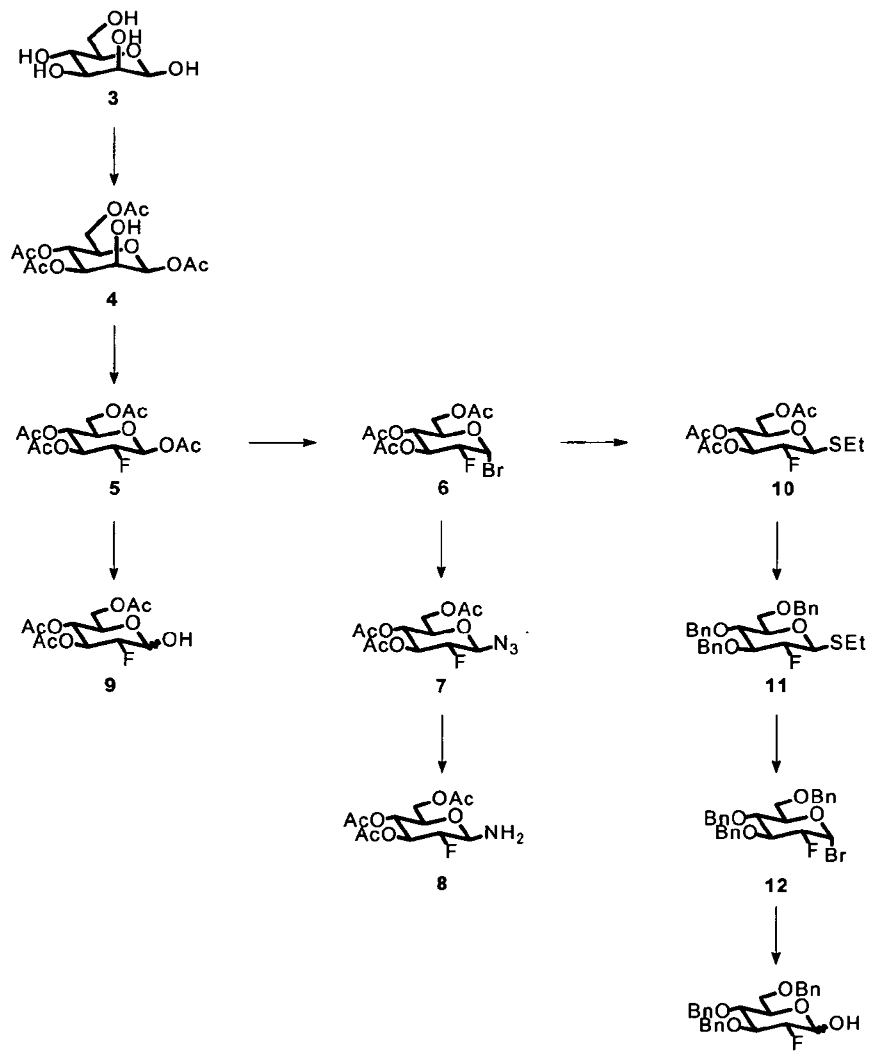

3

4

5 6 10

9 7 11

8 12

13

**3,4,6-tri-O-acetyl-2-deoxy-2-fluoro-β-D-glucopyranosyl azide (7).**

A cold (0˚C) solution of sodium azide (1.76 g, 27.1 mmol) in water (25 mL) is added dropwise, in 20 min, to 3,4,6-tri-O-acetyl-2-deoxy-2-fluoro-α-D-glucopyranosyl bromide 6 (2.51 g, 6.76 mmol), dissolved in acetone (30 mL). After stirring at room temperature, for 1 h, the white precipitate is filtered, thoroughly washed with water and dried under vacuum to offer azide 7 (1.88 g, 84%) as a white powder. The crystalline material was sufficiently pure (t.l.c., 1H NMR) and was used without recrystallization for the next step:

$C_{12}H_{16}FN_3O_7$
M = 333.27 $g.mol^{-1}$
mp 135 ˚C

**3,4,6-tri-O-acetyl-2-deoxy-2-fluoro-β-D-glucopyranosyl amine (8).**

10% Platinum on charcoal (400 mg) was added to a solution of azide 7 (1.99 g, 5.98 mmol) in THF/MeOH (55/55 mL), and the suspension was shaken with hydrogen gas for 1 h at atmospheric pressure and room temperature. After filtration of the suspension on celite, the filtrate was evaporated to dryness to provide amine 8 (1.80 g, 98%) as a white solid:

$C_{12}H_{18}FNO_7$
M = 307.28 $g.mol^{-1}$
mp 110 °C

**3,4,6-tri-O-acetyl-2-deoxy-2-fluoro-α,β-D-glucopyranose (9).**

This compound (443 mg, 1.43 mmol) was prepared as a mixture of α- and β-anomers (57/43), according to the procedure described by Tai et al. (Bioorg. Med. Chem. 2001, 9, 1133-1140), starting from tetraacetate 5 (500 mg, 1.43 mmol):

$C_{12}H_{17}FO_8$
M = 308.26 $g.mol^{-1}$
quantitative yield

**Ethyl 3,4,6-tri-O-acetyl-2-deoxy-2-fluoro-β-D-thioglucopyranoside (10).**

This compound (2.95 g, 8.38 mmol) was prepared according to the procedure described by McCarter et al. (J. Am. Chem. Soc. 1997, 119, 5792-5797), starting from bromide 6 (3.59 g, 9.6 mmol):

$C_{14}H_{21}FO_7S$
M = 352.37 $g.mol^{-1}$
yield 87%;
mp 125°C

**Ethyl 3,4,6-tri-O-benzyl-2-deoxy-2-fluoro-β-D-thioglucopyranoside (11).**

To a stirred solution of acetylated compound 10 (1.20 g, 3.41 mmol) in methanol (12 mL) was added sodium methoxide (54 mg, 1.02 mmol). After 1h15 at room temperature, the mixture was evaporated to dryness. The remaining residue was dissolved in DMF (8 mL) and cooled in an ice cold bath, before the addition of NaH (60% dispersion in mineral oil, 613 mg, 15.3 mmol). The stirring was maintained until return to room temperature. Benzyl bromide (2.43 mL, 20.5 mmol) and potassium iodide (55 mg, 0.34 mmol) were added and the mixture was heated overnight at 60°C. After cooling, MeOH (0.5 mL) followed by water (50 mL) were added and the mixture was extracted twice with $CH_2Cl_2$. The combined organic layers were washed with water (30 mL), dried over $MgSO_4$ and evaporated in vacuo. The crude product was purified by silica gel chromatography (9:1 petroleum benzine:ethyl acetate), to give syrupy compound 11 (1.03 g, 61 %):

$C_{29}H_{33}FO_4S$
M = 496.64 $g.mol^{-1}$

**3,4,6-tri-O-benzyl-2-deoxy-2-fluoro-α-D-glucopyranosyl bromide (12).**

This compound (1.08 g, 2.09 mmol) was prepared according to the procedure described by McCarter et al., starting from benzylated ethyl thioglycoside 11 (1.45 g, 2.92 mmol). Purification on a column of silica gel, eluting with $CH_2Cl_2$, provided benzylated bromide 12 as an oil in 72% yield;

$C_{27}H_{28}BrFO_4$
M = 515.42 $g.mol^{-1}$

**3,4,6-tri-O-benzyl-2-deoxy-2-fluoro-α,β-D-glucopyranose (13).**

To a solution of bromide 12 (1.08 g, 2.01 mmol) and water (0.1 mL, 0.56 mmol) in acetone (10 mL), was added, with stirring at 0°C, silver carbonate (635 mg, 2.3 mmol) in small portions. After stirring for 2 h at room temperature, the mixture was heated to 60°C for 1 h and filtered through celite. Removal of solvent and purification by silica chromatography with cyclohexane:ethyl acetate (6:4) as mobile phase, afforded compound 13 (578 mg, 61%) as a solid corresponding to a mixture of α- and β-anomers (85/15):

$C_{27}H_{29}FO_5$
M = 452.52 $g.mol^{-1}$

The reaction of 8, 9 and 13 with chlorambucil, activated by the N,N'-dicyclohexylcarbodiimide (DCC) procedure, using either 4-(N,N-dimethylamino)pyridine (DMAP) or hydroxybenzotriazole (HOBt), gave the corresponding esters 14 and amides 15.

**Example** 1

**3,4,6-tri-O-acetyl-1-[4-{4-[bis(2-chloroethyl)amino]phenyl}butyrate]-2-deoxy-2-fluoro-α,β-D-glucopyranose (14a) (see Scheme 1a).**

To a solution of 3,4,6-tri-O-acetyl-2-deoxy-2-fluoro-α,β-D-glucopyranose 9 (330 mg, 1.07 mmol) in $CH_2Cl_2$ (50 mL), chlorambucil (490 mg, 1.61 mmol), DCC (376 mg, 1.78 mmol) and DMAP (8.7 mg, 0.071 mmol) were added and stirred at room temperature for 22 h. The solid was removed by filtration, and the filtrate was washed consecutively with a 1N aqueous solution of acetic acid and water, and dried over $MgSO_4$, and concentrated under vacuum. The resulting residue was purified by silica gel chromatography, using cyclohexane:ethyl acetate (7:3) as eluent, to yield 14a (360 mg, 57%) as a syrup (α/β ratio = 25/75):

$C_{26}H_{34}Cl_2FNO_9$
M = 594.46 g.mol$^{-1}$
Rf = 0.38 (cyclohexane:ethyl acetate 6:4)
IR ($CCl_4$ solution) ν 1763 ($\nu_{c=o}$), 1366 ($\delta_{sCH3}$), 1240, 1223, 1075, 1038 ($\nu_{c-o}$) cm$^{-1}$;
$^1$H NMR (200 MHz, $CDCl_3$) δ 7.12 (d, 2H, $J_o$ = 8.7 Hz, $H_{Ar}$α), 7.10 (d, 2H, $J_o$ = 8.7 Hz, $H_{Ar}$β), 6.69 (d, 2H, $H_{Ar}$α), 6.67 (d, 2H, $H_{Ar}$β), 6.50 (d, 1H, $J_{1,2}$ = 3.9 Hz, H-1α), 5.85 (dd, 1H, $J_{1,2}$ = 8.1 Hz, $J_{1,F}$ = 3.1 Hz, H-1β), 5.60 (td, 1H, $J_{2,3}$ = $J_{3,4}$ = 9.6 Hz, $J_{3,F}$ = 12.1 Hz, H-3α), 5.43 (td, 1H, $J_{2,3}$ = $J_{3,4}$ = 9.2 Hz, $J_{3,F}$ = 14.2 Hz, H-3β), 5.14 (t, 1H, $J_{4,5}$ = 9.8 Hz, H-4α), 5.12 (t, 1H, $J_{4,5}$ = 9.7 Hz, H-4β), 4.70 (ddd, 1H, $J_{2,F}$ = 48.6 Hz, H-2α), 4.49 (td, 1H, $J_{2,F}$ = 51.1 Hz, H-2β), 4.40-4.30 (m, 2H, H-6aα, H-6aβ), 4.16-4.04 (m, 3H, H-6bα, H-6bβ, H-5α), 3.90 (ddd, 1H, H-5β), 3.75-3.64 (m, 16H, $N(CH_2CH_2Cl)_2$ α+β), 2.62 (m, 4H, C$\underline{H}_2$Ph α+β), 2.48 (m, 4H, $COCH_2$ α+β), 2.10-1.94 (m, 22H, OAc, $CH_2C\underline{H}_2CH_2$ α+β);
$^{13}$C NMR (50 MHz, $CDCl_3$) δ 171.55, 171.38, 170.59, 170.18, 169.94, 169.61, 169.54 (CO α+β); 144.63, 144.59 ($C_{Ar}$N α+β), 130.31, 130.16 ($\underline{C}_{Ar}CH_2$ α+β), 129.91, 129.87, 112.35 ($CH_{Ar}$ α+β), 91.30 (C-1β, $J_{1,F}$= 24.1 Hz), 88.39 (C-2β, $J_{2,F}$ = 190.9 Hz), 88.31 (C-1α, $J_{1,F}$ = 22.2 Hz), 86.40 (C-2α, $J_{2,F}$ = 193.7 Hz), 72.90 (C-5β), 72.87 (C-3β, $J_{3,F}$ = 19.4 Hz), 70.73 (C-3α, $J_{3,F}$ = 19.4 Hz), 69.71 (C-5α), 67.76 (C-4α/β, $J_{4,F}$ = 7.2 Hz), 65.47 (C-4α/β, $J_{4,F}$ = 7.6 Hz), 61.46 (C-6 α+β), 53.75 ($CH_2N$ α+β), 40.57 ($CH_2Cl$ a+(3), 33.82, 33.31 ($\underline{C}H_2Ph$ α+β, $CO\underline{C}H_2$ α+β), 26.54, 26.46 ($CH_2\underline{C}H_2CH_2$ α+β), 20.78, 20.72, 20.64 ($CH_3$ α+β);

MS (ESI) m/z 594.24 [M+1]$^+$.

**Example 2**

**3,4,6-tri-O-benzyl-1-[4-{4-[bis(2-chloroethyl)amino]phenyl}butyrate]-2-deoxy-2-fluoro-α,β-D-glucopyranose (14b) (see scheme 1a).**

To a solution of 3,4,6-tri-O-benzyl-2-deoxy-2-fluoro-α,β-D-glucopyranose 13 (200 mg, 0.44 mmol) in $CH_2Cl_2$ (5 mL), chlorambucil (201 mg, 0.66 mmol), DCC (136 mg, 0.66 mmol) and DMAP (5 mg, 0.044 mmol) were added and stirred 17 h at room temperature. The solid was removed by filtration, and the filtrate was concentrated under vacuum. The resulting residue was purified by silica gel chromatography, using cyclohexane:ethyl acetate (85:15) as mobile phase, to yield 14b (319 mg, 98%) as a syrup (α/β ratio = 70/30):

$C_{41}H_{46}Cl_2FNO_6$
M = 738.72 g.mol$^{-1}$
Rf= 0.33 (petroleum benzine:ethyl acetate 85:15)
IR (NaCl) ν 3064, 3031 ($\nu_{CHarom}$), 1754 ($\nu_{C=O}$), 1117, 1052, 1027 ($\nu_{C-O}$) cm$^{-1}$;
$^1$H NMR (500 MHz, $CDCl_3$) δ 7.38-7.15 (m, 30H, $H_{Ar}$), 7.10 (d, 4H, $J_o$ = 8.5 Hz, $H_{Ar}$), 6.76 (d, 2H, $J_o$, $H_{Ar}$β), 6.74 (d, 2H, $J_o$ = 8.0 Hz, $H_{Ar}$α), 6.42 (d, 1H, $J_{1,2}$ = 3.9 Hz, H-1α), 5.73 (dd, 1H, $J_{1,F}$ = 3.1 Hz, $J_{1,2}$ = 8.1 Hz, H-1β), 4.91 (d, 1H, J = 11.1 Hz, OC$\underline{H}_2$Ph α+β), 4.86 (d, 1H, $J$ = 10.5 Hz, OC$\underline{H}_2$Ph α), 4.84 (d, 1H, OC$\underline{H}_2$Ph β), 4.78 (d, 1H, $J$= 11.1 Hz, OC$\underline{H}_2$Ph α), 4.77 (d, 1H, $J$ = 11.2 Hz, OC$\underline{H}_2$Ph β), 4.67 (ddd, 1H, $J_{2,3}$ = 9.3 Hz, $J_{2,F}$ = 48.8 Hz, H-2α), 4.62 (d, 1H, $J$ = 11.3 Hz, OC$\underline{H}_2$Ph β), 4.61 (d, 1H, $J$ = 12.3 Hz, OC$\underline{H}_2$Ph α), 4.52 (d, 2H, $J$ = 10.7 Hz, OC$\underline{H}_2$Ph α+β), 4.50 (d, 1H, $J$ = 12.1 Hz, OC$\underline{H}_2$Ph α), 4.48 (d, 1H, J = 11.9 Hz, OC$\underline{H}_2$Ph β), 4.48 (td, 1H, $J_{2,3}$ = 8.2 Hz, $J_{2,F}$ = 51.1 Hz, H-2β), 4.06 (td, 1H, $J_{3,4}$ = 9.0 Hz, $J_{3,F}$ = 12.3 Hz, H-3α), 3.86-3.61 (m, 25H, H-3β, H-4 α+β, H-5 α+β, 2H-6 α+β,

$N(CH_2CH_2Cl)_2$ α+β), 2.62-2.58 (m, 4H, $CH_2C\underline{H}_2Ph$ α+β), 2.45-2.38 (m, 4H, $COCH_2$ α+β), 1.94 (qt, 4H, *J* = 7.5 Hz, $CH_2C\underline{H}_2CH_2$ α+β);
$^{13}C$ NMR (50 MHz, $CDCl_3$) δ 171.97, 171.70 (CO α+β), 144.42 ($C_{Ar}N$ α+β), 138.23, 137.92 ($C_{ipso}$ α+β), 130.58 ($\underline{C}_{Ar}CH_2$ α+β), 129.84, 128.55, 128.14, 128.03, 112.45 ($CH_{Ar}$α+β), 92.21 (C-2β, $J_{2,F}$ = 187.4 Hz), 91.59 (C-1β, $J_{1,F}$ = 24.6 Hz), 90.27 (C-2α, $J_{2,F}$ = 190.6 Hz), 89.23 (C-1α, $J_{1,F}$ = 22.7 Hz), 83.21 (C-3β, $J_{3,F}$ = 16.0 Hz), 80.66 (C-3α, $J_{3,F}$ = 16.0 Hz), 76.25 (C-4α/β, $J_{4,F}$ = 8.8 Hz), 75.79 (C-5β), 75.55, 75.27, 75.20, 75.17, 73.69 ($O\underline{C}H_2Ph$ α+β), 72.79 (C-5α), 67.98 (C-6 α+β), 53.77 ($CH_2N$ α+β), 40.57 ($CH_2Cl$ α+β), 33.88, 33.59, 33.47 ($CH_2\underline{C}H_2Ph$ α+β, $CO\underline{C}H_2$ α+β), 26.60 ($CH_2\underline{C}H_2CH_2$ α+β).

**Example 3**

**1-[4-{4-[bis(2-chloroethyl)amino]phenyl}butyrate]-2-deoxy-2-fluoro-α,β-D-glucopyranose (14c) (see scheme 1a).**

To a suspension of 10% palladium on charcoal (1.5 g) in THF/EtOH (12/24 mL) was added the benzylated glycoside 14b (1.78 g, 2.41 mmol), followed by ammonium formate (850 mg, 13.5 mmol). The mixture was refluxed for 3.5 h and then allowed to cool to room temperature, filtered on celite, and concentrated under vacuum. The crude product was purified by silica gel chromatography, eluting with cyclohexane:ethyl acetate (1:9), to yield the debenzylated product 14c (400 mg, 35%) as a brown oil (α/β ratio = 55/45):

$C_{20}H_{28}Cl_2FNO_6$
M = 468.35 $g.mol^{-1}$
Rf = 0.49 (cyclohexane:ethyl acetate 1:9)
IR(NaCl) ν 3418 ($ν_{OH}$), 1738 ($ν_{C=O}$), 1078 ($ν_{C-O}$) $cm^{-1}$;
$^1H$ NMR (200 MHz, acetone-$d_6$) δ 7.18 (d, 4H, $J_o$ = 8.7 Hz, $H_{Ar}$ α+β), 6.81 (d, 4H, $H_{Ar}$ α+β), 6.41 (d, 1H, $J_{1,2}$ = 3.9 Hz, H-1α), 5.85 (dd, 1H, $J_{1,2}$ = 8.1 Hz, $J_{1,F}$ = 3.1 Hz, H-1β), 5.02 (br s, 1H, OH), 4.90 (br s, 1H, OH), 4.71 (br s, 1H, OH), 4.53 (ddd, 1H, $J_{2,3}$ = 9.4 Hz, $J_{2,F}$ = 48.7 Hz, H-2α), 4.25 (td, 1H, $J_{2,3}$ = 8.5 Hz, $J_{2,F}$ = 52.0 Hz, H-2β), 4.19-3.49 (m, 26H, $N(CH_2CH_2Cl)_2$ α+β, H-3 α+β, H-4 α+β, H-5 α+β, 2H-6 α+β), 2.65 (t, 4H, *J* = 7.6 Hz, $C\underline{H}_2Ph$ α+β), 2.55-2.41 (m, 4H, $COCH_2$ α+β), 2.00-1.93 (m, 4H, $CH_2C\underline{H}_2CH_2$ α+β);
$^{13}C$ NMR (50 MHz, acetone-$d_6$) δ 171.86, 171.81 (CO α+β), 145.25 ($C_{Ar}N$ α+β), 130.57 ($C_{Ar}CH_2$ α+β), 129.96, 112.70 ($CH_{Ar}$ α+β), 92.02 (C-2β, $J_{2,F}$ = 184.2 Hz), 92.00 (C-1β, $J_{1,F}$ = 21.7 Hz), 89.88 (C-2α, $J_{2,F}$ = 184.5 Hz), 89.47 (C-1α, $J_{1,F}$ = 22.6 Hz), 78.00 (C-5β), 75.43 (C-3β, $J_{3,F}$ = 16.6 Hz), 75.18 (C-5α), 72.51 (C-3α, $J_{3,F}$ = 16.8 Hz), 70.44 (C-4α/β, $J_{4,F}$ = 8.0 Hz), 70.16 (C-4α/β, $J_{4,F}$ = 8.1 Hz), 61.62, 61.57 (C-6 α+β), 53.47 ($CH_2N$ α+β), 41.20 ($CH_2Cl$ α+β), 33.99, 33.92, 33.58, 33.37 ($\underline{C}H_2Ph$ α+β, $CO\underline{C}H_2$ α+β), 27.20, 27.05 ($CH_2\underline{C}H_2CH_2$ α+β).
MS (ESI) m/z 468.54 $[M+1]^+$.

**Example 4**

**N-(3,4,6-tri-O-acetyl-2-deoxy-2-fluoro-β-D-glucopyranosyl)-4-{4-[bis(2-chloroethyl)amino]phenyl}butanamide (15a) (see scheme 1b).**

Amino-sugar 8 (189 mg, 0.61 mmol) was dissolved in anhydrous DMF (10 mL) and chlorambucil (187 mg, 0.61 mmol), DCC (127 mg, 0.61 mmol), and HOBt (92 mg, 0.67 mmol) were added. The solution was stirred for 20 h at room temperature, filtered, concentrated in vacuo, and purified by silica gel chromatography (cyclohexane:ethyl acetate 6:4) to give 15a (184 mg, 51%) as a white solid:

$C_{26}H_{35}Cl_2FN_2O_8$
M = 593.48 $g.mol^{-1}$
Rf = 0.25 (cyclohexane:ethyl acetate 6:4)
IR (KBr) ν 3327 ($ν_{NH}$), 1750 ($ν_{C=Oester}$), 1676 ($ν_{C=Oamide}$), 1520 ($δ_{NH}$), 1244, 1069, 1032 ($ν_{C-O}$) $cm^{-1}$;
$^1H$ NMR (200 MHz, $CDCl_3$) δ 7.08 (d, 2H, $J_o$ = 8.6 Hz, $H_{Ar}$), 6.69 (d, 2H, $H_{Ar}$), 6.04 (d, 1H, *J* = 9.3 Hz, NH), 5.44-5.28 (m, 2H, H-3, H-1), 5.03 (t, 1H, $J_{3,4}$ = $J_{4,5}$ = 9.7 Hz, H-4), 4.31 (dd, 1H, $J_{5,6}$ = 4.4 Hz, $J_{6a,6b}$ = 12.5 Hz, H-6a), 4.26 (td, 1H, $J_{2,F}$ = 50.8 Hz, $J_{1,2}$ = $J_{2,3}$ = 9.2 Hz, H-2), 4.06 (dd, 1H, $J_{5,6b}$ = 2.0 Hz, H-6b), 3.86 (ddd, 1H, $H_5$), 3.76-3.58 (m, 8H, $N(CH_2CH_2Cl)_2$), 2.58 (t, 2H, *J* = 7.3 Hz, $C\underline{H}_2Ph$), 2.25 (t, 2H, *J* = 7.3 Hz, $COCH_2$), 2.08, 2.07, 2.04 (each s, 3×3H, OAc), 1.94 (m, 2H, $CH_2C\underline{H}_2CH_2$);
$^{13}C$ NMR (50 MHz, $CDCl_3$): δ 173.24, 170.68, 169.92, 169.85 (CO), 144.13 ($C_{Ar}N$), 131.00 ($\underline{C}_{Ar}CH_2$), 129.88, 112.70 ($CH_{Ar}$), 88.50 (C-2, $J_{2,F}$ = 191.2 Hz), 77.62 (C-1), 73.78 (C-5), 73.50 (C-3, $J_{3-F}$ = 19.3 Hz), 67.93 (C-4, $J_{4-F}$ = 7.0 Hz), 61.65 (C-6), 53.89 ($CH_2N$), 40.45 ($CH_2Cl$), 35.68 ($COCH_2$), 33.89 ($\underline{C}H_2Ph$), 26.77 ($CH_2\underline{C}H_2CH_2$), 20.82, 20.76,

20.70 ($CH_3$);
MS (ESI) m/z 593.30 $[M+1]^+$.

**Example 5**

**4-{4-[bis(2-chloroethyl)amino]phenyl}-N-(2-deoxy-2-fluoro-β-D-glucopyranosyl)butanamide (15b) (see scheme 1b).**

To a stirred solution of acetylated compound 15a (140 mg, 0.24 mmol) in methanol (7 mL) was added sodium methoxide (1 mg). After 4 h at room temperature, the mixture was evaporated to dryness and purified on silica gel (10: 90 to 0:100 cyclohexane:ethyl acetate gradient) to afford 15b (63 mg, 57%) as a solid:

$C_{20}H_{29}Cl_2FN_2O_5$
M = 467.37 $g.mol^{-1}$
mp 168°C
Rf = 0.11 (cyclohexane:ethyl acetate 1:9)
IR (KBr) ν 3310 ($\nu_{NH}$, $\nu_{OH}$), 1655 ($\nu_{C=O}$), 1519 ($\delta_{NH}$), 1092, 1057 ($\nu_{C-O}$) $cm^{-1}$;
$^1H$ NMR (200 MHz, acetone-$d_6$) δ 7.81 (d, 1H, $J$ = 9.1 Hz, NH), 7.06 (d, 2H, $J_o$ = 8.8 Hz, $H_{Ar}$), 6.70 (d, 2H, $H_{Ar}$), 5.19 (td, 1H, $J_{1,2}$ = $J_{1\text{-}NH}$ = 9.2 Hz, $J_{1,F}$ = 2.3 Hz, H-1), 4.77 (d, 1H, $J$ = 4.4 Hz, OH), 4.39 (br s, 1H, OH), 4.06 (td, 1H, $J_{2,3}$ = 9.0 Hz, $J_{2,F}$ = 50.9 Hz, H-2), 3.80-3.58 (m, 12H, $N(CH_2CH_2Cl)_2$, 2H-6, H-3, H-4), 3.63-3.32 (m, 2H, OH, H-5), 2.51 (t, 2H, $J$ = 7.6 Hz, $C\underline{H_2}Ph$), 2.21 (t, 2H, $J$ = 7.9 Hz, $COCH_2$), 1.84 (qt, 2H, $CH_2C\underline{H_2}CH_2$);
$^{13}C$ NMR (50 MHz, acetone-$d_6$) δ 173.47 (NHCO), 145.55 ($C_{Ar}N$), 131.43 ($\underline{C_{Ar}}CH_2$), 130.36, 113.07 ($CH_{Ar}$), 92.42 (C-2, $J_{2,F}$ = 183.6 Hz), 79.10 (C-5), 78.06 (C-1, $J_{1,F}$ = 23.2 Hz), 76.76 (C-3, $J_{3,F}$ = 16.8 Hz), 71.51 (C-4, $J_{4,F}$ = 7.7 Hz), 62.47 (C-6), 53.89 ($CH_2N$), 41.62 ($CH_2Cl$), 36.02 ($CO\underline{C}H_2$), 34.70 ($\underline{C}H_2Ph$), 28.07 ($CH_2\underline{C}H_2CH_2$);
MS (ESI) m/z 467.15 $[M+1]^+$.

The acetylated compound 17a was obtained from chlorambucil in two steps comprising the formation of the isocyanate 16 and its reaction with β-amino-sugar 8.

**Example 6**

**N-(3,4,6-tri-O-acetyl-2-deoxy-2-fluoro-β-D-glucopyranosyl)-N'-{3-(4-[bis(2-chloroethyl)amino]phenyl)propyl} urea (17a) (see scheme 1c).**

**a) N-[bis(2-chloroethyl)1-4-(3-isocyanatopropyl)phenylamine (16)**

This compound was prepared according to the procedure described by Valu et al. (J. Med. Chem., 1990, 33, 3014-3019) to a solution of chlorambucil 2 (500 mg, 1.65 mmol) in acetone (6 mL) cooled to -5°C, was added dropwise $NEt_3$ (0.25 mL, 1.81 mmol). Then the solution was treated with ethylchloroformate (0.17 mL diluted in 2 mL acetone, 1.81 mmol) and ten minutes later, sodium azide (0.21 g, 3.23 mmol) in 1 ml water was added. Stirring is maintened in an ice cold bath for 30 min, then the mixture was poured into ice water (300 mL) and extracted with toluene (2 × 120 mL). The combined, dried, organic layers were heated under reflux for 1.5 h. After evaporation of the solvent under reduced pressure, compound 36 (400 mg, 81 %) was obtained as an orange-coloured oily liquid, directly used for the next step.

$C_{14}H_{18}Cl_2N_2O$
M = 301.21 $g.mol^{-1}$
Rf = 0.30 (petroleum benzine:ethyl acetate 4:6)
IR (NaCl) ν 2276 ($\nu_{N=C=O}$) $cm^{-1}$;
$^1H$ NMR (200 MHz, $CDCl_3$) δ 7.07 (d, 2H, $J_o$ = 8.7 Hz, $H_{Ar}$), 6.64 (d, 2H, $H_{Ar}$), 3.76-3.58 (m, 8H, $N(CH_2CH_2Cl)_2$), 3.30 (t, 2H, $J$ = 6.6 Hz, $C\underline{H_2}NCO$), 2.63 (t, 2H, $J$ = 7.4 Hz, $C\underline{H_2}Ph$), 1.88 (qt, 2H, $CH_2C\underline{H_2}CH_2$);
$^{13}C$ NMR (50 MHz, $CDCl_3$) δ 144.58 (CO), 129.76, 112.37 ($CH_{Ar}$), 53.67 ($\underline{C}H_2N$), 42.22, 40.59 ($CH_2Cl$, $\underline{C}H_2NCO$), 32.92, 31.49 ($\underline{C}H_2Ph$, $CH_2\underline{C}H_2CH_2$).

**b) N-(3,4,6-tri-O-acetyl-2-deoxy-2-fluoro-β-D-glucopyranosyl)-N'-{3-(4-[bis(2-chloroethyl)amino]phenyl)propyl}urea (17a)**

To amino-sugar 8 (200 mg, 0.65 mmol) dissolved in $CH_2Cl_2$/THF (1.5/2 mL) was added isocyanate 16 (215

mg, 0.72 mmol) in $CH_2Cl_2$ (0.5 mL), and the mixture was stirred overnight at room temperature. After evaporation of the solvent, the concentrate was purified by column chromatography on silica gel with ethyl acetate:petroleum benzine (6:4 to 7:3 gradient) as developing solvent to give compound 17a (200 mg, 50%) as a red powder:

$C_{26}H_{36}Cl_2FN_3O_8$
M = 608.49 $g.mol^{-1}$
Rdt = 50 %
mp 91 °C
Rf = 0.40 (petroleum benzine:ethyl acetate 4:6)
IR (NaCl) ν 1746 ($\nu_{C=Oester}$), 1364 ($\delta_{sCH3}$), 1230, 1029 ($\nu_{C-O}$) $cm^{-1}$;
$^1H$ NMR (200 MHz, $CDCl_3$) δ 7.06 (d, 2H, $J_{o=}$ 8.6 Hz, $H_{Ar}$), 6.63 (d, 2H, $H_A$r), 5.46-5.23 (m, 2H, H-1, H-3), 5.01 (t, 1H, $J_{3,4}$ = $J_{4,5}$ = 9.7 Hz, H-4), 4.84 (br t, 1H, $J$ = 5.1 Hz, N<u>H</u>$CH_2$), 4.33 (dd, 1H, $J_{6a,6b}$ = 12.5 Hz, $J_{5,6a}$ = 4.3 Hz, H-6a), 4.20 (td, 1H, $J_{2-F}$ = 50.5 Hz, $J_{1,2}$ = $J_{2,3}$ = 9.2 Hz, H-2), 4.06 (dd, 1H, $J_{5,6b}$ = 2.1 Hz, H-6b), 3.82 (ddd, 1H, H-5), 3.65 (m, 8H, $N(CH_2CH_2Cl)_2$), 3.23 (m, 2H, NHC<u>$H_2$</u>), 2.55 (t, 2H, J = 7.4 Hz, C<u>$H_2$</u>Ph), 2.09, 2.07, 2.05 (each s, 3×3H, OAc), 1.78 (qt, 2H, $CH_2$C<u>$H_2$</u>$CH_2$);
$^{13}C$ NMR (50 MHz, $CDCl_3$) δ 170.76, 170.03, 169.91 (<u>C</u>$OCH_3$), 156.84 (NH<u>C</u>O), 144.43 ($C_{Ar}$N), 130.53 (<u>$C_{Ar}$</u>$CH_2$), 129.63, 112.29 ($CH_{Ar}$), 88.54 (C-2, $J_{2,F}$= 190.6 Hz), 79.59 (C-1, $J_{1,F}$= 22.2 Hz), 73.67 (C-3, $J_{3,F}$= 19.5 Hz), 73.20 (C-5), 68.15 (C-4, $J_{4,F}$= 7.1 Hz), 61.72 (C-6), 53.63 ($CH_2N$), 40.61 ($CH_2Cl$), 40.03 ($NHCH_2$), 31.99, 31.87 (<u>C</u>$H_2$Ph, $CH_2$<u>C</u>$H_2CH_2$), 20.73 ($CH_3$);
MS (ESI) m/z 608.19 $[M+1]^+$.

**<u>Example 8</u>**

**<u>N-{3-(4-[bis(2-chloroethyl)amino]phenyl)propyl}-N'-(2-deoxy-2-fluoro-β-D-glucopyranosyl)urea (17b) (see scheme 1c).</u>**

To a stirred solution of acetylated compound 17a (200 mg, 0.33 mmol) in methanol (12 mL) was added sodium methoxide (2 mg) and the mixture was stirred at room temperature for 3 h. After neutralization with IRC 50 Amberlite ion-exchange resin (H+), filtration and evaporation to dryness, the residue was purified on silica gel (10% methanol in ethyl acetate) to yield compound 17b (75 mg, 45%) as an oil:

$C_{20}H_{30}Cl_2FN_3O_5$
M = 482.38 $g.mol^{-1}$
Rf = 0.15 (ethyl acetate:methanol 9:1)
IR (KBr) ν 3348 ($\nu_{OH}$), 1750 ($\nu_{C=O}$), 1078, 1028 ($\nu_{C-O}$) $cm^{-1}$;
$^1H$ NMR (200 MHz, acetone-$d_6$) δ 7.09 (d, 2H, $J_o$ = 8.8 Hz, $H_{Ar}$), 6.72 (d, 2H, $H_{Ar}$), 6.34 (d, 1H, $J$ = 9.5 Hz, $C_1$NHCO), 5.80 (t, 1H, J = 5.8 Hz, N<u>H</u>$CH_2$), 5.09 (td, 1H, $J_{1,2}$ = 9.3 Hz, $J_{1,F}$ = 2.0 Hz, H-1), 4.80 (d, 1H, $J$ = 4.4 Hz, OH), 4.43 (d, 1H, $J$ = 4.4 Hz, OH), 4.02 (td, 1H, $J_{2,F}$ = 50.9 Hz, $J_{2,3}$ = 8.9 Hz, H-2), 3.84-3.67 (m, 12H, $N(CH_2CH_2Cl)_2$, H-3, H-4, 2H-6), 3.40-3.36 (m, 2H, H-5, OH), 3.16 (t, 2H, $J$ = 6.4 Hz, NHC<u>$H_2$</u>), 2.53 (t, 2H, $J$= 7.7 Hz, C<u>$H_2$</u>Ph), 1.74 (qt, 2H, $CH_2$C<u>$H_2$</u>$CH_2$);
$^{13}C$ NMR (50 MHz, acetone-$d_6$) δ 158.49 (NHCO), 145.38 ($C_{Ar}$N), 131.37 (<u>$C_{Ar}$</u>$CH_2$), 130.23, 113.01 ($CH_{Ar}$), 92.30 (C-2, $J_{2,F}$ = 195.0 Hz), 79.97 (C-1, $J_{1,F}$ = 25.0 Hz), 78.50 (C-5), 76.73 (C-3, $J_{3,F}$ = 16.9 Hz), 71.39 (C-4, $J_{4,F}$ = 7.2 Hz), 62.36 (C-6), 53.84 ($CH_2N$), 41.62 ($CH_2Cl$), 40.27 ($NHCH_2$), 32.84, 32.55 (<u>C</u>$H_2$Ph, $CH_2$<u>C</u>$H_2CH_2$);
MS (ESI) m/z 482.16 $[M+1]^+$.

For the preparation of compounds 20b, 21b and 22b for which 4-aminobenzamide (20b), 2-aminoethanamide (21b) or 4-aminobutanamide (22b) was used as a spacer arm between the sugar residue and chlorambucil, the synthetic methods depicted in Scheme 2b have been adopted.

**<u>Example 9</u>**

**<u>N-(3,4,6-tri-O-acetyl-2-deoxy-2-fluoro-β-D-glucopyranosyl)-4-(4-{4-[bis(2-chloroethyl)amino]phenyl}butanamido)benzamide (20a) (see scheme 2a).</u>**

**a) <u>N-(3,4,6-tri-O-acetyl-2-deoxy-2-fluoro-β-D-glucopyranosyl)-4-nitrobenzamide (18a).</u>**

To a stirred solution of the amino-sugar 8 (200 mg, 0.65 mmol) and triethylamine (274 μL, 1.95 mmol) in anhydrous THF (2 mL) stirred over 4Å molecular sieves, was added dropwise a solution of 4-nitrobenzoyl chloride (181

mg, 0.977 mmol) in anhydrous THF (1 mL). The mixture was stirred at 0°C for 30 min and at room temperature for 4 h. After evaporation, the residue was diluted with ethyl acetate and washed consecutively with 1N aqueous HCl, saturated aqueous $NaHCO_3$ and water. Then the solution was dried over $MgSO_4$ and concentrated under reduced pressure. The resulting residue was purified by silica gel chromatography, using petroleum benzine:ethyl acetate (6:4) as eluent, to yield 18a (200 mg, 67%) as a colourless oil:

$C_{19}H_{21}FN_2O_{10}$
M = 456.38 g.mol$^{-1}$
Rf= 0.46 (petroleum benzine:ethyl acetate 6:4)
IR (NaCl) ν 3368 ($\nu_{NH}$), 1747 ($\nu_{C=Oester}$), 1687 ($\nu_{C=Oamide}$), 1531 ($\nu_{asNO2}$, $\delta_{NH}$), 1368 ($\delta_{sCH3}$), 1350 ($\nu_{sNO2}$), 1231, 1069, 1038 ($\nu_{C-O}$) cm$^{-1}$;
$^1$H NMR (200 MHz, $CDCl_3$) δ 8.30 (d, 2H, $J_o$ = 8.8 Hz, $H_{Ar}$), 8.06 (d, 2H, $H_{Ar}$), 7.68 (d, 1H, $J$ = 9.0 Hz, NH), 5.65 (td, 1H, $J_{1,2}$ = 9.0 Hz, $J_{1,F}$ = 1.8 Hz, H-1), 5.47 (td, 1H, $J_{2,3}$ = $J_{3,4}$ = 9.8 Hz, $J_{3,F}$ = 13.7 Hz, H-3), 5.05 (t, 1H, $J_{4,5}$ = 9.8 Hz, H-4), 4.65 (td, 1H, $J_{2,F}$ = 50.2 Hz, H-2), 4.44 (dd, 1H, $J_{6a,6b}$ = 12.6 Hz, $J_{5,6a}$ = 4.1 Hz, H-6a), 4.07 (dd, 1H, $J_{5,6a}$ = 1.6 Hz, H-6b), 3.97 (ddd, 1H, H-5), 2.11, 2.09, 2.05 (each s, 3×3H, OAc);
$^{13}$C NMR (50 MHz, $CDCl_3$) δ 170.80, 170.47, 169.87 ($\underline{C}OCH_3$), 165.84 (NHCO), 150.22 ($C_{Ar}NO_2$), 138.58 ($\underline{C}_{Ar}CO$), 128.85, 123.97 ($CH_{Ar}$), 88.37 (C-2, $J_{2,F}$ = 191.2 Hz), 78.18 (C-1, $J_{1,F}$ = 22.8 Hz), 73.99 (C-5), 73.42 (C-3, $J_{3,F}$ = 19.2 Hz), 68.01 (C-4, $J_{4,F}$ = 7.0 Hz), 61.51 (C-6), 20.81, 20.68 ($CH_3$).

**b) N-(3,4,6-tri-O-acetyl-2-deoxy-2-fluoro-β-D-glucopyranosyl)-2-(N-benzyloxycarbonylamino)acetamide (18b).**

Amino-sugar 8 (2.06 g, 6.72 mmol) was dissolved in anhydrous DMF (100 mL) and N-Cbz-glycine (1.69 g, 8.06 mmol), DCC (1.66 g, 8.06 mmol), and HOBt (1.09 g, 8.06 mmol) were added. The solution was stirred overnight at room temperature, filtered and concentrated in vacuo. The addition of $CH_2Cl_2$ and a new filtration allowed the elimination of additional DCU. Evaporation of the filtrate followed by purification on silica gel (6:4 to 4:6 petroleum benzine:ethyl acetate gradient) gave compound 18b (2.29 g, 69%) as a yellowish oil:

$C_{22}H_{27}FN_2O_{10}$
M = 498.46 g.mol$^{-1}$
Rf = 0.41 (petroleum benzine:ethyl acetate 5:5)
IR (KBr) ν 3400 ($\nu_{NH}$), 1749 ($\nu_{C=O}$), 1384 ($\delta_{sCH3}$), 1231,1036 ($\nu_{C-O}$) cm$^{-1}$;
$^1$H NMR (200 MHz, $CDCl_3$) δ 7.36 (m, 5H, $H_{Ar}$), 7.19 (d, 1H, $J$ = 9.2 Hz, $C_1NH$), 5.48 (m, 1H, $CH_2N\underline{H}CO$), 5.44-5.23 (m, 2H, H-1, H-3), 5.14 (s, 2H, $OC\underline{H}_2Ph$), 5.03 (t, 1H, $J_{3,4}$ = $J_{4,5}$ = 9.8 Hz, H-4), 4.31 (td, 1H, $J_{1,2}$ = $J_{2,3}$ = 9.0 Hz, $J_{2,F}$ = 50.4 Hz, H-2), 4.29 (dd, 1H, $J_{5,6a}$ = 4.3 Hz, $J_{6a,6b}$ = 12.5 Hz, H-6a), 4.06 (dd, 1H, $J_{5,6b}$ = 2.1 Hz, H-6b), 3.95 (d, 2H, $J$ = 5.1 Hz, $COC\underline{H}_2NH$), 3.83 (ddd, 1H, H-5), 2.08, 2.06, 2.04 (each s, 3×3H, OAc).

**c) N-(3,4,6-tri-O-acetyl-2-deoxy-2-fluoro-β-D-glucopyranosyl)-3-azidopropanamide (18c).**

A stirred solution of 4-azidobutyric acid (354 mg, 2.74 mmol) in $CH_2Cl_2$ (15 mL) was treated with triethylamine (460 μL, 3.29 mmol). The mixture was cooled to 0°C, treated with ethyl chloroformate (275 μL, 2.88 mmol), and stirred at ambient temperature for 30 min. Then, amino-sugar 8 (561 mg, 1.83 mmol) was added, and the mixture was stirred one day at ambient temperature. The mixture was washed with saturated aqueous $Na_2CO_3$ and extracted three times with $CH_2Cl_2$. The organic extracts were combined, dried over $MgSO_4$, and concentrated under reduced pressure. The crude product was purified by column chromatography using petroleum benzine:ethyl acetate (4:6) as the eluent, to give compound 18c (660 mg, 86%) as a solid:

$C_{16}H_{23}FN_4O_8$
M = 418.38 g.mol$^{-1}$
mp 88°C
Rf = 0.33 (petroleum benzine:ethyl acetate 5:5)
IR (NaCl) ν 3368 ($\nu_{NH}$), 2102 ($\nu_{asN3}$), 1750 ($\nu_{C=Oester}$), 1678 ($\nu_{C=Oamide}$), 1544 ($\delta_{NH}$), 1369 ($\delta_{sCH3}$), 1232, 1068, 1035 ($\nu_{C-O}$) cm$^{-1}$ ;
$^1$H NMR (200 MHz, $CDCl_3$) δ 7.05 (d, 1H, $J$ = 9.3 Hz, NH), 5.42 (m, 2H, H-1, H-3), 5.02 (t, 1H, $J_{3,4}$ = $J_{4,5}$ = 9.8 Hz, H-4), 4.40 (td, 1H, $J_{1,2}$ = $J_{2,3}$ = 9.1 Hz, $J_{2,F}$ = 50.3 Hz, H-2), 4.35 (dd, 1H, $J_{6a,6b}$ = 12.6 Hz, $J_{5,6a}$ = 4.2 Hz, H-6a), 4.06 (dd, 1H, $J_{5,6b}$ = 1.9 Hz, H-6b), 3.88 (ddd, 1H, H-5), 3.38 (t, 2H, $J$= 6.5 Hz, $CH_2N_3$), 2.38 (t, 2H, $J$= 7.1 Hz, $COCH_2$), 2.08, 2.07, 2.06 (each s, 3×3H, OAc), 1.91 (qt, 2H, $J$= 7.0 Hz, $CH_2C\underline{H}_2CH_2$);
$^{13}$C NMR (50 MHz, acetone-$d_6$) δ 172.45, 170.27, 169.75, 169.71 (CO), 89.14 (C-2, $J_{2,F}$ = 187.5 Hz), 77.53 (C-1, $J_{1,F}$ = 22.7 Hz), 73.87-73.50 (C-3, C-5), 68.60 (C-4, $J_{4,F}$ = 7.5 Hz), 62.23 (C-6), 50.94 ($CH_2N_3$), 32.79 ($CO\underline{C}H_2$),

24.67 ($CH_2\underline{C}H_2CH_2$), 20.19 ($CH_3$).

**d) 4-amino-N-(3,4,6-tri-O-acetyl-2-deoxy-2-fluoro-β-D-glucopyranosyl)benzamide (19a).**

To a suspension of 10% palladium on charcoal (25 mg) in THF/MeOH (7.5/7.5mL) was added the nitro compound 18a (100 mg, 0.22 mmol). The mixture was hydrogenated at atmospheric pressure for 3 h, filtered on celite, and concentrated to yield the reduced product 19a (86 mg, 91 %) as a light yellow solid which was used in the next step without further purification:

$C_{19}H_{23}FN_2O_8$
M = 426.40 g.mol$^{-1}$
mp 133˚C
Rf = 0.19 (petroleum benzine:ethyl acetate 4:6)
IR (NaCl) ν 3469, 3378 ($\nu_{NH}$), 1744 ($\nu_{C=Oester}$), 1630 ($\nu_{C=Oamide}$), 1604 ($\delta_{NH2}$), 1511 ($\delta_{NH}$), 1368 ($\delta_{sCH3}$), 1244, 1065, 1033 ($\nu_{C-O}$) cm$^{-1}$;
$^1$H NMR (200 MHz, $CDCl_3$) δ 7.69 (d, 2H, $J_o$ = 8.6 Hz, $H_{Ar}$), 7.26 (d, 1H, $J$ = 8.1 Hz, NH), 6.65 (d, 2H, $H_{Ar}$), 5.60 (td, 1H, $J_{1,2}$ = 9.3 Hz, $J_{1,F}$ = 1.8 Hz, H-1), 5.40 (td, 1H, $J_{2,3}$ = $J_{3,4}$ = 9.1 Hz, $J_{3,F}$ = 13.8 Hz, H-3), 5.05 (t, 1H, $J_{4,5}$ = 9.8 Hz, H-4), 4.52 (td, 1H, $J_{2,F}$ = 50.4 Hz, H-2), 4.34 (dd, 1H, $J_{6a,6b}$ = 12.5 Hz, $J_{5,6a}$ = 4.2 Hz, H-6a), 4.03 (dd, 1H, $J_{5,6b}$ = 1.9 Hz, H-6b), 3.84 (ddd, 1H, H-5), 2.07, 2.06, 2.04 (each s, 3×3H, OAc);
$^{13}$C NMR (50 MHz, $CDCl_3$) δ 170.86, 170.17, 170.05 ($\underline{C}OCH_3$), 167.55 (NHCO), 150.76 ($C_{Ar}NH_2$), 129.58 ($CH_{Ar}$), 122.23 ($\underline{C}_{Ar}CO$), 114.12 ($CH_{Ar}$), 88.42 (C-2, $J_{2,F}$ = 190.1 Hz), 78.15 (C-1, $J_{1,F}$ = 22.4 Hz), 73.90-73.57 (C-3, C-5), 68.08 (C-4, $J_{4,F}$ = 6.9 Hz), 61.66 (C-6), 20.76, 20.72 ($CH_3$).

**e) N-(3,4,6-tri-O-acetyl-2-deoxy-2-fluoro-β-D-glucopyranosyl)-2-aminoacetamide (19b).**

A solution of the N-Cbz protected derivative 18b (690 mg, 1.38 mmol) in THF/MeOH (5/5 mL) was stirred under hydrogen atmosphere in the presence of 10% palladium on charcoal (190 mg) for 20 h at room temperature. Filtration of the catalyst on celite and evaporation of the solvent yield the deprotected product 19b (465 mg, 92%) as a solid:

$C_{14}H_{21}FN_2O_8$
M = 364.33 g.mol$^{-1}$
mp 96˚C
Rf = 0.44 (ethyl acetate:ethanol 8:2)
IR (KBr) ν 3435 ($\nu_{NH}$), 1750 ($\nu_{C=O}$), 1370 ($\delta_{sCH3}$), 1239, 1069, 1036 ($\nu_{C-O}$) cm$^{-1}$;
$^1$H NMR (200 MHz, $CDCl_3$) δ 8.24 (d, 1H, $J$= 9.4 Hz, $C_1$NH), 5.47-5.34 (m, 2H, H-1, H-3), 5.06 (t, 1H, $J_{3,4}$ = $J_{4,5}$ = 9.8 Hz, H-4), 4.39 (td, 1H, $J_{1,2}$ = $J_{2,3}$ = 9.1 Hz, $J_{2,F}$ = 50.5 Hz, H-2), 4.30 (dd, 1H, $J_{5,6a}$ = 4.3 Hz, $J_{6a,6b}$ = 12.6 Hz, H-6a), 4.08 (dd, 1H, $J_{5,6b}$ = 2.1 Hz, H-6b), 3.87 (ddd, 1H, H-5), 3.44 (s, 2H, $C\underline{H_2}NH_2$), 2.09, 2.08, 2.05 (each s, 3×3H, OAc);
$^{13}$C NMR (50 MHz, $CDCl_3$) δ 172.84, 170.73, 170.00, 169.74 (CO), 88.52 (C-2, $J_{2,F}$ = 190.7 Hz), 77.30 (C-1, $J_{1,F}$ = 23.8 Hz), 73.79-73.38 (C-3, C-5), 68.05 (C-4, $J_{4,F}$ = 6.8 Hz), 61.78 (C-6), 44.16 ($CH_2NH_2$), 20.76, 20.67 ($CH_3$).

**f) 3-amino-N-(3,4,6-tri-O-acetyl-2-deoxy-2-fluoro-β-D-glucopyranosyl)propanamide (19c).**

To a suspension of 10% palladium on charcoal (75 mg) in $CH_2Cl_2$/MeOH (20/10 mL) was added the azido compound 18c (200 mg, 0.478 mmol). The mixture was hydrogenated at atmospheric pressure for 7 h, filtered on celite, and concentrated to yield the compound 19c (180 mg, 96%) as a white solid:

$C_{16}H_{25}FN_2O_8$
M = 392.38 g.mol$^{-1}$
IR (NaCl) ν 3406 ($\nu_{NH}$), 1748 ($\nu_{C=Oester}$), 1666 ($\nu_{C=Oamide}$), 1370 ($\delta_{sCH3}$), 1236, 1035 ($\nu_{C-O}$) cm$^{-1}$;
$^1$H NMR (200 MHz, acetone-$d_6$) δ 8.86 (d, 1H, $J$ = 8.8 Hz, NH), 8.27 (br s, 2H, $NH_2$), 5.46-5.29 (m, 2H, H-1, H-3), 4.83 (t, 1H, $J_{3,4}$ = $J_{4,5}$ = 9.6 Hz, H-4), 4.44 (td, 1H, $J_{2,F}$ = 50.2 Hz, $J_{2,3}$ = $J_{1,2}$ = 9.0 Hz, H-2), 4.09 (dd, 1H, $J_{6a,6b}$ = 13.4 Hz, $J_{5,6a}$ = 5.1 Hz, H-6a), 3.92-3.87 (m, 2H, H-6b, H-5), 3.00 (m, 2H, $C\underline{H_2}NH_2$), 2.42-2.39 (m, 2H, $COCH_2$), 2.01-1.83 (m, 11H, $3CH_3$, $CH_2C\underline{H_2}CH_2$);
$^{13}$C NMR (50 MHz, acetone-$d_6$) δ 173.28, 170.81, 170.26, 170.09 (CO), 89.30 (C-2, $J_{2,F}$ = 188.2 Hz), 78.15 (C-1, $J_{1,F}$ = 23.0 Hz), 74.38-73.92 (C-3, C-5), 69.15 (C-4, $J_{4,F}$ = 7.6 Hz), 62.68 (C-6), 46.86 ($CH_2NH_2$), 33.40 ($CO\underline{C}H_2$), 24.70 ($CH_2\underline{C}H_2CH_2$), 20.63 ($CH_3$).

**g) N-(3,4,6-tri-O-acetyl-2-deoxy-2-fluoro-β-D-glucopyranosyl)-4-(4-{4-[bis(2-chloroethyl)amino]phenyl}butanamido)benzamide (20a).**

A solution of chlorambucil (217 mg, 0.72 mmol) in $CH_2Cl_2$ (5 mL) was treated with triethylamine (120 μL, 0.86 mmol). The mixture was cooled to 0°C, treated with ethyl chloroformate (72 μL, 0.75 mmol), and stirred at ambient temperature for 30 min. A solution of amine 19a (277 mg, 0.65 mmol) in $CH_2Cl_2$ (5 mL) was added, and the mixture was stirred one day at ambient temperature. The mixture was washed with saturated aqueous $Na_2CO_3$ and extracted three times with $CH_2Cl_2$. The organic extracts were combined, dried over $MgSO_4$, and concentrated under reduced pressure. The crude product was purified by column chromatography using petroleum benzine:ethyl acetate (5:5 to 3:7) as the eluent, to give compound 20a (370 mg, 80%) as a beige powder:

$C_{33}H_{40}Cl_2FN_3O_9$
M = 712.60 g.mol$^{-1}$
mp 165 °C
Rf= 0.37 (petroleum benzine:ethyl acetate 4:6)
IR (KBr) ν 3325 ($\nu_{NH}$), 1749 ($\nu_{C=Oester}$), 1670 ($\nu_{C=Oamide}$), 1520 ($\delta_{NH}$), 1365 ($\delta_{sCH3}$), 1244, 1066, 1036 ($\nu_{C-O}$) cm$^{-1}$;
$^1$H NMR (200 MHz, $CDCl_3$) δ 7.80 (d, 2H, $J_o$ = 8.6 Hz, $H_{Ar}$), 7.66 (s, 1H, PhN<u>H</u>CO), 7.59 (d, 2H, $J_o$, $H_{Ar}$), 7.39 (d, 1H, $J$ = 9.1 Hz, $C_1$NH), 7.07 (d, 2H, $J_o'$= 8.6 Hz, $H_{Ar}$), 6.63 (d, 2H, $J_o'$, $H_{Ar}$), 5.58 (br t, 1H, $J_{1,2}$ = 9.0 Hz, H-1), 5.40 (td, 1H, $J_{2,3}$ = $J_{3,4}$ = 9.2 Hz, $J_{3,F}$ = 13.8 Hz, H-3), 5.06 (t, 1H, $J_{4,5}$ = 9.8 Hz, H-4), 4.55 (td, 1H, $J_{2,F}$ = 50.3 Hz, H-2), 4.32 (dd, 1H, $J_{5,6a}$ = 4.0 Hz, $J_{6a,6b}$ = 12.4 Hz, H-6a), 4.06 (dd, 1H, $J_{5,6b}$ = 1.5 Hz, H-6b), 3.86 (ddd, 1H, H-5), 3.73-3.55 (m, 8H, $N(CH_2CH_2Cl)_2$), 2.61 (t, 2H, J = 7.3 Hz, $C\underline{H_2}Ph$), 2.38 (t, 2H, $J$ = 7.0 Hz, $COCH_2$), 2.07-1.73 (m, 11H, $CH_2C\underline{H_2}CH_2$, 3OAc);
$^{13}$C NMR (50 MHz, MeOD) δ 174.73, 172.27, 171.47, 171.34, 169.88 (CO), 145.99 ($C_{Ar}N$), 143.91 ($C_{Ar}NH$), 131.60 ($\underline{C}_{Ar}CH_2$), 130.66, 129.66 ($CH_{Ar}$), 129.30 ($\underline{C}_{Ar}CO$), 120.23, 113.54 ($C_{Ar}$), 89.46 (C-2, $J_{2,F}$ = 187.7 Hz), 79.24 (C-1, $J_{1,F}$ = 23.2 Hz), 74.95 (C-3, $J_{3,F}$ = 19.4 Hz), 74.60 (C-5), 69.55 (C-4, $J_{4,F}$ = 7.3 Hz), 63.03 (C-6), 54.53 ($CH_2N$), 41.67 ($CH_2Cl$), 37.41 ($CO\underline{C}H_2$), 35.20 ($\underline{C}H_2Ph$), 28.50 ($CH_2\underline{C}H_2CH_2$), 20.58 ($CH_3$);
MS (ESI) m/z 712.33 [M+1]$^+$.

**Example 10**

**4-(4-{4-[bis(2-chloroethyl)amino]phenyl}butanamido)-N-(2-deoxy-2-fluoro-β-D-glucopyranosyl)benzamide (20b).**

To a solution of acetylated compound 20a (172 mg, 0.241 mmol) in methanol (15 mL) was added sodium methoxide (2 mg) and the mixture was stirred at room temperature for 4 h. After neutralization with IRC 50 Amberlite ion-exchange resin (H+), filtration and evaporation to dryness, the residue was purified on silica gel (5% methanol in ethyl acetate) to yield compound 20b (105 mg, 74%) as a white powder:

$C_{27}H_{34}Cl_2FN_3O_6$
M = 586.49 g.mol$^{-1}$
Decomposition at 230°C
Rf = 0.64 (ethyl acetate:methanol 9:1)
IR (KBr) ν 3301 ($\nu_{NH}$, $\nu_{OH}$), 1658 ($\nu_{C=O}$), 1519 ($\delta_{NH}$), 1089, 1045 ($\nu_{C-O}$) cm$^{-1}$;
$^1$H NMR (200 MHz, DSMO) δ 10.12 (s, 1H, PhN<u>H</u>CO), 9.04 (d, 1H, J = 8.9 Hz, $C_1N\underline{H}$), 7.83 (d, 2H, $J$ = 8.8 Hz, $H_{Ar}$), 7.67 (d, 2H, $J_o$, $H_{Ar}$), 7.02 (d, 2H, $J_o'$ = 8.6 Hz, $H_{Ar}$), 6.64 (d, 2H, $J_o'$, $H_{Ar}$), 5.54 (d, 1H, $J$ = 5.4 Hz, OH), 5.19 (m, 2H, OH, H-1), 4.59 (t, 1H, $J$= 5.6 Hz, OH), 4.29 (td, 1H, $J_{1,2}$ = $J_{2,3}$ = 8.9 Hz, $J_{2,F}$ = 50.6 Hz, H-2), 3.70-3.05 (m, 13H, $N(CH_2CH_2Cl)_2$, H-3, H-4, H-5, 2H-6), 2.48 (m, 2H, $C\underline{H_2}Ph$), 2.31 (t, 2H, $J$ = 7.3 Hz, $COCH_2$), 1.81 (qt, 2H, $CH_2C\underline{H_2}CH_2$);
$^{13}$C NMR (50 MHz, DMSO-$d_6$) δ 171.54, 166.02 (CO), 144.44 ($C_{Ar}N$), 142.47 ($C_{Ar}NH$), 129.72 ($\underline{C}_{Ar}CH_2$), 129.32, 128.40 ($CH_{Ar}$), 127.56 ($\underline{C}_{Ar}CO$), 118.15, 111.91 ($CH_{Ar}$), 91.10 (C-2, $J_{2,F}$ = 182.2 Hz), 78.73 (C-5), 77.42 (C-1, $J_{1,F}$ = 22.9 Hz), 75.18 (C-3, $J_{3,F}$ = 16.2 Hz), 69.85 (C-4, $J_{4,F}$ = 7.6 Hz), 60.55 (C-6), 52.22 ($CH_2N$), 41.14 ($CH_2Cl$), 35.85 ($CO\underline{C}H_2$), 33.54 ($\underline{C}H_2Ph$), 26.85 ($CH_2\underline{C}H_2CH_2$);
MS (ESI) m/z 586.14 [M+1]$^+$.

**Example 11**

**N-[2-(3,4,6-tri-O-acetyl-2-deoxy-2-fluoro-β-D-glucopyranosylamino)-2-oxoethyl]-4-{4-[bis(2-chloroethyl)amino]phenyl}butanamide (21a).**

Amino derivative 19b (1.02 g, 2.80 mmol) was dissolved in anhydrous DMF (70 mL) and chlorambucil (939 mg, 3.09 mmol), DCC (636 mg, 3.09 mmol), and HOBt (417 mg, 3.09 mmol) were added. The solution was stirred 19 h at room temperature, filtered and concentrated in vacuo. The addition of ethyl acetate and a new filtration allowed the elimination of additional DCU. Evaporation of the filtrate followed by purification on silica gel (4:6 to 2:8 petroleum benzine:ethyl acetate gradient) gave compound 21a (945 mg, 64%) as a white solid:

$C_{28}H_{38}Cl_2FN_3O_9$
M = 650.53 g.mol$^{-1}$
mp 106 ˚C
Rf = 0.20 (petroleum benzine:ethyl acetate 4:6)
IR (KBr) ν 3368 ($\nu_{NH}$), 1750 ($\nu_{C=Oester}$), 1653 ($\nu_{C=Oamide}$), 1519 ($\delta_{NH}$), 1230, 1069,1034 ($\nu_{C\text{-}O}$) cm$^{-1}$;
$^1$H NMR (200 MHz, $CDCl_3$) δ 8.08 (d, 1H, *J*= 9.1 Hz, $C_1NH$), 7.06 (d, 2H, $J_o$ = 8.5 Hz, $H_{Ar}$), 6.63 (d, 2H, $H_{Ar}$), 6.46 (br t, 1H, *J* = 4.6 Hz, $CH_2N\underline{H}$), 5.41-5.29 (m, 2H, H-1, H-3), 5.03 (t, 1H, $J_{3,4}$ = $J_{4,5}$ = 9.7 Hz, H-4), 4.40 (td, 1H, $J_{1,2}$ = $J_{2,3}$ = 9.0 Hz, $J_{2,F}$ = 50.3 Hz, H-2), 4.26 (dd, 1H, $J_{5,6a}$ = 4.2 Hz, $J_{6a,6b}$ = 12.7 Hz, H-6a), 4.09-4.01 (m, 3H, H-6b, $COC\underline{H}_2NH$), 3.84 (ddd, 1H, $J_{5,6b}$ = 1.7 Hz, H-5), 3.75-3.57 (m, 8H, $N(CH_2CH_2Cl)_2$), 2.56 (t, 2H, J = 7.3 Hz, $C\underline{H}_2Ph$), 2.26 (t, 2H, *J* = 7.3 Hz, $COCH_2$), 2.08, 2.04, 1.99 (each s, 3×3H, OAc), 1.92 (qt, 2H, $CH_2C\underline{H}_2CH_2$);
$^{13}$C NMR (50 MHz, $CDCl_3$) δ 173.98, 170.71, 170.01, 169.84, 169.74 (CO), 145.58 ($C_{Ar}N$), 130.36 ($\underline{C}_{Ar}CH_2$), 129.78, 112.36 ($CH_{Ar}$), 88.37 (C-2, $J_{2,F}$ = 191.0 Hz), 77.38 (C-1), 73.76-73.29 (C-3, C-5), 68.02 (C-4, $J_{4,F}$ = 6.7 Hz), 61.77 (C-6), 53.70 ($CH_2N$), 43.68 ($CO\underline{C}H_2NH$), 40.67 ($CH_2Cl$), 35.50 ($CO\underline{C}H_2CH_2$), 34.02 ($\underline{C}H_2Ph$), 27.19 ($CH_2\underline{C}H_2CH_2$), 20.79, 20.74 ($CH_3$);
MS (ESI) m/z 650.26 $[M+1]^+$.

**Example 12**

**4-{4-[bis(2-chloroethyl)amino]phenyl}-N-[2-(2-deoxy-2-fluoro-β-D-glucopyranosylamino)-2-oxoethyl]butanamide (21b).**

To a solution of acetylated compound 21a (126 mg, 0.194 mmol) in methanol (5 mL) was added sodium methoxide (2 mg) and the mixture was stirred at room temperature for 3.5 h. After neutralization with IRC 50 Amberlite ion-exchange resin (H+), filtration and evaporation to dryness, the residue was purified on silica gel (10% methanol in ethyl acetate) to yield compound 21b (100 mg, 98%) as a white powder:

$C_{22}H_{32}Cl_2FN_3O_6$
M = 524.42 g.mol$^{-1}$
mp 110˚C
Rf= 0.50 (10% methanol in ethyl acetate)
IR (KBr) ν 3315 ($\nu_{OH}$, $\nu_{NH}$), 1647 ($\nu_{C=O}$), 1519 ($\delta_{NH}$), 1249, 1081, 1039 ($\nu_{C\text{-}O}$) cm$^{-1}$;
$^1$H NMR (200 MHz, acetone-$d_6$) δ 8.19 (d, 1H, *J* = 9.3 Hz, $C_1NH$), 7.51 (t, 1H, *J* = 5.5 Hz, $CH_2N\underline{H}$), 7.08 (d, 2H, $J_o$ = 8.6 Hz, $H_{Ar}$), 6.70 (d, 2H, $H_{Ar}$), 5.21 (td, 1H, $J_{1,2}$ = 9.1 Hz, $J_{1,F}$ = 1.8 Hz, H-1), 4.99 (br d, 1H, *J* = 3.4 Hz, OH), 4.62 (br s, 1H, OH), 4.15 (td, 1H, $J_{2,3}$ = 8.8 Hz, $J_{2,F}$ = 50.8 Hz, H-2), 4.09 (br s, 1H, OH), 3.95 (d, 2H, $COC\underline{H}_2NH$), 3.79-3.66 (m, 11H, $N(CH_2CH_2Cl)_2$, H-3, 2H-6), 3.42 (m, 2H, H-4, H-5), 2.53 (t, 2H, *J* = 7.5 Hz, $C\underline{H}_2Ph$), 2.27 (t, 2H, *J* = 7.3 Hz, $COC\underline{H}_2CH_2$), 1.86 (qt, 2H, $CH_2C\underline{H}_2CH_2$);
$^{13}$C NMR (50 MHz, acetone-$d_6$) δ 174.39, 171.08 (CO), 145.45 ($C_{Ar}N$), 131.49 ($\underline{C}_{Ar}CH_2$), 130.36, 113.04 ($CH_{Ar}$), 92.13 (C-2, $J_{2,F}$ = 183.9 Hz), 79.08 (C-5), 78.13 (C-1, $J_{1,F}$ = 23.3 Hz), 76.55 (C-3, $J_{3,F}$ = 16.7 Hz), 70.94 (C-4, $J_{4,F}$ = 4.0 Hz), 62.02 (C-6), 53.89 ($CH_2N$), 43.31 ($CO\underline{C}H_2NH$), 41.65 ($CH_2Cl$), 35.87 ($CO\underline{C}H_2CH_2$), 34.76 ($\underline{C}H_2Ph$), 28.27 ($CH_2\underline{C}H_2CH_2$);
MS (ESI) m/z 524.12 $[M+1]^+$.

**Example 13**

**N-[4-(3,4,6-tri-O-acetyl-2-deoxy-2-fluoro-β-D-glucopyranosylamino)-4-oxobutyl]-4-{4-[bis(2-chloroethyl)amino]phenyl}butanamide (22a).**

A solution of chlorambucil (130 mg, 0.43 mmol) in $CH_2Cl_2$ (4 mL) was treated with triethylamine (72 μL, 0.52 mmol). The mixture was cooled to 0°C, treated with ethyl chloroformate (43 μL, 0.45 mmol), and stirred at ambient temperature for 30 min. Amine 19c (168 mg, 0.43 mmol) was then added, and the mixture was stirred 2 days at ambient temperature. The mixture was washed with saturated aqueous $Na_2CO_3$ and extracted three times with $CH_2Cl_2$. The organic extracts were combined, dried over $MgSO_4$, and concentrated under reduced pressure. The crude product was purified by column chromatography using ethyl acetate as developing solvent, to give compound 22a (70 mg, 24%) as a beige powder:

$C_{30}H_{42}Cl_2FN_3O_9$
M = 678.58 g.mol$^{-1}$
mp 95 °C
Rf = 0.26 (ethyl acetate)
IR (NaCl) ν 3295 ($\nu_{NH}$), 1749 ($\nu_{C=Oester}$), 1647 ($\nu_{C=Oamide}$), 1542 ($\delta_{NH}$), 1366 ($\delta_{sCH3}$), 1230, 1069, 1034 ($\nu_{C-O}$) cm$^{-1}$;
$^1$H NMR (200 MHz, $CDCl_3$) δ 8.26 (d, 1H, *J*= 9.0 Hz, $C_1N\underline{H}$), 7.07 (d, 2H, $J_o$ = 8.6 Hz, $H_{Ar}$), 6.63 (d, 2H, $H_{Ar}$), 5.86 (br t, 1H, *J* = 6.0 Hz, $CH_2N\underline{H}CO$), 5.45-5.34 (m, 2H, H-1, H-3), 5.06 (t, 1H, $J_{3,4}$ = $J_{4,5}$ = 9.8 Hz, H-4), 4.47 (td, 1H, $J_{1,2}$ = $J_{2,3}$ = 9.1 Hz, $J_{2,F}$ = 50.3 Hz, H-2), 4.29 (dd, 1H, $J_{6a,6b}$ = 12.5 Hz, $J_{5,6a}$ = 4.3 Hz, H-6a), 4.08 (dd, 1H, $J_{5,6b}$ = 2.1 Hz, H-6b), 3.83 (ddd, 1H, H-5), 3.76-3.58 (m, 8H, $N(CH_2CH_2Cl)_2$), 3.43 (m, 1H, $C\underline{H}NH$), 3.17 (m, 1H, $C\underline{H}'NH$), 2.56 (t, 2H, *J* = 7.3 Hz, $C\underline{H_2}Ph$), 2.30-2.17 (m, 4H, $2CH_2CO$), 2.07, 2.05, 2.04 (each s, 3×3H, OAc), 2.00-1.76 (m, 4H, $2CH_2C\underline{H_2}CH_2$);
$^{13}$C NMR (50 MHz, $CDCl_3$) δ 174.45, 173.62 (NHCO), 170.76, 170.04, 169.75 ($\underline{C}OCH_3$), 144.52 ($C_{Ar}N$), 130.48 ($\underline{C}_{Ar}CH_2$), 129.78, 112.32 ($CH_{Ar}$), 88.42 (C-2, $J_{2,F}$ = 189.8 Hz), 77.63 (C-1, $J_{1,F}$ = 23.2 Hz), 73.65 (C-5), 73.64 (C-3, $J_{3,F}$ = 22.6 Hz), 68.06 (C-4, $J_{4,F}$ = 6.7 Hz), 61.82 (C-6), 53.69 ($CH_2N$), 40.65 ($CH_2Cl$), 38.42 ($CH_2CH_2\underline{C}H_2NH$), 36.10 ($\underline{C}H_2CH_2CH_2Ph$), 34.19 ($CH_2CH_2\underline{C}H_2Ph$), 33.59 ($\underline{C}H_2CH_2CH_2NH$), 27.56 ($CH_2\underline{C}H_2CH_2Ph$), 26.42 ($CH_2\underline{C}H_2CH_2NH$), 20.86, 20.79, 20,71 ($CH_3$);
MS (ESI) m/z 678.29 $[M+1]^+$.

**Example 14**

**4-{4-[bis(2-chloroethyl)amino]phenyl}-N-[4-(2-deoxy-2-fluoro-β-D-glucopyranosylamino)-4-oxobutyl]butanamide (22b).**

To a solution of acetylated compound 22a (100 mg, 0.18 mmol) in methanol (5 mL) was added sodium methoxide (2 mg) and the mixture was stirred at room temperature for 6.5 h. After neutralization with IRC 50 Amberlite ion-exchange resin (H+), filtration and evaporation to dryness, the residue was purified on silica gel (10% methanol in ethyl acetate) to yield compound 22b (69 mg, 72%) as a solid:

$C_{24}H_{36}Cl_2FN_3O_6$
M = 552.47 g.mol$^{-1}$
mp 161 °C
Rf = 0.24 (10% methanol in ethyl acetate)
IR (KBr) ν 3525, 3309 ($\nu_{OH}$, $\nu_{NH}$), 1641 ($\nu_{C=O}$), 1550, 1519 ($\delta_{NH}$), 1355 ($\delta_{sCH3}$), 1093, 1045 ($\nu_{C-O}$) cm$^{-1}$;
$^1$H NMR (200 MHz, acetone-$d_6$) δ 8.13 (d, 1H, *J* = 9.2 Hz, $C_1\underline{NH}$), 7.18-7.05 (m, 3H, $H_{Ar}$, $CH_2N\underline{H}$), 6.72 (d, *2H*, $J_o$ = 8.7 Hz, $H_{Ar}$), 5.19 (td, 1H, $J_{1,2}$ = 9.0 Hz, $J_{1,F}$ = 2.2 Hz, H-1), 4.78 (br s, 1H, OH), 4.45 (br s, 1H, OH), 4.12 (td, 1H, $J_{2,3}$ = 9.0 Hz, $J_{2,F}$ = 50.9 Hz, H-2), 3.90-3.51 (m, 12H, $N(CH_2CH_2Cl)_2$, H-3, H-4, 2H-6), 3.40 (m, 2H, OH, H-5), 3.23 (q, 2H, *J* = 6.4 Hz, $C\underline{H_2}NH$), 2.52 (t, 2H, *J* = 7.4 Hz, $C\underline{H_2}Ph$), 2.28-2.13 (m, 4H, $2CH_2CO$), 1.96-1.73 (m, 4H, $2CH_2C\underline{H_2}CH_2$);
$^{13}$C NMR (50 MHz, MeOD) δ 176.30, 176.18 (NHCO), 145.98 ($C_{Ar}N$), 131.73 ($\underline{C}_{Ar}CH_2$), 130.60, 113.54 ($CH_{Ar}$), 92.27 (C-2, $J_{2,F}$ = 184.4 Hz), 79.75 (C-5), 78.59 (C-1, $J_{1,F}$ = 23.3 Hz), 76.93 (C-3, $J_{3,F}$ = 16.9 Hz), 71.11 (C-4, $J_{4,F}$ = 7.6 Hz), 62.35 (C-6), 54.55 ($CH_2N$), 41.71 ($CH_2Cl$), 39.75 ($CH_2CH_2\underline{C}H_2NH$), 36.55 ($\underline{C}H_2CH_2CH_2Ph$), 35.23 ($CH_2CH_2\underline{C}H_2Ph$), 34.34 ($\underline{C}H_2CH_2CH_2NH$), 28.94 ($CH_2\underline{C}H_2CH_2Ph$), 26.34 ($CH_2\underline{C}H_2CH_2NH$);
MS (ESI) m/z 552.14 $[M+1]^+$.

Following compounds 31 have been obtained by the reaction pathway depicted on Scheme 2c, proceeding by

the preliminary coupling of chlorambucil with the spacer before the N-glycosylation, contrary to the formers 20, 21 and 22.

**Example 15**

**N-[3-(3,4,6-tri-O-acetyl-2-deoxy-2-fluoro-β-D-glucopyranosylamino)-3-oxopropyl]-4-{4-[bis(2-chloroethyl)amino]phenyl}butanamide (31a) (see scheme 2c).**

**a) 4-azidobutyric acid ethyl ester (24).**

A stirred solution of 4-bromobutyric acid ethyl ester 23 (2.21 mL, 15.4 mmol) and sodium azide (2 g, 30.8 mmol) in acetone/water (22.5/7.5 mL) was heated under reflux for 7 h. The MeOH was removed in vacuo and residue was partitioned between $CH_2Cl_2$ and water. The product was extracted three times with $CH_2Cl_2$, the combined organic extracts were dried over $MgSO_4$ and evaporated to dryness, to give the azido ester 24 (2.19 g, 90%) as a colorless oil:

$C_6H_{11}N_3O_2$
M = 157.17 g.mol$^{-1}$
IR (NaCl) ν 2100 ($\nu_{asN3}$) 1735 ($\nu_{C=O}$), 1256, 1175 ($\nu_{C-O}$) cm$^{-1}$
$^1$H NMR (200 MHz, $CDCl_3$) δ 4.15 (q, 2H, *J* = 7.1 Hz, $CH_3C\underline{H}_2O$), 3.36 (t, 2H, *J* = 6.7 Hz, $CH_2N_3$), 2.41 (t, 2H, J = 7.3 Hz, $COCH_2$), 1.91 (qt, 2H, J = 7.1 Hz, $CH_2C\underline{H}_2CH_2$), 1.27 (t, 3H, $CH_3$);
$^{13}$C NMR (50 MHz, $CDCl_3$) δ 172.54 (CO), 60.53 ($\underline{C}H_2CH_3$), 50.66 ($CH_2N_3$), 31.38 ($CO\underline{C}H_2$), 24.28 ($CH_2\underline{C}H_2CH_2$), 14.18 ($CH_3$).

**4-azidobutyric acid (25).**

Potassium hydroxide (3.9 g, 69.6 mmol) was added to a solution at 0°C of 4-azidobutyric acid ethyl ester 24 (2.19 g, 13.9 mmol) in a mixture of 32 mL MeOH and 26 mL water. The solution was stirred at room temperature for 6 h and then evaporated. The residue was partitioned between $CH_2Cl_2$ and water. The aqueous layer was extracted twice with $CH_2Cl_2$, acidified to pH 1 with 1N aqueous HCl and extracted five times with $Et_2O$. The organic extracts were combined, dried over $MgSO_4$ and evaporated to dryness, to afford the azido acid 25 (1.77 g, 99%) as a colorless oil. The $^1$H NMR analysis confirmed the product to be pure and it was used directly in the next step:

$C_4H_7N_3O_2$
M = 129.12 g.mol$^{-1}$
IR (NaCl) ν 3650-2500 ($\nu_{OH}$), 2103 ($\nu_{asN3}$), 1712 ($\nu_{C=O}$), 1259, 1169 ($\nu_{C-O}$) cm$^{-1}$;
$^1$H NMR (200 MHz, $CDCl_3$) δ 11.07 (br s, 1H, COOH), 3.38 (t, 2H, *J* = 6.7 Hz, $CH_2N_3$), 2.48 (t, 2H, *J*= 7.2 Hz, $COCH_2$), 1.92 (qt, 2H, *J*= 7.0 Hz, $CH_2C\underline{H}_2CH_2$);
$^{13}$C NMR (50 MHz, $CDCl_3$) δ 179.33 (CO), 50.49 ($CH_2N_3$), 30.99 ($CO\underline{C}H_2$), 23.96 ($CH_2\underline{C}H_2CH_2$).

**3-azidopropionic acid ethyl ester (27).**

A stirred solution of 3-bromopropionic acid ethyl ester 26 (2.11 mL, 16.6 mmol) and sodium azide (2.15 g, 33.2 mmol) in MeOH/water (22.5/7.5 mL) was heated under reflux for 6.5 h. The MeOH was removed in vacuo and residue was partitioned between $CH_2Cl_2$ and water. The product was extracted four times with $CH_2Cl_2$ and the combined organic extracts were dried over $MgSO_4$. After removal of the solvent followed by flash chromatography, eluting with petroleum benzine:ethyl acetate (9:1), provided the azido ester 27 (1.01 g, 43%) as yellowish liquid:

$C_5H_9N_3O_2$
M = 143.15 g.mol$^{-1}$
Rf = 0.53 (petroleum benzine:ethyl acetate 9:1)
IR (NaCl) ν 2103 ($\nu_{asN3}$), 1732 ($\nu_{C=O}$), 1190, 1026 ($\nu_{C-O}$) cm$^{-1}$;
$^1$H NMR (200 MHz, $1CDCl_3$) δ 4.18 (q, 2H, *J*= 7.1 Hz, $CH_3C\underline{H}_2O$), 3.58 (t, 2H, *J*= 6.5 Hz, $CH_2N_3$), 2.58 (t, 2H, $COCH_2$), 1.28 (t, 3H, $CH_3$);
$^{13}$C NMR (50 MHz, $CDCl_3$) δ 170.82 (CO), 60.91 ($\underline{C}H_2CH_3$), 46.78 ($CH_2N_3$), 33.98 ($CO\underline{C}H_2$), 14.11 ($CH_3$).

**3-aminopropionic acid ethyl ester (28).**

10% palladium on charcoal (92 mg) was added to a solution of the azido derivative 27 (460 mg, 3.21 mmol) in THF/MeOH (7.5/7.5 mL). The reaction mixture was then submitted to a hydrogen atmosphere at room temperature

for 6 h. After filtration on celite and evaporation of solvent, compound 28 (306 mg, 81%) was obtained as a colourless oil:

$C_5H_{11}NO_2$
M = 117.15 g.mol$^{-1}$
$^1$H NMR (200 MHz, $CDCl_3$) δ 4.16 (q, 2H, J = 7.1 Hz, $CH_3CH_2O$), 2.99 (t, 2H, J = 6.3 Hz, $CH_2NH_2$), 2.46 (t, 2H, $COCH_2$), 1.54 (s, 2H, $NH_2$), 1.27 (t, 3H, $CH_3$).

**b) 3-(4-{4-[bis(2-chloroethyl)amino]phenyl}butyramido)propionic acid ethyl ester (29).**

A solution of chlorambucil (874 mg, 2.79 mmol) in $CH_2Cl_2$ (16 mL) was treated with triethylamine (480 μL, 3.45 mmol). The mixture was cooled to 0°C, treated with ethyl chloroformate (290 μL, 3.02 mmol), and stirred at ambient temperature for 30 min. A solution of the amino ester 28 (306 mg, 2.61 mmol) in $CH_2Cl_2$ (3 mL) was added, and the mixture was stirred one day at ambient temperature. The mixture was washed with saturated aqueous $Na_2CO_3$ and extracted three times with $CH_2Cl_2$. The organic extracts were combined, dried over $MgSO_4$, and concentrated under reduced pressure. The crude product was purified by silica gel chromatography (8:2 to 3:7 petroleum benzine:ethyl acetate gradient), to give compound 29 (491 mg, 47%) as a brown oil:

$C_{19}H_{28}Cl_2N_2O_3$
M = 403.35 g.mol$^{-1}$
Rf = 0.20 (petroleum benzine:ethyl acetate 5:5)
IR (NaCl) ν 3410 ($\nu_{NH}$), 1727 ($\nu_{C=Oester}$), 1653 ($\nu_{C=Oamide}$), 1518 ($\delta_{NH}$), 1265 ($\nu_{C-O}$) cm$^{-1}$;
$^1$H NMR (200 MHz, $CDCl_3$) δ 7.06 (d, 2H, $J_o$ = 8.5 Hz, $H_{Ar}$), 6.61 (d, 2H, $H_{Ar}$), 6.10 (br s, 1H, NH), 4.15 (q, 2H, *J* = 7.1 Hz, $CH_3C\underline{H_2}O$), 3.75-3.57 (m, 8H, $N(CH_2CH_2Cl)_2$), 3.50 (q, 2H, *J*= 5.9 Hz, $C\underline{H_2}NH$), 2.52 (m, 4H, $C\underline{H_2}Ph$, $C\underline{H_2}COOEt$), 2.16 (t, 2H, *J*= 6.8 Hz, $C\underline{H_2}CONH$), 1.89 (qt, 2H, $CH_2C\underline{H_2}CH_2$), 1.26 (t, 3H, $CH_3$);
$^{13}$C NMR (50 MHz, $CDCl_3$) δ 172.86 (CO), 144.38 ($C_{Ar}N$), 130.74 ($\underline{C}_{Ar}CH_2$), 129.76, 112.26 ($CH_Ar$), 60.81 ($CH_3\underline{C}H_2O$), 53.68 ($CH_2N$), 40.60 ($CH_2Cl$), 35.98, 34.85, 34.14, 34.10 ($C\underline{H_2}COOEt$, $CH_2NH$, $\underline{C}H_2Ph$, $\underline{C}H_2CONH$), 27.40 ($CH_2C\underline{H_2}CH_2$), 14.26 ($CH_3$).

**c) 3-(4-{4-[bis(2-chloroethyl)amino]phenyl}butyramido)propionic acid (30).**

Lithium hydroxide (65 mg, 2.7 mmol) was added to a solution of 29 (435 mg, 1.08 mmol) in a mixture of 15 mL EtOH and 7.5 mL water. The solution was stirred at room temperature for 4.5 h and then evaporated. The residue was partitioned between $CH_2Cl_2$ and water. The aqueous layer was acidified to pH 1 with 1N aqueous HCl (2.7 mL) and extracted twice with $CH_2Cl_2$. The organic extracts were combined, dried over $MgSO_4$ and evaporated to dryness, to afford acid 30 (400 mg, 98%) as a solid:

$C_{17}H_{24}Cl_2N_2O_3$
M = 375.30 g.mol$^{-1}$
mp 102 °C
Rf = 0.63 (ethyl acetate:methanol 9:1)
IR (NaCl) ν 3326 ($\nu_{NH}$), 3200-2300 ($\nu_{OH}$), 1720 ($\nu_{C=Oacide}$), 1615 ($\nu_{C=Oamide}$), 1518 ($\delta_{NH}$) cm$^{-1}$;
$^1$H NMR (200 MHz, $CDCl_3$) δ 9.53 (br s, 1H, OH), 7.05 (d, 2H, $J_o$ = 8.6 Hz, $H_{Ar}$), 6.61 (d, 2H, $H_{Ar}$), 6.25 (br t, 1H, *J*= 5.8 Hz, NH), 3.74-3.56 (m, 8H, $N(CH_2CH_2Cl)_2$), 3.50 (q, 2H, J = 5.8 Hz, $C\underline{H_2}NH$), 2.55 (m, 4H, $C\underline{H_2}Ph$, $C\underline{H_2}COOH$), 2.18 (t, 2H, *J* = 7.2 Hz, $C\underline{H_2}CONH$), 1.88 (qt, 2H, $CH_2C\underline{H_2}CH_2$);
$^{13}$C NMR (50 MHz, $CDCl_3$) δ 176.63, 173.79 (CO), 144.49 ($C_{Ar}N$), 130.56 ($C_{Ar}CH_2$), 129.79, 112.30 ($CH_{Ar}$), 53.69 ($CH_2N$), 40.67 ($CH_2Cl$), 35.96, 34.99, 34.10 ($\underline{C}H_2COOH$, $CH_2NH$, $\underline{C}H_2Ph$, $NHCO\underline{C}H_2$), 27.39 ($CH_2\underline{C}H_2CH_2$).

**d) N-[3-(3,4,6-tri-O-acetyl-2-deoxy-2-fluoro-β-D-glucopyranosylamino)-3-oxopropyl]-4-{4-[bis(2-chloroethyl) amino]phenyl}butanamide (31a).**

A solution of acid 30 (33 mg, 0.09 mmol) in $CH_2Cl_2$ (2 mL) was treated with triethylamine (26 μL, 0.18 mmol). The mixture was cooled to 0°C, treated with ethyl chloroformate (9 μL, 0.09 mmol), and stirred at ambient temperature for 30 min. Then, amino-sugar 8 (24.5 mg, 0.08 mmol) was added, and the mixture was stirred 4 days at ambient temperature. The mixture was washed with saturated aqueous $Na_2CO_3$ and extracted three times with $CH_2Cl_2$. The organic extracts were combined, dried over $MgSO_4$, and concentrated under reduced pressure. The crude product was purified by silica gel chromatography (2:8 to 1:9 petroleum benzine:ethyl acetate gradient), to give expected compound 31a (47 mg, 88%) as a beige solid:

$C_{29}H_{40}Cl_2FN_3O_9$
M = 664.56 g.mol$^{-1}$
mp 103 °C
Rf = 0.19 (petroleum benzine: ethyl acetate 2:8)
IR (KBr) ν 3309 ($\nu_{NH}$), 1751 ($\nu_{C=Oester}$), 1676, 1647 ($\nu_{C=Oamide}$), 1519 ($\delta_{NH}$), 1367 ($\delta_{sCH3}$), 1228, 1066, 1034 ($\nu_{C-O}$) cm$^{-1}$;
$^1$H NMR (200 MHz, $CDCl_3$) δ 7.90 (d, 1H, $J$= 8.9 Hz, $C_1N\underline{H}$), 7.04 (d, 2H, $J_o$ = 8.5 Hz, $H_{Ar}$), 6.61 (d, 2H, $H_{Ar}$), 6.37 (br t, 1H, $J$ = 5.5 Hz, $CH_2N\underline{H}CO$), 5.46-5.30 (m, 2H, H-1, H-3), 5.03 (t, 1H, $J_{3,4}$ = $J_{4,5}$ = 9.8 Hz, H-4), 4.40 (td, 1H, $J_{1,2}$ = $J_{2,3}$ = 9.1 Hz, $J_{2,F}$ = 50.4 Hz, H-2), 4.28 (dd, 1H, $J_{6a,6b}$ = 12.5 Hz, $J_{5,6a}$ = 4.3 Hz, H-6a), 4.04 (dd, 1H, $J_{5,6b}$ = 1.4 Hz, H-6b), 3.84 (m, 1H, H-5), 3.72-3.47 (m, 10H, $N(CH_2CH_2Cl)_2$, $C\underline{H}_2NH$), 2.52 (m, 4H, $C\underline{H}_2Ph$, $COC\underline{H}_2CH_2NH$), 2.19-2.04 (m, 11H, $COC\underline{H}_2CH_2CH_2$, 3OAc), 1.87 (m, 2H, $CH_2C\underline{H}_2CH_2$);
$^{13}$C NMR (50 MHz, MeOD) δ 176.25, 174.45 (CONH), 172.19, 171.43, 171.27 ($\underline{C}OCH_3$), 145.99 ($C_{Ar}N$), 131.81 ($\underline{C}_{Ar}CH_2$), 130.63, 113.57 ($CH_{Ar}$), 89.57 (C-2, $J_{2,F}$ = 188.6 Hz), 78.51 (C-1, $J_{1,F}$ = 22.9 Hz), 74.81 (C-3, $J_{3,F}$ = 19.5 Hz), 74.53 (C-5), 69.50 (C-4, $J_{4,F}$ = 7.3 Hz), 62.98 (C-6), 54.58 ($CH_2N$), 41.72 ($CH_2Cl$), 36.44, 35.15 ($CO\underline{C}H_2\underline{C}H_2NH$, $CO\underline{C}H_2CH_2\underline{C}H_2Ph$), 28.89 ($CH_2\underline{C}H_2CH_2$), 20.58 ($CH_3$);
MS (ESI) m/z 664.33 [M+1]$^+$.

**Example 16**

**4-{4-[bis(2-chloroethyl)amino]phenyl}-N-[3-(2-deoxy-2-fluoro-β-D-glucopyranosylamino)-3-oxopropyl]butanamide (31b).**

To a stirred solution of acetylated compound 31a (120 mg, 0.18 mmol) in methanol (10 mL) was added sodium methoxide (2 mg) and the mixture was stirred at room temperature for 6.5 h. After neutralization with IRC 50 Amberlite ion-exchange resin (H$^+$), filtration and evaporation to dryness, the residue was purified on silica gel (10% methanol in ethyl acetate) to yield compound 31b (80 mg, 83%) as a white solid:

$C_{23}H_{34}Cl_2FN_3O_6$
M = 538.44 g.mol$^{-1}$
mp 179 °C
Rf = 0.30 (10% methanol in ethyl acetate)
IR (KBr) ν 3525, 3305 ($\nu_{NH}$, $\nu_{OH}$), 1647 ($\nu_{C=O}$), 1544 ($\delta_{NH}$), 1091, 1043 ($\nu_{C-O}$) cm$^{-1}$;
$^1$H NMR (200 MHz, acetone-$d_6$) δ 8.05 (d, 1H, $J$ = 9.1 Hz, $C_1N\underline{H}$), 7.06 (m, 3H, $H_{Ar}$, $CH_2N\underline{H}CO$), 6.72 (d, 2H, $J_o$ = 8.8 Hz, $H_{Ar}$), 5.19 (td, 1H, $J_{1,2}$ = 9.1 Hz, $J_{1,F}$ = 2.3 Hz, H-1), 4.84 (d, 1H, $J$ = 4.5 Hz, OH), 4.48 (d, 1H, $J$ = 4.5 Hz, OH), 4.09 (td, 1H, $J_{2,3}$ = 9.0 Hz, $J_{2,F}$ = 50.9 Hz, H-2), 3.80-3.68 (m, 11H, $N(CH_2CH_2Cl)_2$, H-3, 2H-6), 3.45-3.38 (m, 5H, H-4, H-5, $C\underline{H}_2NH$, OH), 2.47 (m, 4H, $C\underline{H}_2Ph$, $COC\underline{H}_2CH_2NH$), 2.15 (m, 2H, $COC\underline{H}_2CH_2CH_2$), 1.83 (m, 2H, $CH_2C\underline{H}_2CH_2$);
$^{13}$C NMR (50 MHz, MeOD) δ 176.19, 174.58 (NHCO), 145.92 ($C_{Ar}N$), 131.73 ($\underline{C}_{Ar}CH_2$), 130.60, 113.49 ($CH_{Ar}$), 92.20 (C-2, $J_{2,F}$ = 184.8 Hz), 79.71 (C-5), 78.50 (C-1, $J_{1,F}$ = 23.1 Hz), 76.90 (C-3, $J_{3,F}$ = 16.8 Hz), 71.13 (C-4, $J_{4,F}$ = 7.5 Hz), 62.38 (C-6), 54.53 ($CH_2N$), 41.69 ($CH_2Cl$), 36.52, 35.16 ($CO\underline{C}H_2\underline{C}H_2NH$, $CO\underline{C}H_2CH_2\underline{C}H_2Ph$), 28.87 ($CH_2CH_2CH_2$);
MS (ESI) m/z 538.06 [M+1]$^+$.

**Example 17**

**N-[4-(4-{4-[bis(2-chloroethyl)amino]phenyl}butanoyloxy)phenyl]-3,4,6-triO-benzyl-2-deoxy-2-fluoro-β-D-glucopyranosylamine (34a) (Scheme 2d)**

**a) 4-{4-[bis(2-chloroethyl)amino]phenyl}butyric acid 4-nitrophenyl ester (32).**

To a solution of 4-nitrophenol (0.152 mg, 1.09 mmol) in $CH_2Cl_2$ (20 mL), chlorambucil (500 mg, 1.64 mmol), DCC (375 mg, 1.82 mmol) and DMAP (9 mg, 0.073 mmol) were added and stirred one day at room temperature. The solid was removed by filtration, and the filtrate was diluted with $CH_2Cl_2$ (25 mL). The mixture was washed consecutively with a 1N aqueous solution of acetic acid (35 mL) and water (35 mL), dried over $MgSO_4$, and concentrated under vacuum. The resulting residue was purified by silica gel chromatography, using petroleum benzine:ethyl acetate (8:2) as mobile phase, to yield 32 (432 mg, 93%) as a yellow syrup:

$C_{20}H_{22}Cl_2N_2O_4$

M = 425.31 $g.mol^{-1}$
Rf = 0.49 (petroleum benzine:ethyl acetate 6 : 4)
IR (NaCl) ν 1762 ($\nu_{C=O}$), 1519 ($\nu_{asNO2}$), 1347 ($\nu_{sNO2}$), 1206, 1112 ($\nu_{C-O}$) $cm^{-1}$;
$^1H$ NMR (200 MHz, $CDCl_3$) δ 8.28 (d, 2H, $J_o$ = 9.2 Hz, $H_{Ar}$), 7.26 (d, 2H, $J_o$, $H_{Ar}$), 7.19 (d, 2H, $J_o'$ = 8.7 Hz, $H_{Ar}$), 6.67 (d, 2H, $J_o'$, $H_{Ar}$), 3.65 (m, 8H, $N(CH_2CH_2Cl)_2$), 2.67 (t, 2H, $J$ = 7.5 Hz, $C\underline{H_2}Ph$); 2.63 (t, 2H, $J$ = 7.6 Hz, $COCH_2$), 2.06 (qt, 2H, $CH_2\underline{CH_2}CH_2$);
$^{13}C$ NMR (50 MHz, $CDCl_3$) δ 169.13 (CO), 153.51 ($C_{Ar}O$), 143.33, 142.58 ($C_{Ar}NO_2$, $C_{Ar}N$), 128.05 ($C_{Ar}CH_2$), 127.82, 123.25, 120.47, 110.31 ($CH_{Ar}$), 51.65 ($CH_2N$), 38.54 ($CH_2Cl$), 31.92, 31.64 ($\underline{C}H_2Ph$, $COCH_2$), 24.52 ($CH_2\underline{C}H_2CH_2$).

**b) 4-{4-[bis(2-chloroethyl)amino]phenyl}butyric acid 4-aminophenyl ester (33).**

To a suspension of 10% palladium on charcoal (1.5 g) in THF/EtOH (40/50 mL) was added the nitro compound 32 (9 g, 21 mmol). The mixture was hydrogenated at atmospheric pressure for one day, filtered on celite, and concentrated to yield the compound 33 (6.6 g, 79%) as an orange oil:

$C_{20}H_{24}Cl_2N_2O_2$
M = 395.33 $g.mol^{-1}$
Rf= 0.37 (cyclohexane :ethyl acetate 6:4)
IR (NaCl) ν 3453, 3373 ($\nu_{NH2}$), 1747 ($\nu_{C=O}$), 1616 ($\delta_{NH2}$), 1194, 1135 ($\nu_{C-O}$) $cm^{-1}$;
$^1H$ NMR (200 MHz, $CDCl_3$) δ 7.09 (d, 2H, $J_o$ = 8.5 Hz, $H_{Ar}$), 6.83 (d, 2H, $J_o'$= 8.7 Hz, $H_{Ar}$), 6.65 (d, 4H, $H_A$r), 3.73-3.56 (m, 10H, $N(CH_2CH_2Cl)_2$, $NH_2$), 2.63 (t, 2H, $J$= 7.5 Hz, $C\underline{H_2}Ph$), 2.52 (t, 2H, $J$ = 7.4 Hz, $COCH_2$), 2.00 (qt, 2H, $CH_2\underline{CH_2}CH_2$);
$^{13}C$ NMR (50 MHz, $CDCl_3$) δ 172.73 (CO), 144.47, 144.22 ($2C_{Ar}N$), 142.93 ($C_{Ar}O$), 130.52 ($C_{Ar}CH_2$), 129.85, 122.21, 115.69, 112.26 ($CH_A$r), 53.68 ($CH_2N$), 40.63 ($CH_2Cl$), 34.05, 33.72 ($\underline{C}H_2Ph$, $\underline{C}OCH_2$), 26.89 ($CH_2\underline{C}H_2CH_2$).

**c) N-[4-(4-{4-[bis(2-chloroethyl)amino]phenyl}butanoyloxy)phenyl]-3,4,6-tri-O-benzyl-2-deoxy-2-fluoro-β-D-glucopyranosylamine (34a).**

A solution of amino compound **33** (307 mg, 0.78 mmol) in $CH_3CN$ (5 ml) was added to a solution of bromide **12** (400 mg, 0.78 mmol) in $CH_3CN$ (10 ml) containing $Ag_2O$ (770 mg, 3.32 mmol). After stirring at room temperature for 20h, the mixture was filtered on celite to remove Ag salts, and the solvent was evaporated. The crude product was purified by column chromatography on silica gel using petroleum benzine:ethyl acetate (8:2) as eluent to give the β-anomer 34a as an oil (222 mg, 35%):

$C_{47}H_{51}Cl_2FN_2O_6$
M = 829.84 $g.mol^{-1}$
Rf = 0.54 (cyclohexane:ethyl acetate 7:3)
IR (NaCl) ν 3393 ($\nu_{NH}$), 1750 ($\nu_{C=O}$), 1518 ($\delta_{NH}$), 1200, 1180, 1133, 1027 ($\nu_{C-O}$) $cm^{-1}$;
$^1H$ NMR (500 MHz, $CDCl_3$) δ 7.39-7.19 (m, 16H, $H_{Ar}$, NH), 7.12 (d, 2H, $J_o$ = 8.6 Hz, $H_{Ar}$), 6.91 (d, 2H, $J_o'$ = 8.8 Hz, $H_{Ar}$), 6.76 (d, 2H, $J_o'$, $H_{Ar}$), 6.66 (d, 2H, $J_o$, $H_{Ar}$), 4.94 (d, 1H, $J$ = 11.1 Hz, $OC\underline{H_2}Ph$), 4.87 (d, 1H, $J$ = 10.8 Hz, $OC\underline{H_2}Ph$), 4.80 (d, 1H, $J$ = 11.1 Hz, $OC\underline{H_2}Ph$), 4.69 (br t, 1H, $J_{1,2}$ = $J_{1,NH}$ = 7.5 Hz, H-1), 4.60 (d, 1H, $J$ = 12.1 Hz, $OC\underline{H_2}Ph$), 4.56 (d, 1H, $J$ = 10.9 Hz, $OC\underline{H_2}Ph$), 4.52 (m, 1H, H-4), 4.50 (d, 1H, $J$ = 12.1 Hz, $OC\underline{H_2}Ph$), 4.41 (td, 1H, $J_{2,3}$ = 8.5 Hz, $J_{2,F}$ = 51.0 Hz, H-2), 3.88 (td, 1H, $J_{3,4}$ = 8.5 Hz, $J_{3,F}$ = 15.0 Hz, H-3), 3.77 (dd, 1H, $J_{6a,6b}$ = 11.0 Hz, $J_{6a,5}$ = 2.0 Hz, H-6a), 3.72 (t, 5H, $J$ = 6.9 Hz, $N(CH_2C\underline{H_2}Cl)_2$, H-6b), 3.64 (t, 4H, $J$ = 6.9 Hz, $N(C\underline{H_2}CH_2Cl)_2$), 3.61 (m, 1H, H-5), 2.66 (t, 2H, $J$ = 7.5 Hz, $CH_2C\underline{H_2}Ph$), 2.56 (t, 2H, $J$ = 7.4 Hz, $COCH_2$), 2.06 (qt, 2H, $J$ = 7.5 Hz, $CH_2\underline{CH_2}CH_2$).

**Example 18**

**N-[4-(4-{4-[bis(2-chloroethyl)amino]phenyl}butanoyloxy)phenyl]-2-deoxy-2-fluoro-β-D-glucopyranosylamine (34b).**

A solution of the benzylated derivative 34a (189 mg, 0.23 mmol) in EtOH/THF (5/2.5 mL) was stirred under hydrogen atmosphere in the presence of 10% palladium on charcoal (47 mg) for 18 h at room temperature. After filtration of the catalyst on celite and evaporation of the solvent, the crude product was purified by silica gel chromatography (cyclohexane:ethyl acetate 1:9), to yield the deprotected product 34b (100 mg, 78%) as a white powder (α/β ratio =20/80):

$C_{26}H_{33}Cl_2FN_2O_6$

M = 559.46 g.mol$^{-1}$
Rf = 0.66 (cyclohexane:ethyl acetate 1:9)
IR (KBr) ν 3349 ($\nu_{NH}$, $\nu_{OH}$), 1750 ($\nu_{C=O}$), 1519 ($\delta_{NH}$), 1201, 1179, 1135, 1078, 1012 ($\nu_{C-O}$) cm$^{-1}$;
$^1$H NMR (200 MHz, acetone-$d_6$) δ 7.10 (d, 2H, $J_o$ = 8.5 Hz, $H_{Ar}$), 6.86 (d, 2H, $J_o'$ = 9.1 Hz, $H_{Ar}$), 6.75 (2d, 4H, $H_{Ar}$), 6.00 (d, 1H, $J_{1,NH}$ = 8.4 Hz, NHβ), 5.76 (d, 1H, $J_{1,NH}$ = 4.8 Hz, NHα), 5.27 (br t, 1 H, $J_{1,2}$ = 5.2 Hz, H-1α), 4.83 (br t, 1H, $J_{1,2}$ = 8.5 Hz, H-1β), 4.75 (d, 1H, $J$ = 4.5 Hz, OHβ), 4.61 (d, 1H, $J$ = 4.4 Hz, OHα), 4.41 (d, 1H, $J$ = 4.0 Hz, OHβ), 4.38 (d, 1H, $J$ = 5.0 Hz, OHα), 4.13 (td, 1H, $J_{2,3}$ = 8.7 Hz, $J_{2-F}$ = 51.8 Hz, H-2β), 4.01 (m, 1H, H-3β), 3.82-3.38 (m, 13H, $N(CH_2CH_2Cl)_2$, H-4, H-5, 2H-6, OH), 2.60 (t, 2H, $J$ = 7.5 Hz, $C\underline{H_2}Ph$), 2.50 (t, 2H, $J$ = 7.3 Hz, $COCH_2$), 1.94 (qt, 2H, $J$ = 7.4 Hz, $CH_2\underline{CH_2}CH_2$);
$^{13}$C NMR (50 MHz, acetone-$d_6$): 172.74 (CO), 145.65, 145.00 ($2C_{Ar}N$), 143.91 ($C_{Ar}O$), 130.98 ($C_{Ar}CH_2$), 130.41, 122.94, 114.84, 113.06 ($CH_{Ar}$), 92.84 (C-2, $J_{2-F}$ = 184.7 Hz), 83.67 (C-1, $J_{1-F}$ = 20.2 Hz), 77.92 (C-5), 77.01 (C-3, $J_{3-F}$ = 16.9 Hz), 71.73 (C-4, $J_{4-F}$ = 7.7 Hz), 62.53 ($C_6$), 53.86 ($NCH_2$), 41.59 ($CH_2Cl$), 34.54, 33.93 ($\underline{C}H_2Ph$, $CO\underline{C}H_2$), 27.71 ($CH_2\underline{C}H_2CH_2$);
MS (ESI) m/z 559.10 [M+1]$^+$.

**Example 19**

**N-[4-(3,4,6-tri-O-acetyl-2-deoxy-2-fluoro-β-D-glucopyranosyloxy)phenyl]-4-{4-[bis(2-chloroethyl)amino]phenyl}butanamide (37a) (see scheme 2a).**

**a) 4-nitrophenyl 3,4,6-tri-O-acetyl-2-deoxy-2-fluoro-β-D-glucopyranoside (35).**

A solution of 4-nitrophenol (1.88 g, 13.5 mmol) in $CH_3CN$ (25 mL) was added to a solution of bromide 6 (5 g, 13.5 mmol) in $CH_3CN$ (75 mL) containing $Ag_2O$ (4 g, 17.3 mmol). After stirring at room temperature for 23 h, the mixture was filtered on celite to remove Ag salts, and the solvent was evaporated. The crude product was purified by column chromatography on silica gel using cyclohexane:ethyl acetate (7:3) as eluent, thus affording the β-anomer 35 as a white solid (4.59 g, 79%):

$C_{18}H_{20}FNO_{10}$
M = 429.36 g.mol$^{-1}$
mp 142-143°C
Rf = 0.35 (petroleum benzine:ethyl acetate 7:3)
IR (KBr) ν 1755 ($\nu_{C=O}$), 1526 ($\nu_{asNO2}$), 1347 ($\nu_{sNO2}$), 1231, 1076 ($\nu_{C-O}$) cm$^{-1}$;
$^1$H NMR (200 MHz ; $CDCl_3$) δ 8.23 (d, 2H, $J_o$ = 9.2 Hz, $H_{Ar}$), 7.14 (d, 2H, $H_{Ar}$), 5.46 (td, 1H, $J_{2,3}$ = $J_{3,4}$ = 9.2 Hz, $J_{3,F}$ = 14.6 Hz, H-3), 5.30 (dd, 1H, $J_{1,F}$ = 3.1 Hz, $J_{1,2}$ = 7.6 Hz, H-1), 5.12 (t, 1H, $J_{4,5}$ = 9.7 Hz, H-4), 4.63 (ddd, 1H, $J_{2,F}$ = 50.4 Hz, H-2), 4.31 (dd, 1H, $J_{6a,6b}$ = 12.4 Hz, $J_{5,6a}$ = 5.4 Hz, H-6a), 4.17 (dd, 1H, $J_{5,6b}$ = 2.4 Hz, H-6b), 3.97 (ddd, 1H, H-5), 2.13, 2.09, 2.07 (each s, 3×3H, OAc);
$^{13}$C NMR (50 MHz, $CDCl_3$) δ 170.48, 170.03, 169.59 ($COCH_3$), 161.13 ($C_{Ar}O$), 143.57 ($C_{Ar}NO_2$), 125.94, 116.95 ($CH_{Ar}$), 97.98 (C-1, $J_{1,F}$ = 23.6 Hz), 88.96 (C-2, $J_{2,F}$ = 191.6 Hz), 72.57 (C-5), 72.47 (C-3, $J_{3,F}$ = 19.9 Hz), 67.94 (C-4, $J_{4,F}$ = 7.3 Hz), 61.83 (C-6), 20.66 ($CH_3$).

**b) 4-aminophenyl 3,4,6-tri-O-acetyl-2-deoxy-2-fluoro-β-D-glucopyranoside (36).**

A solution of the nitro derivative 35 (4.20 g, 9.78 mmol) in THF/EtOH (50/50 mL) was stirred under hydrogen atmosphere in the presence of 10% palladium on charcoal (550 mg) for 23 h at room temperature. Filtration of the catalyst on celite and evaporation of the solvent yield the amino product 36 (3.90 g, 100%) as a beige powder:

$C_{18}H_{22}FNO_8$
M = 399.37 g.mol$^{-1}$
mp 109-110°C
Rf = 0.21 (cyclohexane:ethyl acetate 6:4)
IR (KBr) ν 3466 ($\nu_{asNH2}$); 3376 ($\nu_{sNH2}$), 1753 (νc=o), 1224, 1067, 1039 ($\nu_{C-O}$) cm$^{-1}$;
$^1$H NMR (200 MHz, $CDCl_3$) δ 6.92 (d, 2H, $J_o$ = 8.9 Hz, $H_{Ar}$), 6.62 (d, 2H, $H_Ar$), 5.38 (td, 1H, $J_{3,4}$ = $J_{2,3}$ = 9.3 Hz, $J_{3,F}$ = 14.5 Hz, H-3), 5.09 (t, 1H, $J_{4,5}$ = 9.7 Hz, H-4), 4.96 (dd, 1H, $J_{1,F}$ = 3.0 Hz, $J_{1,2}$ = 7.7 Hz, H-1), 4.52 (ddd, 1H, $J_{2,F}$ = 50.4 Hz, H-2), 4.30 (dd, 1H, $J_{6a,6b}$ = 12.3 Hz, $J_{5,6a}$ = 5.2 Hz, H-6a), 4.14 (dd, 1H, $J_{5,6b}$ = 2.5 Hz, H-6b), 3.79 (ddd, 1H, H-5), 3.10 (br s, 2H, $NH_2$), 2.11, 2.08, 2.05 (each s, 3×3H, OAc);
$^{13}$C NMR (50 MHz, $CDCl_3$) δ 170.69, 170.17, 169.67 ($\underline{C}OCH_3$), 149.69 ($C_{Ar}O$), 142.95 ($C_{Ar}NH_2$), 119.60, 116.02 ($CH_{Ar}$), 100.50 (C-1, $J_{1,F}$ = 23.1 Hz), 89.20 (C-2, $J_{2,F}$ = 190.4 Hz), 72.97 (C-3, $J_{3,F}$ = 19.8 Hz), 72.03 (C-5), 68.33

(C-4, $J_{4,F}$ = 7.3 Hz), 62.00 (C-6), 20.80, 20.69 ($CH_3$).

**c) N-[4-(3,4,6-tri-O-acetyl-2-deoxy-2-fluoro-β-D glucopyranosyloxy)phenyl]-4-{4-[bis(2-chloroethyl)amino]phenyl}butanamide (37a).**

Amino derivative 36 (318 mg, 0.80 mmol) was dissolved in anhydrous DMF (20 mL) and chlorambucil (243 g, 0.80 mmol), DCC (165 mg, 0.80 mmol), and HOBt (118 mg, 0.90 mmol) were added. The solution was stirred for 20 h at room temperature, filtered and concentrated in vacuo. Evaporation of the filtrate followed by purification on silica gel (cyclohexane:ethyl acetate 6:4) gave compound 37a (431 mg, 79%) as a white solid:

$C_{32}H_{39}Cl_2FN_2O_9$
M = 685.57 g.mol$^{-1}$
mp 110-113°C
Rf = 0.31 (cyclohexane: ethyl acetate 6:4)
IR (KBr) ν 3326 ($\nu_{NH}$), 1757 ($\nu_{C=Oester}$), 1671 ($\nu_{C=Oamide}$), 1519 ($\delta_{NH}$), 1221, 1069, 1044 ($\nu_{C-O}$) cm$^{-1}$;
$^1$H NMR (200 MHz, $CDCl_3$) δ 7.44 (d, 2H, $J_o$ = 9.0 Hz, $H_{Ar}$), 7.23 (br s, 1H, NH), 7.09 (d, 2H, $J_o'$ = 8.6 Hz, $H_{Ar}$), 7.02 (d, 2H, $J_o$, $H_{Ar}$), 6.63 (d, 2H, $J_o'$, $H_{Ar}$), 5.41 (td, 1H, $J_{3,4}$ = $J_{2,3}$ = 9.2 Hz, $J_{3,F}$ = 14.5 Hz, H-3), 5.10 (t, 1H, $J_{4,5}$ = 9.5 Hz, H-4), 5.07 (dd, 1H, $J_{1,F}$ = 2.7 Hz, $J_{1,2}$ = 7.5 Hz, H-1), 4.56 (ddd, 1H, $J_{2,F}$ = 50.4 Hz, H-2), 4.30 (dd, 1H, $J_{5,6a}$ = 5.3 Hz, $J_{6a,6b}$ = 12.3 Hz, H-6a), 4.15 (dd, 1H, $J_{5,6b}$ = 2.1 Hz, H-6b), 3.79 (ddd, 1H, H-5), 3.73-3.58 (m, 8H, N($CH_2CH_2Cl)_2$), 2.62 (t, 2H, $J$ = 7.3 Hz, $C\underline{H_2}Ph$), 2.34 (t, 2H, $J$ = 7.3 Hz, $COCH_2$), 2.12, 2.08, 2.05 (each s, 3×3H, OAc), 2.11-1.98 (m, 2H, $CH_2C\underline{H_2}CH_2$);
$^{13}$C NMR (50 MHz, $CDCl_3$) δ 171.22, 170.66, 170.13, 169.65 ($COCH_3$), 153.16 ($C_{Ar}O$), 144.48 ($C_{Ar}N$), 133.94 ($C_{Ar}NH$), 130.52 ($\underline{C_{Ar}}CH_2$), 129.83, 121.40, 118.12, 112.26 ($CH_{Ar}$), 99.39 (C-1, $J_{1,F}$ = 23.2 Hz), 89.09 (C-2, $J_{2,F}$ = 190.6 Hz), 72.78 (C-3, $J_{3,F}$ = 19.9 Hz), 72.10 (C-5), 68.14 (C-4, $J_{4,F}$ = 7.3 Hz), 61.91 (C-6), 53.66 ($CH_2N$), 40.63 ($CH_2Cl$), 36.79 ($CO\underline{C}H_2$), 34.03 ($\underline{C}H_2Ph$), 27.21 ($CH_2\underline{C}H_2CH_2$), 20.81, 20.79, 20.68 ($CH_3$);
MS (ESI) m/z 685.30 [M+1]$^+$.

**Example 20**

**4-{4-[bis(2-chloroethyl)amino]phenyl}-N-[4-(2-deoxy-2-fluoro-β-D-glucopyranosyloxy)phenyl]butanamide (37b).**

To a solution of acetylated compound 37a (1.37 g, 2.0 mmol) in methanol (50 mL) was added sodium methoxide (20 mg) and the mixture was stirred at room temperature for 20 h. After neutralization with IRC 50 Amberlite ion-exchange resin ($H^+$), filtration and evaporation to dryness, a precipitation in diisopropylic ether provided compound 37b (1.1 g, 98%) as a white solid:

$C_{26}H_{33}Cl_2FN_2O_6$
M = 559.46 g.mol$^{-1}$
mp 170°C
Rf= 0.15 (cyclohexane:ethyl acetate 1:9)
IR (KBr) ν 3293 ($\nu_{NH}$, $\nu_{OH}$), 1667 ($\nu_{C=O}$), 1521 ($\delta_{NH}$), 1221, 1073, 1049 ($\nu_{CO}$) cm$^{-1}$;
$^1$H NMR (200 MHz, acetone-$d_6$) δ 9.07 (s, 1H, NH), 7.61 (d, 2H, $J_o$ = 9.0 Hz, $H_{Ar}$), 7.10 (d, 2H, $J_o'$ = 8.7 Hz, $H_{Ar}$), 7.03 (d, 2H, $J_o$, $H_{Ar}$), 6.73 (d, 2H, $J_o'$, $H_{Ar}$), 5.19 (dd, 1H, $J_{1,2}$ = 7.7 Hz, $J_{1,F}$ = 3.8 Hz, H-1), 4.88 (d, 1H, $J$ = 4.6 Hz, OH), 4.58 (d, 1H, $J$ = 4.6 Hz, OH), 4.24 (td, 1H, $J_{2,3}$ = 8.3 Hz, $J_{2,F}$ = 51.5 Hz, H-2), 3.89-3.56 (m, 14H, N($CH_2CH_2Cl)_2$, H-3, H-4, H-5, 2H-6, OH), 2.59 (t, 2H, $J$= 7.5 Hz, $C\underline{H_2}Ph$), 2.36 (t, 2H, $J$ = 7.4 Hz, $COCH_2$), 1.96 (qt, 2H, $CH_2C\underline{H_2}CH_2$);
$^{13}$C NMR (50 MHz, acetone-$d_6$) δ 171.45 (NHCO), 153.93 ($C_{Ar}O$), 145.53 ($C_{Ar}N$), 135.49 ($C_{Ar}NH$), 131.43 ($\underline{C_{Ar}}CH_2$), 130.37, 121.26, 117.68, 113.07 ($CH_{Ar}$), 99.59 (C-1, $J_{1,F}$ = 23.4 Hz), 93.13 (C-2, $J_{2,F}$ = 184.9 Hz), 77.79 (C-5), 76.11 (C-3, $J_{3,F}$ = 17.1 Hz), 71.21 (C-4, $J_{4,F}$ = 7.9 Hz), 62.31 (C-6), 53.95 ($CH_2N$), 41.56 ($CH_2Cl$), 36.93 ($CO\underline{C}H_2$), 34.86 ($\underline{C}H_2Ph$), 28.19 ($CH_2\underline{C}H_2CH_2$);
MS (ESI) m/z 559.30 [M+1]$^+$.

**Example 21**

**N-{2-[4-(3,4,6-tri-O-acetyl-2-deoxy-2-fluoro-β-D-glucopyranosyloxy) phenyl]amino-2-oxoethyl}-4-{4-[bis (2-chloroethyl)amino]phenyl} butanamide (40a).**

**a) N-[4-(3,4,6-tri-O-acetyl-2-deoxy-2-fluoro-β-D-glucopyranosyloxy) phenyl]-2-(N-Cbz-amino)acetamide (38).**

Amino derivative 36 (2.49 g, 6.24 mmol) was dissolved in anhydrous DMF (95 mL) and N-Cbz-glycine (1.44 g, 6.87 mmol), DCC (1.41 mg, 6.87 mmol), and HOBt (927 mg, 6.87 mmol) were added. The solution was stirred for 25 h at room temperature, filtered and concentrated in vacuo. The addition of ethyl acetate and a new filtration allowed the elimination of additional DCU. Evaporation of the filtrate followed by purification on silica gel (petroleum benzine:ethyl acetate 4:6) gave compound 38 (2.73 g, 74%) as a white solid:

$C_{28}H_{31}FN_2O_{11}$
M = 590.56 g.mol$^{-1}$
mp 84˚C
Rf = 0.33 (petroleum benzine:ethyl acetate 4:6)
IR(KBr) ν 3326 ($\nu_{NH}$), 1755 ($\nu_{C=Oester}$), 1694 ($\nu_{C=Oamide}$), 1510 ($\delta_{NH}$), 1369 ($\delta_{sCH3}$), 1224, 1067, 1046 ($\nu_{C-O}$) cm$^{-1}$ ;
$^1$H NMR (200 MHz, $CDCl_3$) δ 8.44 (br s, 1H, PhN<u>H</u>), 7.40 (d, 2H, $J_o$ = 9.0 Hz, $H_{Ar}$), 7.33 (s, 5H, $H_{Ar}$), 6.96 (d, 2H, $H_{Ar}$), 5.85 (br t, 1H, $CH_2N\underline{H}$), 5.41 (td, 1H, $J_{2,3}$ = $J_{3,4}$ = 9.2 Hz, $J_{3,F}$ = 14.6 Hz, H-3), 5.13 (s, 2H, $C\underline{H}_2Ph$), 5.12-5.03 (m, 2H, H-4, H-1), 4.55 (ddd, 1H, $J_{1,2}$ = 7.8 Hz, $J_{2,F}$ = 50.4 Hz, H-2), 4.28 (dd, 1H, $J_{6a,6b}$ = 12.3 Hz, $J_{5,6a}$ = 5.3 Hz, H-6a), 4.12 (dd, 1H, $J_{5,6b}$ = 2.1 Hz, H-6b), 3.99 (d, 2H, J = 5.2 Hz, $C\underline{H}_2NH$), 3.84 (ddd, 1H, $J_{4,5}$ = 10.0 Hz, H-5), 2.11, 2.05, 2.04 (each s, 3×3H, OAc);
$^{13}$C NMR (50 MHz, $CDCl_3$) δ 170.66, 170.16, 169.65, 167.43 (CO), 157.08 (NH<u>C</u>OO), 153.41 ($C_{Ar}O$), 136.08 ($\underline{C}_{Ar}CH_2$), 133.27 ($C_{Ar}NH$), 128.71, 128.44, 128.11, 121.64, 118.00 ($CH_{Ar}$), 99.17 (C-1, $J_{1,F}$ = 23.3 Hz), 89.11 (C-2, $J_{2,F}$ = 190.7 Hz), 72.77 (C-3, $J_{3,F}$ = 19.9 Hz), 72.06 (C-5), 68.16 (C-4, $J_{4,F}$ = 7.2 Hz), 67.47 ($\underline{C}H_2Ph$), 61.90 (C-6), 45.43 ($CO\underline{C}H_2NH$), 20.76, 20.64 ($CH_3$).

**b) N-[4-(3,4,6-tri-O-acetyl-2-deoxy-2-fluoro-β-D-glucopyranosyloxy) phenyl]-2-aminoacetamide (39).**

N-Cbz protected derivative 38 (2.73 mg, 4.62 mmol) was added, with stirring, to a suspension of ammonium formate (1.46 g, 23.1 mmol) in THF/EtOH (27/27 mL) containing 10% palladium on charcoal (545 mg). The mixture was heated at 70˚C for 50 min, filtered on celite, and evaporated under reduced pressure, to provide the expected compound 39 (2.07 g, 98%) as a solid:

$C_{20}H_{25}FN_2O_9$
M = 456.42 g.mol-1
mp 78˚C
Rf = 0.10 (10% methanol in ethyl acetate)
IR(KBr) ν 3402 ($\nu_{NH}$), 1750 ($\nu_{C=Oester}$), 1682 ($\nu_{C=Oamide}$), 1510 ($\delta_{NH}$), 1370 ($\delta_{sCH3}$), 1227, 1068, 1043 ($\nu_{C-O}$) cm$^{-1}$;
$^1$H NMR (200 MHz, $CDCl_3$) δ 9.46 (br s, 1H, NH), 7.52 (d, 2H, $J_0$ = 8.9 Hz, $H_{Ar}$), 7.00 (d, 2H, $H_{Ar}$), 5.40 (td, 1H, $J_{2,3}$ = $J_{3,4}$ = 9.2 Hz, $J_{3,F}$ = 14.4 Hz, H-3), 5.13-5.04 (m, 2H, H-1, H-4), 4.55 (td, 1H, $J_{1,2}$ = 8.5 Hz, $J_{2,F}$ = 50.5 Hz, H-2), 4.29 (dd, 1H, $J_{6a,6b}$ = 12.3 Hz, $J_{5,6a}$ = 5.1 Hz, H-6a), 4.13 (dd, 1H, $J_{5,6b}$ = 1.9 Hz, H-6b), 3.84 (ddd, 1H, $J_{4,5}$ = 9.6 Hz, H-5), 3.57 (br s, 2H, $CH_2$), 2.71 (m, 2H, $NH_2$), 2.11, 2.07, 2.05 (each s, 3×3H, OAc);
$^{13}$C NMR (50 MHz, $CDCl_3$) δ 170.74, 170.17, 169.95, 169.68 (CO), 153.18 ($C_{Ar}O$), 133.53 ($C_{Ar}NH$), 121.08, 118.03 ($CH_{Ar}$), 99.21 (C-1, $J_{1,F}$ = 23.2 Hz), 89.16 (C-2, $J_{2,F}$ = 190.7 Hz), 72.80 (C-3, $J_{3,F}$ = 19.9 Hz), 72.05 (C-5), 68.18 (C-4, $J_{4,F}$ = 7.1 Hz), 61.93 (C-6), 44.67 ($CO\underline{C}H_2NH_2$), 20.80, 20.67 ($CH_3$).

**c) N-{2-[4-(3,4,6-tri-O-acetyl-2-deoxy-2-fluoro-β-D-glucopyranosyloxy) phenyl]amino-2-oxoethyl}-4-{4-[bis (2-chloroethyl)amino] phenyl}butanamide (40a).**

Amino derivative 38 (1.5 g, 3.28 mmol) was dissolved in anhydrous DMF (100 mL) and chlorambucil (1.10 g, 3.62 mmol), DCC (745 mg, 3.62 mmol), and HOBt (488 mg, 3.62 mmol) were added. The solution was stirred for 40 h at room temperature, filtered and concentrated in vacuo. The addition of ethyl acetate and a new filtration allowed the elimination of more than DCU. Evaporation of the filtrate followed by purification on silica gel (5:5 to 1:9 cyclohexane: ethyl acetate gradient) gave compound 40a (2 g, 82%) as a white solid:

$C_{34}H_{42}Cl_2FN_3O_{10}$

M = 742.63 g.mol$^{-1}$
mp 130˚C
Rf = 0.54 (cyclohexane:ethyl acetate 2:8)
IR(KBr) ν 3307 ($\nu_{NH}$), 1755 ($\nu_{C=Oester}$), 1650 ($\nu_{C=Oamide}$), 1509 ($\delta_{NH}$), 1367 ($\delta_{sCH3}$), 1247, 1068, 1044 ($\nu_{C-O}$) cm$^{-1}$;
$^1$H NMR (200 MHz, $CDCl_3$) δ 9.29 (s, 1H, PhN<u>H</u>), 7.49 (d, 2H, $J_o$ = 9.0 Hz, $H_{Ar}$), 7.05-6.96 (m, 4H, $H_{Ar}$), 6.86 (t, 1H, $J$ = 4.6 Hz, $CH_2N$<u>H</u>), 6.59 (d, 2H, $J_o$' = 8.7 Hz, $H_{Ar}$), 5.41 (td, 1H, $J_{2,3}$ = $J_{3,4}$ = 9.2 Hz, $J_{3,F}$ = 14.5 Hz, H-3), 5.14-5.05 (m, 2H, H-4, H-1), 4.56 (ddd, 1H, $J_{1,2}$ = 7.8 Hz, $J_{2,F}$ = 50.4 Hz, H-2), 4.29 (dd, 1H, $J_{6a,6b}$ = 12.3 Hz, $J_{5,6a}$ = 5.3 Hz, H-6a), 4.16-4.10 (m, 3H, C<u>$H_2$</u>NH, H-6b), 3.84 (ddd, 1H, $J_{5,6b}$ = 2.3 Hz, $J_{4,5}$ = 9.9 Hz, H-5), 3.73-3.56 (m, 8H, N($CH_2CH_2Cl)_2$), 2.55 (t, 2H, $J$ = 7.4 Hz, C<u>$H_2$</u>Ph), 2.31 (t, 2H, $J$ = 7.3 Hz, COC<u>$H_2$</u>$CH_2$), 2.11, 2.06, 2.05 (each s, 3×3H, OAc), 1.93 (qt, 2H, $CH_2$C<u>$H_2$</u>$CH_2$);
$^{13}$C NMR (50 MHz, $CDCl_3$) δ 174.18, 170.64, 170.15, 169.65, 167.16 (CO), 153.33 ($C_{Ar}$O), 144.55 (<u>$C_{Ar}$</u>N), 133.75 (<u>$C_{Ar}$</u>NH), 130.36 (<u>$C_{Ar}$</u>$CH_2$), 129.78, 121.40, 118.12, 112.32 ($CH_{Ar}$), 99.36 (C-1, $J_{1,F}$ = 23.4 Hz), 89.15 (C-2, $J_{2,F}$ = 190.8 Hz), 72.80 (C-3, $J_{3,F}$ = 19.8 Hz), 72.14 (C-5), 68.18 (C-4, $J_{4,F}$ = 7.3 Hz), 61.93 (C-6), 53.68 ($NCH_2$), 44.55 (CO<u>C</u>$H_2$NH), 40.64 ($CH_2Cl$), 35.61 (CO<u>C</u>$H_2CH_2$), 34.10 (<u>C</u>$H_2$Ph), 27.36 ($CH_2$<u>C</u>$H_2CH_2$), 20.81, 20.69 ($CH_3$);
MS (ESI) m/z 742.35 [M+1]$^+$.

**Example 22**

**4-{4-[bis(2-chloroethyl)amino]phenyl}-N-{2-[4-(2-deoxy-2-fluoro-β-D-glucopyranosyloxy)phenyl]amino-2-oxoethyl}butanamide (40b).**

To a solution of acetylated compound 40a (100 mg, 0.135 mmol) in methanol (4 mL) was added sodium methoxide (2 mg) and the mixture was stirred at room temperature for 5 h. After neutralization with IRC 50 Amberlite ion-exchange resin (H$^+$), filtration and evaporation to dryness, a precipitation in EtOH provided compound 40b (83 mg, 100%) as a white solid:

$C_{28}H_{36}Cl_2FN_3O_7$
M = 616.51 g.mol$^{-1}$
mp 114˚C
Rf = 0.63 (10% methanol in ethyl acetate)
IR (KBr) ν 3312 ($\nu_{NH}$, $\nu_{OH}$), 1684 ($\nu_{C=O}$), 1510 ($\delta_{NH}$ 1231, 1075 ($\nu_{C-O}$) cm$^{-1}$;
$^1$H NMR (200 MHz, acetone-$d_6$) δ 9.22 (br s, 1H, PhN<u>H</u>CO), 7.56 (d, 2H, $J_o$ = 8.9 Hz, $H_{Ar}$), 7.43 (br t, 1H, $CH_2$NHCO), 7.09 (d, 2H, $J_o$' = 8.6 Hz, $H_{Ar}$), 7.03 (d, 2H, $J_o$, $H_{Ar}$), 6.72 (d, 2H, $J_o$', $H_{Ar}$), 5.19 (dd, 1H, $J_{1,2}$ = 7.9 Hz, $J_{1,F}$ = 2.6 Hz, H-1), 4.88 (br s, 1H, OH), 4.61 (br s, 1H, OH), 4.23 (td, 1H, $J_{2,3}$ = 8.2 Hz, $J_{2,F}$ = 51.2 Hz, H-2), 4.01-3.45 (m, 16H, N($CH_2CH_2Cl)_2$, H-3, H-4, H-5, 2H-6, $CH_2$NH, OH), 2.55 (t, 2H, $J$ = 7.6 Hz, C<u>$H_2$</u>Ph), 2.29 (t, 2H, $J$ = 7.4 Hz, $CH_2$C<u>$H_2$</u>CO), 1.89 (qt, 2H, $CH_2$C<u>$H_2$</u>$CH_2$);
$^{13}$C NMR (50 MHz, acetone-$d_6$) δ 173.83, 168.37 (CO), 154.23 ($C_{Ar}$O), 145.55 (<u>$C_{Ar}$</u>N), 134.78 (<u>$C_{Ar}$</u>NH), 131.56 (<u>$C_{Ar}$</u>$CH_2$), 130.40, 121.52, 117.73, 113.09 ($CH_{Ar}$), 99.47 (C-1, $J_{1,F}$ = 23.3 Hz), 93.16 (C-2, $J_{2,F}$ = 184.8 Hz), 77.82 (C-5), 76.09 (C-3, $J_{3,F}$ = 16.8 Hz), 71.18 (C-4, $J_{4,F}$ = 7.6 Hz), 62.27 (C-6), 53.94 ($NCH_2$), 44.16 (CO<u>C</u>$H_2$NH), 41.65 ($CH_2Cl$), 35.85 (CO<u>C</u>$H_2CH_2$), 34.82 (<u>C</u>$H_2$Ph), 28.40 ($CH_2$<u>C</u>$H_2CH_2$);
MS (ESI) m/z 616.31 [M+1]$^+$.

The table which follows illustrates the chemical structures of some compounds according to the invention.

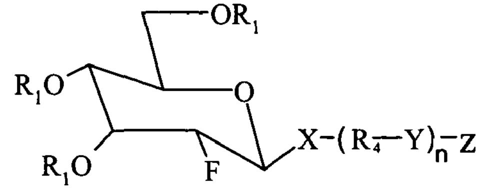

(I)

| No. | $R_1$ | X | $R_4$ | Y | n | Z |
|---|---|---|---|---|---|---|
| 14a | Ac | 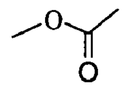 | | | 0 | |
| 14b | Bn | | | | | |
| 14c | H | | | | | |
| 15a | Ac | 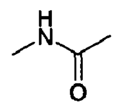 | | | 0 | |
| 15b | H | | | | | |
| 17a | Ac | 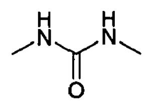 | | | 0 | |
| 17b | H | | | | | |
| 20a | Ac | 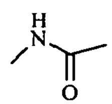 | 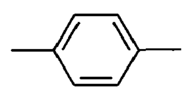 | 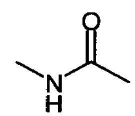 | 1 | |
| 20b | H | | | | | |
| 21 a | Ac | 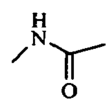 | $-CH_2-$ | 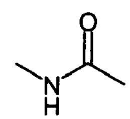 | 1 | |
| 21b | H | | | | | |

(continued)

| No. | $R_1$ | X | $R_4$ | Y | n | Z |
|---|---|---|---|---|---|---|
| 22a | Ac | 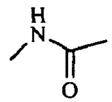 | $-(CH_2)3-$ | 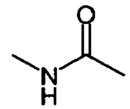 | 1 | 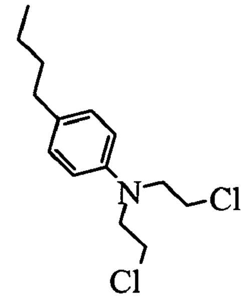 |
| 22b | H | | | | | |
| 31 a | Ac | 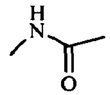 | $-(CH_2)_2-$ | 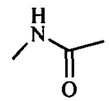 | 1 | |
| 31b | H | | | | | |
| 34a | Bn | 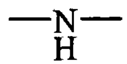 | 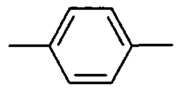 | 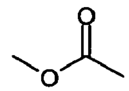 | 1 | |
| 34b | H | | | | | |
| 37a | Ac | 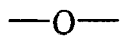 | 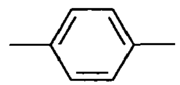 | 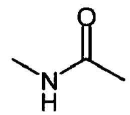 | 1 | |
| 37b | H | | | | | |
| 40a | Ac | 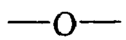 | 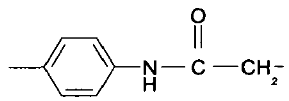 | 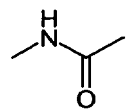 | 1 | |
| 40b | H | | | | | |

"Bn" means a benzyl group,
"Ac" means an acetyl group.

**Example 23: Pharmacological test.**

Some compounds of the invention have been the subject of pharmacological tests which have demonstrated their relevance as active substances in therapy and in particular in the treatment of cancer.

a) *In vitro* Cytotoxicity assay:

The materials and methods used for performing these pharmacological tests are as follows:

Cell cultures

Normal human fibroblasts were purchased from Promocell (Heidelberg, Germany). This frozen culture was obtained from foreskin waste from a 6-year old Caucasian male and the cells used in this work were from the seventh to twelfth passage of the culture. M4Beu, a human melanoma cell line, was established in the laboratory of Dr. J. F. Doré (INSERM, Unit 218, Lyon, France) from metastatic biopsy specimens and maintained in cell culture for almost 20 years in our lab. Breast cancer adenocarcinoma MCF 7, prostatic adenocarcinoma PC 3, colon adenocarcinoma DLD-1, lung non-small cell carcinoma A 549, ovary adenocarcinoma PA1 human cell lines and L 929 murine cell line were purchased from the European Collection of Cell Cultures (ECACC; Salisbury, United Kingdom). Murine Melanoma B16 was purchased from American Type of Culture Collection (ATCC, Manassas, USA) and murine colon carcinoma cell line CT-26 was obtained from Dr I. J. Fidler (University of Texas M.D. Anderson Cancer Center, Houston).

Stock cell cultures were maintained as monolayers in $75cm^2$ culture flasks in Glutamax Eagle's minimum essential medium with Earle's salts (MEM; Gibco BRL, Paisley, UK) supplemented with 10% fetal calf serum (Sigma, St Quentin Fallavier, France), 1 mM sodium pyruvate (Gibco), 1X vitamins solution (Gibco), 1X non essential amino acids solution (Gibco) and 4 μg/ml of gentamicine (Gibco). Cells were grown at 37˚C in a humidified atmosphere containing 5% $CO_2$.

Cells were plated at a density of $5 \times 10^3$ cells per well in 96-well microplates (Nunclon™, Nunc, Roskilde, Denmark) in 150 μl of culture medium and were allowed to adhere for 16 h before treatment with the compound tested.

Stock solution of each compound was prepared in dimethylsulfoxide (DMSO) and kept at -20˚C. until use. The percentage of DMSO was kept at 0.5% (v/v) whichever the concentration tested. This percentage did not modify cellular growth. Fifty microliters of a 4X solution in MEM was then added and a 48-h continuous drug exposure protocol was used. Then, the cytotoxic effect of compounds on tumour cells was tested by using Resazurin reduction test.

Resazurin Reduction Test

Resazurin was identified as the Alamar blue dye (O'Brien, J. et al.; European Journal of Biochemistry, 267, 5421-6, (2000)). The resazurin reduction test (RRT) was carried out according to the protocol described here. Briefly, plates were rinsed by 200 μl PBS (37˚ C, Gibco) using a multichannel dispenser (Labsystems, Helsinki, Finland) and emptied by overturning on absorbent toweling. Then, 150 μl of a 25 μg/ml solution of resazurin in MEM without SVF nor Phenol red was added in each well. The plates were incubated 1 h at 37˚ C. in an humidified atmosphere with 5% of $CO_2$ for fluorescence development by living cells. Fluorescence was then measured on the automated 96-well plate reader Fluoroskan Ascent FL™ (Labsystems) using an excitation wavelength of 530 nm and an emission one of 590 nm. The fluorescence is proportional to the number of living cells in the well and $IC_{50}$ (drug concentration required to decrease final cell population by 50%) was calculated from the curve of concentration-dependent cell number decrease, defined as the fluorescence in experimental wells as a percentage of that in control wells, with blank values subtracted.

Cytotoxic activity has been tested against the six human tumour cell lines breast adenocarcinoma MCF-7, colon adenocarcinoma DLD-1, ovary adenocarcinoma PA1, prostatic adenocarcinoma PC3, lung carcinoma A549, melanoma M4Beu and a primary culture of human fibroblasts.

The table 1 hereafter submits data obtained for the compounds: 17a, 20a, 21a, 22a, 31a, 37a, 40a and chlorambucil.

Table 1

| | $IC_{50}$ (μM) | | | | | | | | |
|---|---|---|---|---|---|---|---|---|---|
| Compound | Fibroblasts | PC3 | MCF7 | A549 | DLD1 | M4Beu | PA1 | CT26 | B16 |
| 17a | 13±2 | 4±1 | 26.4±0.6 | 8±2 | 7±3 | 27±11 | 0.6±0.2 | ND | ND |
| 20a | 4±2 | 1.4±0.3 | 5±2 | 2.7±0.6 | 3±2 | 7±3 | 0.3±0.1 | 1.7±0.3 | 4.6±0.3 |
| 21a | 4.1±0.1 | 10±2 | 8±2 | 5±2 | 4±3 | 6.8±0.9 | 1.0±0.1 | 7.0±1.0 | 6.4±0.6 |
| 22a | 4.1±0.1 | 3±1 | 12±1 | 13±5 | 9±2 | 9±3 | 0.27±0.04 | ND | ND |
| 31a | 7±5 | 3.7±0.3 | 13±5 | 9.2±0.5 | 11±4 | 15±3 | 0.24±0.08 | ND | ND |
| 37a | >50 | 11±4 | 26±11 | 1.90±0.01 | 10±5 | >50 | 0.76±0.06 | ND | ND |
| 40a | 17±6 | 7±2 | ND | 10±5 | 6±2 | 15±6 | 1.5±0.5 | 4.3±0.2 | 10.0±0.5 |
| chlorambucil | 99±2 | ND | >100 | 43±14 | 47±17 | >100 | 2.8±0.4 | 6.3±0.4 | 42.6±9.5 |
| ND: Non determined | | | | | | | | | |

As described in the table above, $IC_{50}$ lower than the Chlorambucil one is observed for all the acetylated compounds indicated here and whichever the cell line considered. These results underline a significant improvement of the cytotoxicity *in vitro* of peracetylated glucoconjugated comparatively to the free molecule. Chlorambucil *per se* is only moderately cytotoxic in these conditions ($IC_{50}$ values superior to 40 μM except in PA1 cells, a very sensitive cell line).

b) *In vivo* study of molecules 21 a and 40

This *in vivo* study is concerned with the evaluation of the acute toxicity of the two selected molecules in mice, and their antitumour activity in murine syngeneic models of solid tumours, i.e. B16 melanoma and CT-26 colon carcinoma.

Drugs were dissolved in a mixture of dimethylacetamide (Sigma) and oleic macrogolglycerides (Labrafil® M1944Cs, Gattefossé) (10:90%, vol/vol).

Animals were handled and cared for in accordance with the Guide for the Care and Use of Laboratory Animals (National Research Council, 1996) and the European Directive EEC/86/809. Protocols were conducted under the supervision of authorized investigators in accordance with the institution recommendations for the use of laboratory animals, based on the guidelines (décret n°87-848 du 19 octobre 1987).

- Determination of the Maximum Tolerated Dose (MTD)

Initially, the Maximum Tolerated Dose (MTD) after intraperitoneal (IP) administration was determinated.

For MTD determination, study was conducted on male OF1 mice weighing about 30 g, 6-weeks old (Charles River, France). Groups of five to six mice received a single IP injection of each drug at variable doses according to the compound (see Tables 2, 3 and 4).

Individual dose were based on the body weight of each mouse. All the mice received a constant injection volume of 250 μL per 25 g of body weight. A group of seven control mice received the vehicle.

After IP injection, mice were weighed and observed every two days over a 2 week-period to evaluate general clinical state. For each animal, a clinical state score (CSS) was calculated based on the absence (value 0) or presence (value 1) of diarrhoea, lethargy, rough coat and closed eyes. CSS was then calculated per group by summing individual scores. Loss of weight and mortality were also recorded.

The MTD was defined as the highest single dose that satisfy all the following criteria: zero deaths per group, maximal weight loss 15 % occuring at Day 1 (24 hours after injection) and CSS value as low as possible. Within a group, when mortality was observed to be 50% of the animals, weight loss and clinical state score were not considered to be relevant.

The following tables 2 to 4 report the so-obtained results:

Table 2

MTD for drug 21a in non-tumour bearing mice after a single IP administration.

| Dose / injection (mmol/kg) | Number of animals | Number of deaths (day) | Clinical State Score (CSS) | | | | | Weight loss at day 1 |
|---|---|---|---|---|---|---|---|---|
| | | | diarrhoea | rough coat | closed eyes | lethargy | total | |
| 0.07 | 6 | 0 | 1 | 0 | 0 | 0 | 1 | 5.6 % |
| 0.14 | 6 | 0 | 4 | 0 | 0 | 0 | 4 | 8.4% |
| 0.18 | 5 | 1 | 4 | 4 | 0 | 0 | 8 | 10.4% |
| 0.21 | 5 | 1 (D1) | 1 | 4 | 0 | 2 | 7 | 9.7 % |
| 0.28 | 6 | 4 (D1) | - | - | - | - | - | - |
| 0.56 | 6 | 4 (D1) | - | - | - | - | - | - |
| 0.76 | 5 | 5 (D1) | - | - | - | - | - | - |

Table 3

| MTD for drug 40a in non-tumour bearing mice after a single IP administration. | | | | | | | | |
|---|---|---|---|---|---|---|---|---|
| Dose / injection (mmol/kg) | Number of animals | Number of deaths (day) | Clinical state Score (CSS) | | | | | Weight loss at day 1 |
| | | | diarrhoea | rough coat | closed eyes | lethargy | total | |
| 0.07 | 6 | 0 | 2 | 0 | 0 | 0 | 2 | 6.3 % |
| 0.14 | 6 | 0 | 2 | 0 | 0 | 0 | 2 | 14.9% |
| 0.18 | 5 | 2 | 3 | 3 | 0 | 0 | 6 | 8.2% |
| 0.21 | 5 | 3 (D6) | - | - | - | - | - | - |
| 0.28 | 6 | 2 (D1) | 4 | 0 | 2 | 2 | 8 | 18.3% |
| 0.42 | 6 | 3(D6) | - | - | - | - | - | - |
| 0.56 | 5 | 5 (D6,7,9,13,15) | - | - | - | - | - | - |

Table 4

| MTD for chlorambucil in non-tumour bearing mice after a single IP administration. | | | | | | | | |
|---|---|---|---|---|---|---|---|---|
| Dose / injection (mmol/kg) | Number of animals | Number of deaths (day) | Clinical State Score (CSS) | | | | | Weight loss at day 1 |
| | | | diarrhoea | rough coat | closed eyes | lethargy | total | |
| 0.0175 | 5 | 0 | 1 | 0 | 0 | 0 | 1 | 1.4% |
| 0.035 | 5 | 0 | 2 | 0 | 0 | 0 | 2 | 6.1 % |
| 0.05 | 5 | 0 | 2 | 0 | 0 | 0 | 2 | 12.9 % |
| 0.07 | 5 | 0 | 2 | 5 | 0 | 0 | 7 | 13.1 % |
| 0.09 | 6 | 1 (D1) | 4 | 4 | 0 | 0 | 8 | 26 % |
| 0.14 | 5 | 4 (D1) | - | - | - | - | - | - |

It is noticed that the two drugs and chlorambucil induce high side effects (mainly diarrhoea) occuring as early as Day 1 after IP administration for dosage equal or higher than 0.14 mmol/kg for 21a, 0.07 mmol/kg for 40a and 0.0175 mmol/kg for chlorambucil. The doses inducing 100% mortality were 0.76 mmol/kg, 0.56 mmol/kg and 0.14 mmol/kg respectively for compound 2 1 a, 40a and chlorambucil.
According to the criteria defined above, MTD was 0.14 mmol/kg for drugs 21 a and 40a and 0.05 mmol/kg for chlorambucil.
In regard to MTD values, chlorambucil glucogonjugates appeared to be less toxic than chlorambucil.

Assessment of antimour activity

It is performed in tumour-bearing mice following an IP chemotherapy protocol.
Antitumour activity of both molecules and chlorambucil was assessed in B16-melanoma bearing mice (67 animals) and in CT-26 colon carcinoma bearing mice (30 animals).
To establish B16 melanoma model, 3 x $10^5$ B16F0 cells (ATCC, Manassas, USA ; in vitro doubling time of 16 hours) were suspended in 100 $\mu$L of phosphate buffer solution (PBS®, life Technologies) and injected into the subcutaneous tissue of the right flank of C57BL6J male mice, 6-weeks-old (Charles River, France) on day zero.
To establish CT-26 colon carcinoma model, 2.5 x $10^5$ CT26 cells (Dr Iaiaih J.Filder of MDAnderson Cancer Center, University of Texas; in vitro doubling time of 20 hours) were suspended in 100 $\mu$L of phosphate buffer solution and injected into the subcutaneous tissue of the right flank of BALB/c male mice, 6-weeks-old (Charles River, France) on day zero.
For both molecules and both animal models, treatment was initiated eight days post tumour grafting, when the tumours had reached a measurable size, corresponding to a calculated tumour weight (CTW) of 40 and 70 mg for B16

and CT-26 models respectively.

Animals were then randomly divided in both treatment and control groups. Mice were then given three doses of the drug tested (molecule 21a/ molecule 40a/ chlorambucil) at four-day intervals, (i.e at Day 8, Day 12 and Day 16 after tumour inoculation) according to the Q4d x 3 schedule typically employed in discovery screning (For more details, see table 5). Two dosages have been evaluated, MTD and 0.75 MTD

Mice were weighed and observed every two days over a 30-day -period to evaluate general clinical state. Length and width of tumours were measured every two days over a 30-day-period using a dedicated slide calliper.

The Calculated Tumour Weight (CTW) of each tumour was determined according to the formula:

$$CTW\ (mg) = L \times l^2/2$$

with L = length in mm and 1= width in mm.

The table 5 thereafter discloses the protocol of treatment.

Table 5

| Drug | Dose / injection (mmol/kg) | Treatment schedule | Tumour model | Nb animals / groups |
|---|---|---|---|---|
| Non treated controls | - | - | melanoma B16 | 12 |
| | - | - | colon carcinoma CT-26 | 6 |
| Solvant-treated controls (DMA/ Labrafil) | 250 $\mu$L / 25 g-weight | D8, D12, D16 | melanoma B16 | 12 |
| | 250 $\mu$L / 25 g-weight | D8, D12, D16 | colon carcinoma CT-26 | 6 |
| Chlorambucil | MTD = 0.05 | D8, D12, D16 | melanoma B16 | 5 |
| | | D8, D12, D16 | colon carcinoma CT-26 | 6 |
| | 0.75 MTD = 0.035 | D8, D12, D16 | melanoma B16 | 7 |
| 21a | MTD = 0.14 | D8, D12, D16 | melanoma B16 | 7 |
| | 0.75 MTD = 0.10 | D8, D12, D16 | melanoma B16 | 5 |
| | | D8, D12, D16 | colon carcinoma CT-26 | 6 |
| 40a | MTD=0.14 | D8, D12, D16 | melanoma B16 | 7 |
| | 0.75 MTD = 0.10 | D8, D12, D16 | melanoma B16 | 5 |
| | | D8, D12, D16 | colon carcinoma CT-26 | 6 |

Considering physiopathological features of the CT26 colon carcinoma model, mainly cachexy, drugs 21a and 40a, were assessed at the lowest dosage, i.e. 0.75 MTD administered at Day 8, Day 12, Day 16.

The reference treatment chlorambucil was given at the MTD dosage according to the same schedule.

Figures 1 and 2 report the so-obtained results.

Figure 1 presents the CTW time-course obtained for the B16 melanoma model for both drugs 21a and 40a and chlorambucil administered at the MTD dosage (figure 1a) and
0,75 MTD dosage (figure 1b).

For both MTD and 0,75 MTD dosages, each of the two chlorambucil derivative drugs was observed to induce a significant tumour growth inhibition ($p < 0.05$, student t-test,) as compared to non-treated control group and vehicle-treated group. In contrast, chlorambucil administered at both MTD and 0,75 MTD dosages failed to induce any tumour growth inhibition.

The Figure 2 presents the CTW time-course of CT26 carcinoma model

Each of the two drugs 21a and 40a, IP administered at the 0,75 MTD dosage at Day 8, 12 and 16 was observed

to induce a significant tumour growth inhibition ($p < 0.05$, student t-test) as compared to non-treated control group and vehicle-treated group. Chlorambucil administered at the MTD dosage according to the same schedule was observed to induce a significant tumour growth inhibition ($p < 0.05$, student t-test,) as compared to non-treated control group and vehicle-treated group.

For subcutaneously growing tumours, antitumour activity is often assessed by using the $\log_{10}$ cell kill (LCK) which is calculated from the following formula:

$$LCK = T\text{-}C \text{ value in days} / 3.32 \times Td$$

T is the median time (in days) required for the treatment group tumours to reach a predetermined CTW (e.g. 1000 mg). C is the median time (in days) required for the control group tumours to reach the same CTW. Td is the tumour volume doubling time (in days) estimated from the best fit straight line from a log-linear growth plot of the control group tumours in exponential growth.

According to the National Cancer Institute (NCI) guidelines, criteria of decision are :

$$LCK < 0.7 \qquad \text{inactive drug (-)}$$

$$0.7 < LCK < 1.2 \qquad \text{active drug (+)}$$

$$1.2 < LCK < 1.9 \qquad \text{highly active drug (++)}$$

According to the NCI criteria decisions, drug 21a exhibited active antimour activity on both B16 melanoma and CT26 colon carcinoma models, LCK values being around 1.1. More interesting, drug 40a exhibited highly active antitumour activity on both B16 melanoma and CT26 colon carcinoma models, LCK values being around 1.5.

In contrast, chlorambucil was inactive on B16 melanoma growth inhibition, with LCK < 0.7. Considering CT26 colon carcinoma model, chlorambucil exhibited a discrete antitumour activity, LCK value being 0.79.

An increase in life span was defined by the ratio of the median survival of drug-treated mice ($M_t$) versus controls ($M_c$): a survival $M_t/M_c$ equal or higher than 125 % was sought

Mean weight loss of the group was used to evaluate treatment side effects. A mean body weight loss of greater 20% was considered to indicate an excessively toxic dosage.

Life span of the animals was increased for both B16 melanoma and CT-26 colon carcinoma-bearing mice only treated with drugs 21a and 40a, and not with chlorambucil (see Table 6). Nevertheless, a high toxicity was observed for CT-26 bearing mice treated with drug 40a (maximum weight loss of 21 %).

The following table 6 reports end-points for anti-tumour activing assessment.

Table 6

| Drug | Dose / inj. (mmol/kg) | Schedule | Tumour model | Number animals | LCK | Weight loss (% max) | Survival Mt/Mc | Antitumour Efficacy |
|---|---|---|---|---|---|---|---|---|
| **Chlorambucil** | MTD (0.05) | D8, D12, D16 | B16 melanoma | 5 | 0.24 | 13.6% (J17) | 1 | - |
| | | | CT26 colon carcinoma | 6 | **0.79** | 13.9% (J16) | 0.74 | +/- |
| | 75% MTD (0.035) | D8, D12, D16 | B16 melanoma | 7 | 0.05 | 7.3% (J13) | 0.67 | - |
| **21a** | MTD (0.14) | D8, D12, D16 | B16 melanoma | 7 | **1.02** | 15.7% (J21) | 0.74 | + |
| | 75% MTD (0.1) | D8, D12, D16 | B16 melanoma | 5 | **1.10** | 16% (J17) | **1.18** | + |
| | | | CT26 colon carcinoma | 6 | **1.10** | 17.1% (J15) | **1.5** | + |
| **40a** | MTD (0.14) | D8, D12, D16 | B16 melanoma | 7 | **1.53** | 16.9% (J16) | 0.8 | ++ |
| | 75% MTD (0.1) | D8, D12, D16 | B16 melanoma | 6 | **1.81** | 13.6% (J17) | **1.5** | ++ |
| | | | CT26 colon carcinoma | 6 | **1.52** | 21.5% (J15) | **1.29** | ++ |

Accordingly, with respect to the NCI criteria decisions, chlorambucil glucoconjugates 21a and 40a exhibited active and highly active antitumour activities respectively on both B16 melanoma and CT-26 colon carcinoma experimental models. Chlorambucil parent drug failed in exhibiting any antitumour activity on the same experimental models.

Administration of the deoxyglucose conjugates provided herein can be effected by any method that enables delivery of the conjugates to the site of the cancer or suspected cancer.

In one embodiment, delivery may be via circulation in the bloodstream. To place the conjugates in contact with cancerous tissues or cells, the methods of administration include oral, buccal intraduodenal, parenteral injection (including intravenous, subcutaneous, intramuscular, intravascular, or infusion), topical administration, and rectal.

The amount of the conjugate administered will be dependent upon the subject being treated, the severity of the cancer, localization of the cancer, the rate of administration, the disposition of the conjugate (e.g., solubility and fluorescence intensity) and the discretion of the administrator.

However, an effective dosage is typically in the range of about 0.001 to about 100 mg per kg body weight, preferably about 1 to about 35 mg/kg/day, in single or divided doses. For a 70 kg human, this dosage would amount to about 0.05 to about 7 g/day, preferably about 0.2 to about 2.5 g/day.

In some instances, dosage levels below the lower limit of the aforesaid range may be more than adequate, while in other cases still larger doses may be employed without causing any harmful side effect, although such larger doses may be divided into several smaller doses for administration throughout the day.

The conjugate according to the present invention may, for example, be in a form suitable for oral administration, such as a tablet, capsule, pill, powder, sustained release formulation, solution, or suspension; for parenteral injection, such as a sterile solution, suspension or emulsion; for topical administration, such as an ointment or cream; or for rectal administration, such as a suppository.

The conjugate composition may be in unit dosage forms suitable for single administration of precise dosages and can include a conventional pharmaceutical carrier or excipient.

Exemplary parenteral administration forms include solutions or suspensions of the imaging conjugate in sterile aqueous solutions, for example, aqueous propylene glycol or dextrose solutions. Such dosage forms can be suitably buffered, if desired.

Suitable pharmaceutical carriers include inert diluents or fillers, water, and various organic solvents. The pharmaceutical compositions may, if desired, contain additional ingredients such as flavorings, binders, excipients, and the like. Thus for oral administration, tablets containing various excipients, such as citric acid, may be employed together with various disintegrants such as starch, alginic acid, and certain complex silicates, and with binding agents such as sucrose, gelatin, and acacia. Additionally, lubricating agents such as magnesium stearate, sodium lauryl sulfate, and talc are often useful for tableting purposes. Solid compositions of a similar type may also be employed in soft and hard filled gelatin capsules. Preferred materials, therefore, include lactose or milk sugar and high molecular weight polyethylene glycols. When aqueous suspensions or elixirs are desired for oral administration the conjugate therein may be combined with various sweetening or flavoring agents, coloring matters or dyes, and, if desired, emulsifying agents or suspending agents, together with diluents such as water, ethanol, propylene glycol, glycerin, or combinations thereof.

It may prove desirable to stabilize the instant compounds e.g. to increase their shelf life and/or pharmacokinetic half-life.

Shelf-life stability may be improved by adding excipients such as: a) hydrophobic agents (e.g., glycerol); b) non-linked sugars (e.g., sucrose, mannose, sorbitol, rhamnose, xylose); c) non-linked complex carbohydrates (e.g., lactose); and/or d) bacteriostatic agents.

It may be also advantageous to co-administrate a compound according to the invention with anti-neoplasic agent, i.e. under a free form in particular as disclosed previously.

## Claims

**1.** Compound of general formula (I):

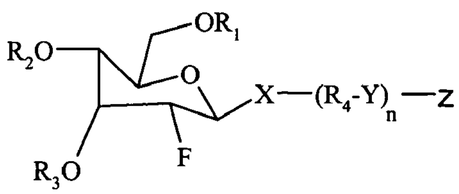

(I)

wherein:

- $R_1$, $R_2$ and $R_3$ mean, independently from the others, an hydrogen atom or an optionally substituted lower alkyl, a ($C_1$-$C_7$)acyl group or a benzyl,
- $R_4$ is an optionally substituted and/or interrupted hydrocarbon linker,
- X and Y are selected independently from each other from the group consisting of -NH-, -NR'-, -CO-, -NH(CO)-, -NH(CO)NH-, -NR'(CO)-, -O(CO)NH-,
- O(CO)NR'-, -(CO)NH-, -(CO)NR'-, -NH(CO)(R")NH(CO)-, -(CO)NH(R")(CO)NH-, - O-, -(CO)O-, -NH(CO)O-, -SC(O)NH-, -$NHSO_2$-, -$NR'SO_2$- and -O(CO)- and preferably are different one from the other, with R' being an optionally substituted lower alkyl group, and R" being a ($C_1$-$C_3$)alkylene,
- Z moiety represents an anti-neoplasic agent,
- n means 0 or 1,
- and the pharmaceutically acceptable salts thereof.

**2.** Compound according to claim 1, wherein $R_4$ is of formula

- -(Arylene)m-(P)p-(Alkylene)q-
or
- -(Alkylene)m-(P)p-(Arylene)q-

wherein

- m, p and q mean independently one from the others 0 or 1 with the proviso that at least one of them is different from 0,
- Alkylene means an optionally substituted ($C_1$-$C_7$)alkylene group,
- Arylene means an optionally substituted $C_5$ to $C_{10}$ arylene or heteroarylene, and
- P means a group as proposed for X and Y as defined in claim 1.

**3.** Compound according to claim 1 or 2, wherein alkylene means a methylene group.

**4.** Compound according to claim 2 or 3, wherein P means a group selected from -NH(CO)-,-NR'(CO)-,-O(CO)NH-,-O (CO)NR',-(CO)NH,-(CO)NR'-,-O(CO)-,-(CO)O-,-NH(CO)O-,-NR'(CO)O- with R' as defined in claim 1.

**5.** Compound according to anyone of previous claims, wherein n is equal to zero.

**6.** Compound according to the previous claim, wherein X means -O(CO)-, -NH(CO)- or -NH(CO)NH-.

**7.** Compound according to anyone of claim 1 to 4, wherein n is different from zero.

**8.** Compound according to the previous claim, wherein $R_4$ comprises or is

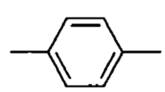

**9.** Compound according to claim 7 or 8, wherein X is selected from the group consisting of -NH-, NH(CO)-, -O-, -O (CO)-, -O(CO)NH- and -NH(CO)O-.

**10.** Compound according to any one of claims 7 to 9, wherein Y is -NHCO-, -O(CO)-, -O(CO)NH- and -NH(CO)O-.

**11.** Compound according to claim 7, wherein $R_4$ includes or is a ($C_1$-$C_7$) alkylene group and more particularly a methylene group.

**12.** Compound according to the previous claim, wherein X and Y represent a group selected from the group consisting of -NH(CO)-, -(CO)NH-, -NH(CO)NH- and -O(CO)-, -O(CO)NH- and-NH(CO)O-.

**13.** Compound according to claim 7, wherein $R_4$ means a radical of formula (Arylene)-P-(Alkylene) or (Alkylene)-P-(Arylene) wherein :

- Arylene is

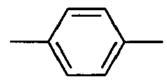,

- Alkylene is a ($C_1$-$C_3$)alkylene group, and
- P is -NHCO-, -CONH-, - NR' or -NH- with R' being as defined in claim 1.

**14.** Compound according to the previous claim, wherein Alkylene means a methylene group.

**15.** Compound according to any one of the previous claims, wherein $R_1$, $R_2$ and $R_3$ are different from hydrogen.

**16.** Compound according to any one of the previous claims, wherein $R_1$, $R_2$ and $R_3$ mean an acyl group and in particular an acetyl group.

**17.** Compound according to any one of the previous claims, wherein the anti-neoplasic agent is selected from the group consisting of cyclophosphamide, doxorubicin, vincristine, etoposide, cisplatin, ifosfamide, melphalan, chlorambucil, thiotepa, carboplatin, estramustine phosphate, prednimustine, 5-fluorouracil, camptothecin, vinblastine, paclitaxel, vinorelbine, docetaxel, epothilone B or D, discodermolid, nitrosoureas like procarbazine, lomustine, carmustine, estramustine, nucleoside like mercaptopurine, thioguanine, hydroxyurea, cytarabine, floxuridine, fludarabine, pentostatin, cladribine, gemcitabine, and capecitabine.

**18.** Compound according to any one of the previous claims, selected from the group consisting of:

3,4,6-tri-O-acetyl-1-[4-{4-[bis(2-chloroethyl)amino]phenyl}butyrate]-2-deoxy-2-fluoro-α,β-D-glucopyranose
3,4,6-tri-O-benzyl-1-[4-{4-[bis(2-chloroethyl)amino]phenyl}butyrate]-2-deoxy-2-fluoro-α,β-D-glucopyranose
1-[4-{4-[bis(2-chloroethyl)amino]phenyl}butyrate]-2-deoxy-2-fluoro-α,β-D-glucopyranose
N-(3,4,6-tri-O-acetyl-2-deoxy-2-fluoro-β-D-glucopyranosyl)-4-{4-[bis(2-chloroethyl)amino]phenyl}butanamide
4-{4-[bis(2-chloroethyl)amino]phenyl}-N-(2-deoxy-2-fluoro-β-D-glucopyranosyl)butanamide
N-(3,4,6-tri-O-acetyl-2-deoxy-2-fluoro-β-D-glucopyranosyl)-N'-{3-(4-[bis(2-chloroethyl)amino]phenyl)propyl} urea
N-{3-(4-[bis(2-chloroethyl)amino]phenyl)propyl}-N'-(2-deoxy-2-fluoro-β-D-glucopyranosyl)urea
N-(3,4,6-tri-O-acetyl-2-deoxy-2-fluoro-β-D-glucopyranosyl)-4-(4-{4-[bis(2-chloroethyl)amino]phenyl}butanamido)benzamide
4-(4-{4-[bis(2-chloroethyl)amino]phenyl}butanamido-N-(2-deoxy-2-fluoro-β-D-glucopyranosyl)benzamide
N-[2-(3,4,6-tri-O-acetyl-2-deoxy-2-fluoro-β-D-glucopyranosylamino)-2-oxoethyl-4-{4-[bis(2-chloroethyl)amino] phenyl}butanamide
4-{4-[bis(2-chloroethyl)amino]phenyl}-N-[2-(2-deoxy-2-fluoro-β-D-glucopyranosylamino)-2-oxoethyl]butanamide
N-[4-(3,4,6-tri-O-acetyl-2-deoxy-2-fluoro-β-D-glucopyranosylamino)-4-oxobutyl]-4-{4-[bis(2-chloroethyl)amino]phenyl}butanamide
4-{4-[bis(2-chloroethyl)amino]phenyl}-N-[4-(2-deoxy-2-fluoro-β-D-glucopyranosylamino)-4-oxobutyl]butanamide
N-[3-(3,4,6-tri-O-acetyl-2-deoxy-2-fluoro-β-D-glucopyranosylamino)-3-oxopropyl]-4-{4-[bis(2-chloroethyl)amino]phenyl}butanamide
4-{4-[bis(2-chloroethyl)amino]phenyl}-N-[3-(2-deoxy-2-fluoro-β-D-glucopyranosylamino)-3-oxopropyl]butanamide
N-[4-(4-{4-[bis(2-chloroethyl)amino]phenyl}butanoyloxy)phenyl]-3,4,6-tri-O-benzyl-2-deoxy-2-fluoro-β-D-glucopyranosylamine
N-[4-(4-{4-[bis(2-chloroethyl)amino]phenyl}butanoyloxy)phenyl]-2-deoxy-2-fluoro-β-D-glucopyranosylamine
N-[4-(3,4,6-tri-O-acetyl-2-deoxy-2-fluoro-β-D-glucopyranosyloxy)phenyl]-4-{4-bis(2-chloroethyl)amino]phenyl} butanamide
4-{4-[bis(2-chloroethyl)amino]phenyl}-N-[4-(2-deoxy-2-fluoro-β-D-glucopyranosyloxy)phenyl]butanamide
N-{2-[4-(3,4,6-tri-O-acetyl-2-deoxy-2-fluoro-β-D-glucopyranosyloxy)phenyl] amino-2-oxoethyl}-4-{4-[bis(2-chloroethyl)amino]phenyl}butanamide
4- {4-[bis(2-chloroethyl)amino]phenyl}-N- {2-[4-(2-deoxy-2-fluoro-β-D-glucopyranosyloxy)phenyl]amino-2-oxoethyl}butanamide and their salts

**19.** Pharmaceutical composition including in a physiological acceptable vehicle, at least one compound according to any one of the previous claims.

**20.** A compound according to any one of claims 1 to 18, as a anti-cancer agent.

**21.** Use of a compound according to any one of claims 1 to 18, for preparing a pharmaceutical composition intended to the treatment of cancer.

**22.** Method for treatment of cancer comprising the administration to a patient in need thereof, of at least one compound of any one of claims 1 to 18, or a composition according to anyone of claim 20.

# FIGURES

Figure 1A

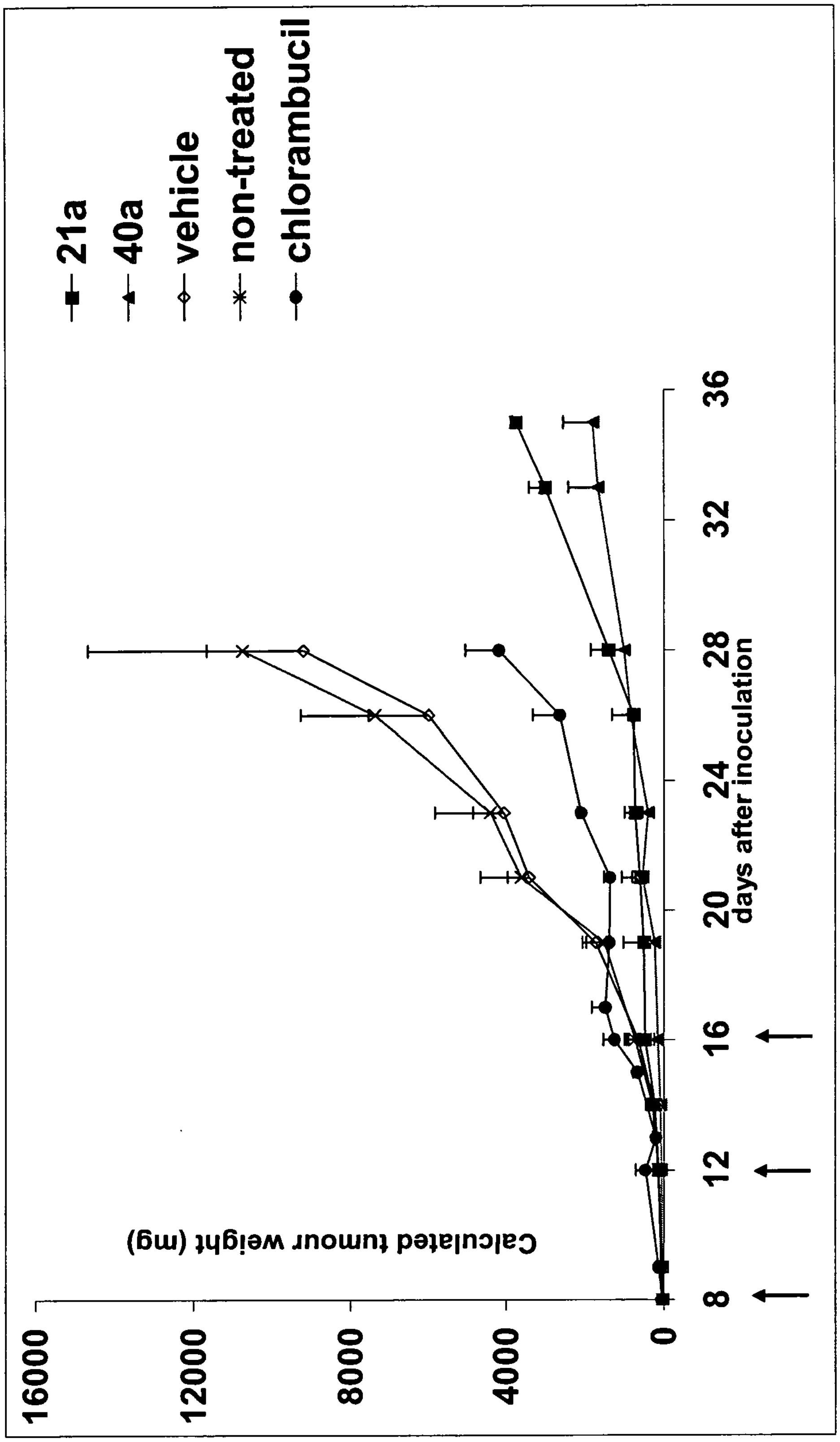

Figure 1B

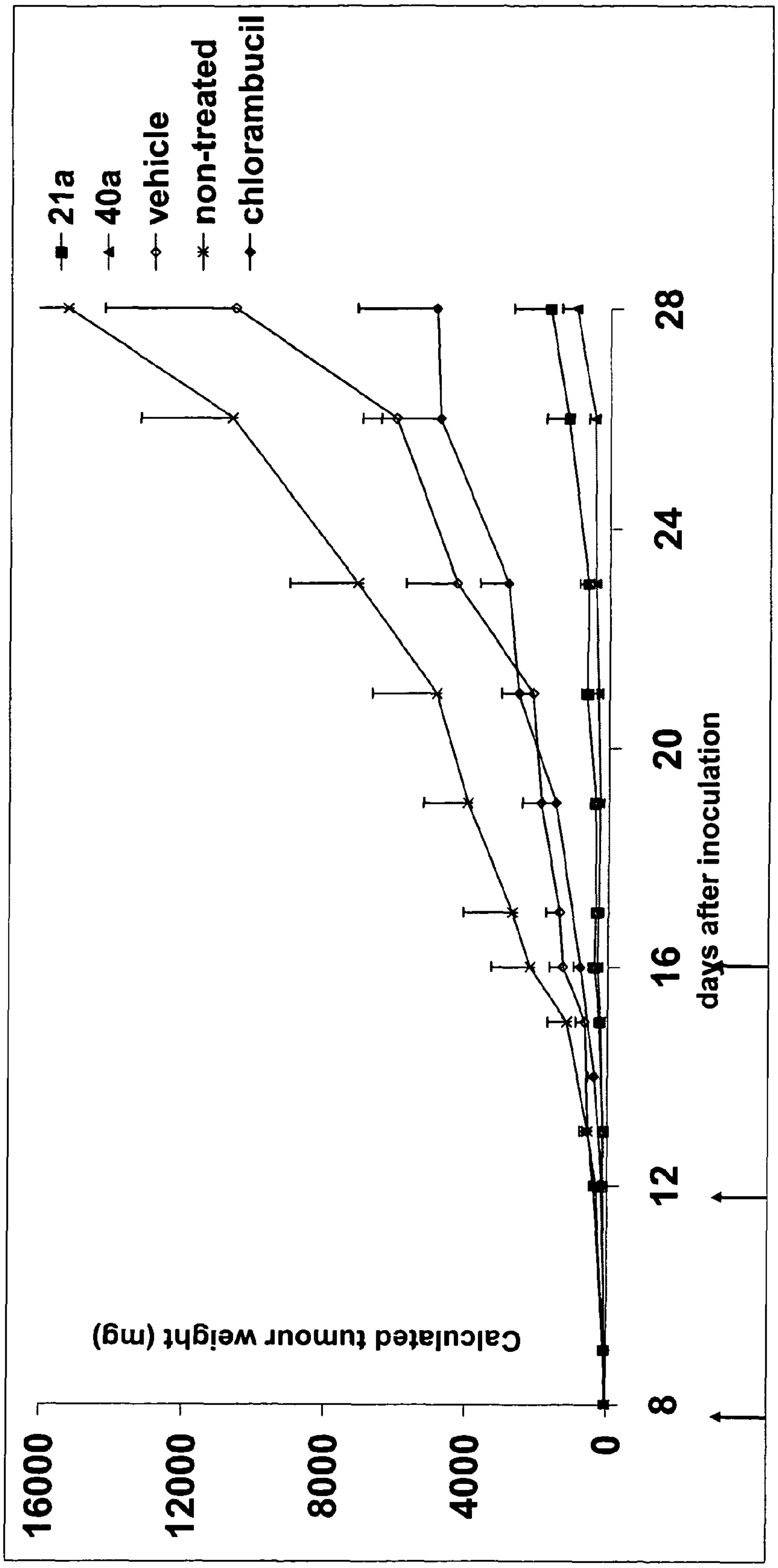

Calculated tumour weight (mg)
16000
12000
8000
4000
0
8
12
16
20
24
28
days after inoculation
21a
40a
vehicle
non-treated
chlorambucil

Figure 2

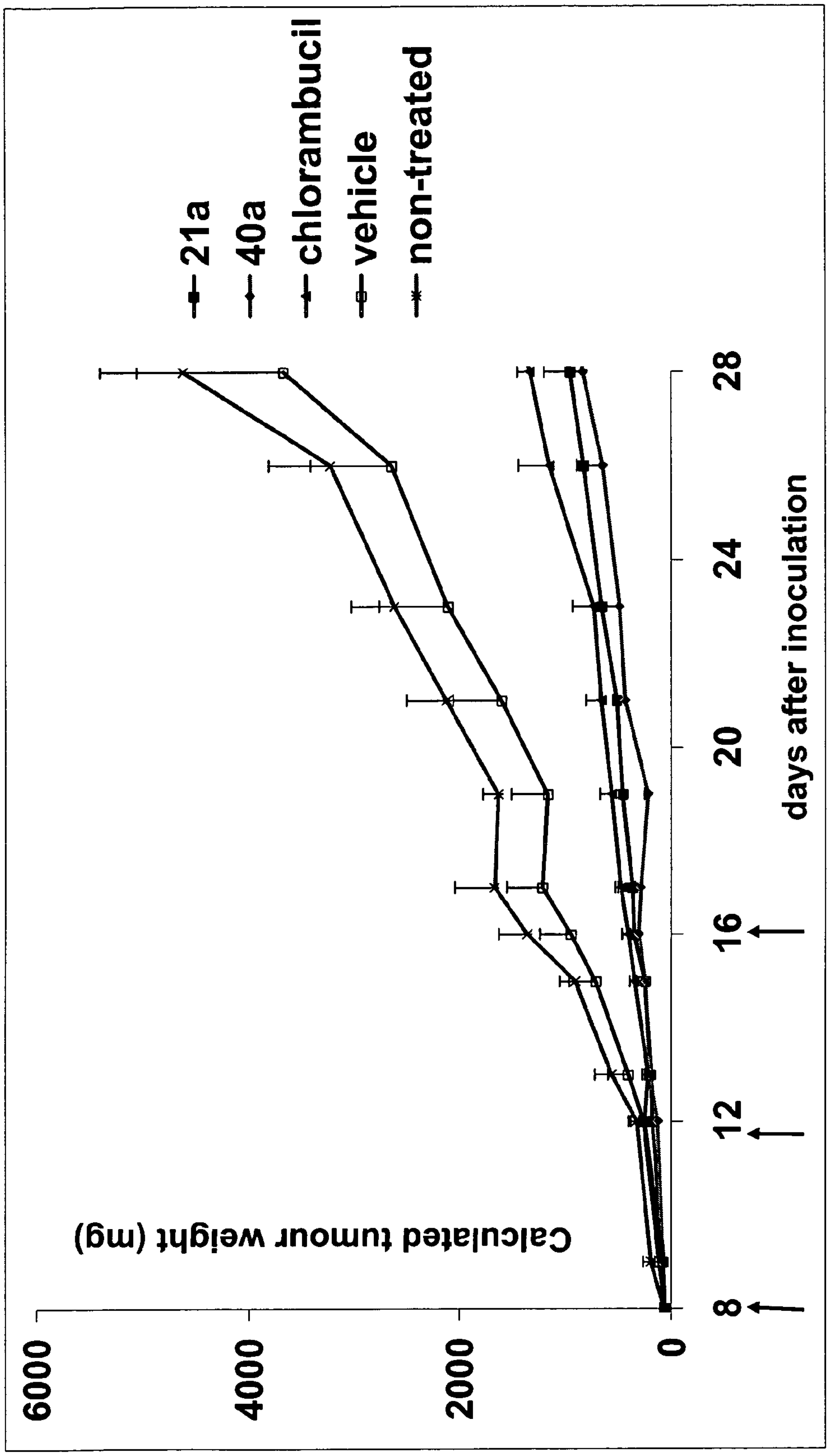
Calculated tumour weight (mg)
6000
4000
2000
0
8
12
16
20
24
28
days after inoculation
21a
40a
chlorambucil
vehicle
non-treated

European Patent Office

# PARTIAL EUROPEAN SEARCH REPORT

which under Rule 45 of the European Patent Convention shall be considered, for the purposes of subsequent proceedings, as the European search report

Application Number
EP 06 29 1157

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | US 5 621 002 A (BOSSLET KLAUS [DE] ET AL) 15 April 1997 (1997-04-15)<br>* column 3; compound III *<br>* claims 2,7-9 *<br>----- | 1-22 | INV.<br>C07H13/04<br>C07H13/12<br>A61K31/7028<br>A61P35/00 |
| Y | WO 2006/002381 A (WISCONSIN ALUMNI RES FOUND [US]; THORSON JON S [US]; LANGENHAM JOSEPH) 5 January 2006 (2006-01-05)<br>* page 2, line 26 - page 3, line 4 *<br>* page 4, line 8; compound 12BETA *<br>* figure 6 *<br>----- | 1-22 | |
| Y | JOSEPH M. LANGENHAN ET AL.: "Enhancing the anticancer properties of cardiac glycosides by neoglycorandomization" PROCEEDINGS OF THE NATIONAL ACADEMY OF SCIENCES OF THE UNITED STATES OF AMERICA, vol. 102, no. 35, 2005, pages 12305-12310, XP002413370<br>* figure 4; compound 12BETA *<br>----- | 1-22 | TECHNICAL FIELDS SEARCHED (IPC) |
| Y | EP 0 358 197 A2 (BRISTOL MYERS CO [US]) 14 March 1990 (1990-03-14)<br>* claims 1,6-8 *<br>-----<br>-/-- | 1-22 | C07H |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 5 January 2007 | Nikolai, Joachim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C07)

European Patent Office

# PARTIAL EUROPEAN SEARCH REPORT

Application Number
EP 06 29 1157

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/82926 A (LAMPIDIS THEODORE J [US]; PRIEBE WALDEMAR [US]) 8 November 2001 (2001-11-08) * page 4, line 1 - line 9 * ----- | 1-22 | |
| | | | TECHNICAL FIELDS SEARCHED (IPC) |

1

EPO FORM 1503 03.82 (P04C10)

European Patent Office

**INCOMPLETE SEARCH SHEET C**

Application Number
EP 06 29 1157

Although claim 22 is directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO.

EP 06 29 1157

This annex lists the patent family members relating to the patent documents cited in the above-mentioned European search report.
The members are as contained in the European Patent Office EDP file on
The European Patent Office is in no way liable for these particulars which are merely given for the purpose of information.

05-01-2007

| Patent document cited in search report | | Publication date | Patent family member(s) | | | Publication date |
|---|---|---|---|---|---|---|
| US 5621002 | A | 15-04-1997 | AT | 208213 | T | 15-11-2001 |
| | | | AU | 678494 | B2 | 29-05-1997 |
| | | | AU | 7169994 | A | 23-03-1995 |
| | | | CA | 2131662 | A1 | 10-03-1995 |
| | | | DE | 69428957 | D1 | 13-12-2001 |
| | | | DE | 69428957 | T2 | 06-06-2002 |
| | | | DK | 642799 | T3 | 25-02-2002 |
| | | | EP | 0647450 | A1 | 12-04-1995 |
| | | | ES | 2167343 | T3 | 16-05-2002 |
| | | | JP | 3773120 | B2 | 10-05-2006 |
| | | | JP | 7149667 | A | 13-06-1995 |
| | | | NO | 943319 | A | 10-03-1995 |
| | | | PT | 642799 | T | 29-04-2002 |
| | | | ZA | 9406920 | A | 12-04-1995 |
| WO 2006002381 | A | 05-01-2006 | NONE | | | |
| EP 0358197 | A2 | 14-03-1990 | AU | 606536 | B2 | 07-02-1991 |
| | | | AU | 4115089 | A | 26-04-1990 |
| | | | CA | 1323628 | C | 26-10-1993 |
| | | | DK | 169240 | B1 | 19-09-1994 |
| | | | FI | 894124 | A | 07-03-1990 |
| | | | HU | 51284 | A2 | 28-04-1990 |
| | | | IL | 91521 | A | 25-01-1994 |
| | | | JP | 1851814 | C | 21-06-1994 |
| | | | JP | 2115194 | A | 27-04-1990 |
| | | | JP | 5067640 | B | 27-09-1993 |
| | | | NO | 893548 | A | 07-03-1990 |
| | | | NZ | 230496 | A | 26-11-1991 |
| | | | US | 4912204 | A | 27-03-1990 |
| | | | YU | 161989 | A1 | 28-02-1991 |
| | | | ZA | 8906778 | A | 27-06-1990 |
| WO 0182926 | A | 08-11-2001 | AU | 4006701 | A | 12-11-2001 |
| | | | CA | 2411406 | A1 | 08-11-2001 |
| | | | EP | 1322304 | A1 | 02-07-2003 |
| | | | US | 2003181393 | A1 | 25-09-2003 |
| | | | US | 6670330 | B1 | 30-12-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5622936 A
- US 5621002 A
- US 7001888 B

### Non-patent literature cited in the description


- **WOODWARD.** *Cancer Res.,* 1954, vol. 14, 599-605
- **TAI et al.** *Bioorg. Med. Chem.,* 2001, vol. 9, 1133-1140
- **MCCARTER et al.** *J. Am. Chem. Soc.,* 1997, vol. 119, 5792-5797
- **VALU et al.** *J. Med. Chem.,* 1990, vol. 33, 3014-3019
- **O'BRIEN, J. et al.** *European Journal of Biochemistry,* 2000, vol. 267, 5421-6
- Guide for the Care and Use of Laboratory Animals. National Research Council, 1996